# EUROPEAN PATENT APPLICATION

(11) **EP 4 042 881 A1**
(43) Date of publication of application: **17.08.2022**
(21) Application number: 20883408.5
(22) Date of filing: 30.04.2020
(51) Int. Cl.: A23L 33/135, A23P 10/30, A23P 10/35

(54) **MICROCAPSULE, PREPARATION METHOD AND APPLICATION THEREOF**

(30) Priority: 29.10.2019 CN 201911036807; 29.10.2019 CN 201911036790; 30.10.2019 CN 201911043504; 30.10.2019 CN 201911043477; 31.10.2019 CN 201911062990; 05.11.2019 CN 201911069119
(71) Applicant: INNER MONGOLIA MENGNIU DAIRY (GROUP) CO., LTD., Hohhot, Inner Mongolia 011500 (CN)
(72) Inventor: Kang, Jiaqi, Inner Mongolia 011500 (CN); Zhang, Zhiyao, Inner Mongolia 011500 (CN); Zheng, Lijun, Inner Mongolia 011500 (CN); Dong, Yang, Inner Mongolia 011500 (CN); Liu, Yeting, Inner Mongolia 011500 (CN); Li, Yuan, Inner Mongolia 011500 (CN); Li, Wenqing, Inner Mongolia (CN); Bai, Haoxue, Inner Mongolia 011500 (CN)
(74) Representative: Bals & Vogel Patentanwälte PartGmbB
(86) International application number: PCT/CN2020/088467
(87) International publication number: WO 2021/082382

(57) **Abstract**

A microcapsule and a preparation method thereof, especially a multi-layer microcapsule containing an active substance and a preparation method thereof, the microcapsule having a single-layer or a multiple-layer encapsulation structure. A probiotic microcapsule and a preparation method thereof, the probiotic microcapsule having a single-layer or a multi-layer encapsulation structure. A food product or a health product containing a probiotic microcapsule (comprising a dairy product, a fermented-style food product, a beverage, chocolate, candy, a baked good, a fruit or vegetable juice product, etc. containing a probiotic microcapsule) and a preparation method thereof. A probiotic microcapsule soft powder and a preparation method thereof, the probiotic microcapsule soft powder comprising probiotic microcapsule nata de coco soft powder and probiotic microcapsule gel ball soft powder.

## Description

### FIELD OF THE INVENTION

This application belongs to the field of food biotechnology. More specifically, this application relates to a microcapsule and a preparation method thereof, in particular to a multi-layered microcapsule containing an active substance and a preparation method thereof. This application also relates to a probiotic microcapsule and a preparation method thereof. The probiotic microcapsule has one or more layers of embedding structure. In addition, the present application relates to a composite wall material for probiotic microcapsules, probiotic microcapsules, and a preparation method thereof. This application also relates to a food or health product containing probiotic microcapsules, including dairy products, fermented flavored foods, beverages, chocolates, candies, baked foods, fruit and vegetable juice foods, etc. which contain probiotic microcapsules, and also relates to a preparation method of a food or health product containing probiotics microcapsules. This application also relates to a soft particle of a probiotic microcapsule and a preparation method thereof. The soft particle of a probiotic microcapsule includes a coconut fiber fruit soft particle of a probiotic microcapsule and a gel ball soft particle of a probiotic microcapsule.

### BACKGROUND OF THE INVENTION

In addition to providing energy to people, satisfying people's hobbies, and providing various ingredients of a human body, many food ingredients also have physiological activities that can regulate human functions, such as enhancing immunity, preventing diseases and other functional properties. Food scientists and food companies in many countries pay more and more attention to the research and development of this functional food.

Functional food is very important for improving physical fitness and nutritional intervention for chronic diseases. It covers nutritious foods, formula foods for special medical purposes, special dietary foods, health foods and functional general foods, etc., and is a gold sector with the most market demand and development potential in the health industry. In the context of general health, the restructuring and integration of the functional food industry will provide consumers with a complete whole-process health solution.

Functional ingredients contained in functional foods include: functional polysaccharides (including chitosan, tea polysaccharides, dietary fiber, etc.); functional lipids (including polyunsaturated fatty acids such as DHA, etc.); functional proteins/peptides/amino acids (e.g., taurine, lactoferrin, immunoglobulin, etc.); micro-ecological regulators (including probiotics, prebiotics, synbiotics); and vitamins and minerals, etc.

In the preparation process of functional foods, a microencapsulation technology is an effective and promising method. By forming one or more layers of capsule-like protective film around the embedded active material, the microencapsulation technology can significantly improve the survival rate of active substances in unfavorable environments, and can partially mask the bad flavor of the embedded substances. In the microencapsulation technology, the coating material, the embedding method, the granulation method and the drying method in the preparation process are very important for the function of the microcapsule.

The existing microcapsule coating materials are mostly edible gum materials such as gelatin, pectin, sodium alginate, etc. If often consumed in large quantities, they will have certain side effects on special groups such as infants and the elderly. Single-layered microcapsules are the most commonly used form of microcapsules. However, regardless of the particle surface bacteria content or water and gas barrier properties, single-layered microcapsules are weaker than double-layered or multi-layered microcapsules, making the stability of the single-layered microcapsules relatively low.

In view of the shortcomings of the prior art, the purpose of this application includes providing a safe, non-toxic, and no side effect microcapsule with small particle size and rich in active substances, which has good stability under normal temperature conditions and has a sustained release effect. Moreover, it can be added to products with high water activity.

Probiotics are a kind of living microorganisms that have a certain promoting effect on the health of the host, and have multiple physiological functions in terms of treatment and health care. However, ordinary probiotics such as bifidobacteria are relatively fragile and are easily affected by surrounding environmental factors. The direct addition of spore probiotics to a product can easily affect the state, quality and shelf life of a product. Therefore, most of the current probiotic products need to be stored in low temperature refrigeration, so as to maximize the number of active probiotics, and the number of colonies will decrease with time. In the application of probiotics in the field of food and dairy products, all kinds of probiotics in the products with probiotics added in the field of dairy products, for example, due to pasteurization in room temperature yogurt, have been killed, and no longer have the probiotic function of lactic acid bacteria and probiotics. In addition, in low-temperature yogurt products, due to the poor resistance of lactic acid bacteria and other probiotics, they are affected by gastric acid and enzymes, resulting in a sharp decrease in the number of probiotics that enter the intestines and cannot effectively exert their probiotic effects.

Therefore, how to effectively increase the number of live probiotic bacteria has become an urgent issue to be solved at present. Based on the above problems, this application adopts microencapsulation technology to embed probiotics. By forming a layer, double layer or multi-layered capsule-like protective film around the live probiotic bacteria, the survival rate of probiotic bacteria in adverse environments can be significantly improved, wherein the selection of wall materials, embedding methods, granulation methods and drying methods are very important to the protective effect of microcapsules.

In addition, adding probiotic microcapsules to food or health products can protect the activity of probiotics, effectively prevent the environment from affecting the survival of probiotics, and at the same time can greatly reduce the impact of gastric acid and enzymes, so that probiotics can maintain a relatively high number and activity when they reach the human intestinal tract.

### SUMMARY OF THE INVENTION

In order to solve the defects in the prior art, the present application relates to a microcapsule and a preparation method thereof, and in particular to a multi-layered microcapsule containing an embedded active substance and a preparation method thereof.

A microcapsule, the microcapsule having one or more layers of embedding structure, comprising: a pellet core and optionally at least one layer of shell coating the pellet core; wherein the pellet core includes embedded substance and microcapsule core material, and the at least one layer of shell coating the pellet core includes one, two or more layers of wall materials.

The outer diameter of the pellet core of a microcapsule is about 50-500 µm (preferably about 50 to about 300 µm), and the outer diameter of a single-layered or multi-layered coated microcapsule is about 200 to about 1000 µm (preferably about 100 to about 500 µm).

In the microcapsule, the embedded substance includes a functional active substance, and the active substance is selected from one or more of functional polysaccharides, functional lipids, functional proteins/peptides/amino acids, micro-ecological regulators, vitamins and minerals.

In the microcapsule, the functional polysaccharide is selected from one or more of chitosan, tea polysaccharide, dietary fiber, and dextran; preferably, the functional lipid is selected from one or more of lecithin, EPA and DHA; preferably, the functional protein/peptide/amino acid is selected from one or more of taurine, lactoferrin, immunoglobulin, and whey protein peptide; preferably, the micro-ecological regulator is selected from one or more of probiotics, prebiotics, and synbiotics.

The preparation method of the above microcapsules comprises the following steps:
(1) preparation of a primary pellet core: uniformly mixing an embedded substance and a microcapsule core material, optionally curing, and filtering to collect wet particles, and then drying to obtain dry particles of primary pellet core; and optionally
(2) preparation of a multi-layered microcapsule: using the above-mentioned dry particles of single-layered microcapsule as pellet core particles, uniformly coating a layer of wall material outside the pellet core particles, and optionally spraying curing solution to cure the capsule wall, drying the prepared microcapsules, and collecting the double-layered dry microcapsule particles or further coating once or more times.

In the step (1) of the preparation method, the pellet cores are prepared preferably by extrusion spheronization granulation, centrifugal granulation, acute angle extrusion granulation, or fluidized bed spray granulation (preferably extrusion spheronization granulation or centrifugal granulation), dried and collected.

In the step (2), the wall material is preferably coated by means of fluidized bed spray.

For example, a specific embodiment includes the method of combining extrusion spheronization granulation (centrifugal granulation) and fluidized spray coating to prepare microcapsule particles; in this way, spherical particles with good roundness can be produced, and the outer layer of the particles can also achieve the effect of blocking water and odor and protect the embedded active material. Moreover, the particles prepared in this way can be subsequently added to an aqueous product without being affected. The extrusion spheronization granulation method or the centrifugal granulation method is used to prepare a round and spherical primary pellet core, and then the water-blocking coating layer material and the hydrophilic coating layer material are uniformly fluidized to the surface of the spherical pellet core. Then, a water-blocking coating layer is fluidized so that the microcapsule particles do not disintegrate when exposed to water, and protects the embedded substance from contacting the outside world (e.g., water or other solvents), thereby prolonging the shelf life of the microcapsule particles in aqueous products.

One of the objectives of this application includes providing a solid dry particle microcapsule that can be stored for a long time under normal temperature conditions, wherein one, double or multi-layered wall material is used to embed probiotics into solid particles of 50-500 µm, so that microcapsules are less affected by the environment and can be applied to the fields of medicine, health products and food. The microcapsules obtained in the present application are especially double-layered and multi-layered coated microcapsules, wherein the amount of viable probiotics in the microcapsules is significantly increased, and particles have uniform size and are shiny. The microcapsules can be stored for a long time under normal temperature conditions. For example, after 30 days, 60 days or 120 days of storage, the amount of viable probiotics in the microcapsules decreases slightly, and is still high. Moreover, the above-mentioned microcapsules are resistant to acid, increasing the number of probiotic bacteria that eventually enter the intestinal tract. In addition, the microcapsules also have good hydrophobicity, and the residual bacteria on the surface of the particles are less, thereby reducing the probability of leakage of probiotics.

Another object of the present application is to provide a method for preparing probiotic microcapsules, the probiotic microcapsules having one or more layers of embedding structure, comprising the following steps:
(1) preparation of single-layered microcapsules: uniformly mixing probiotic powder or probiotic mud with materials such as the first wall material, and optionally curing and filtering to collect wet particles, and drying to obtain dry particles of single-layered microcapsule; and optionally
(2) preparation of multi-layered microcapsules: using the above-mentioned dry particles of single-layered microcapsule as probiotic core particles, and uniformly coating a layer of wall material outside the probiotic core particles (preferably by fluidized bed spray granulation method), and optionally spraying a curing solution to cure the capsule wall, drying the prepared microcapsules (preferably drying in a fluidized bed), collecting dry particles of double-layered microcapsules or further coating them once or more times.

Preferably, in the above-mentioned double-layered or multi-layered probiotic microcapsules, the outer diameter of the core particles obtained after coating the first layer is about 50-500 µm (preferably about 50 to about 300 µm), and the outer diameter of the double-layered or multi-layered microcapsules is about 200 to about 1000 µm (preferably about 100 to about 500 µm).

In one aspect, the present application also relates to the pretreatment method of the wall material in the preparation method of the probiotic microcapsules, which includes: mixing the wall material with water, fully dissolving the wall material, and carrying out alternate cooling and heating treatments to form a stable gel.

Preferably, the wall material is mixed with water, and stirred at a low temperature of about 2 to 8°C for 4 to 16 hours, and more preferably, the whey protein solution is further heat-treated at about 75 to 96°C for about 30 to 180 minutes; more preferably, cooled immediately at a cooling temperature of -20 to 4°C; stored at 4°C for more than 10-60 hours to obtain a whey protein gel solution. More preferably, the wall material is whey protein.

In one aspect, the present application also relates to a probiotic microcapsule. The probiotic microcapsule has a one-layered or multi-layered embedding structure, which is prepared according to the above-mentioned preparation method of the probiotic microcapsule.

In one aspect, the probiotic microcapsules of the present application can be used in the food and health product industries such as dietary supplements. This application also relates to the application of probiotic microcapsules as food additives, such as heat-processed foods or frozen foods, preferably fermented flavored foods, beverages, chocolates, sweets such as chewing gum, baked foods such as pudding, fruit and vegetable juice foods, etc.

Preferably, the probiotic microcapsules of the present application are dairy additives, and more preferably added to milk, yogurt, cheese, ice cream, milk powder, and dairy beverages.

Preferably, the added amount of microcapsules containing probiotics is 0.03 to 0.15%.

Another object of the application is to provide a method for preparing the probiotic microcapsules. The method used in this application is a combination of extrusion granulation and fluidized bed spray granulation. Compared with the common granulation method currently used, the probiotic microcapsule particles obtained are denser and more hydrophobic, and have lower residual bacteria on the surface, thereby reducing the probability of probiotic leakage, increasing the number of viable probiotic within the same shelf life, and expanding the scope of use of microcapsules.

Another object of the present application is to provide a method for preparing probiotic microcapsules having one or more layers of embedding structure, including: probiotic core particles and optionally at least one layer of outer shell coating the probiotic core particles, wherein the probiotic core particles comprise a core material and a first layer of wall material, the core material comprises one or more kinds of probiotic powder or probiotic mud, and the core material is coated by the first layer of wall material. The at least one layer of the outer shell coating the probiotic core particles includes one, two or more layers of wall materials, which are respectively a second layer of wall material, a third layer of wall material or more layer of wall material, which comprises the following steps:
(1) preparation of single-layered microcapsules: optionally mixing the probiotic powder or probiotic mud with a binder, and granulating, and then uniformly mixing with the first wall material (preferably an acute angle extrusion granulation method) and optionally curing and filtering to collect wet particles and drying to obtain dry particles of single-layered microcapsule; and optionally
(2) preparation of multi-layered microcapsules: using the above-mentioned dry particles of single-layered microcapsule as probiotic core particles, uniformly coating a layer of wall material outside the probiotic core particles (preferably by fluidized bed spray granulation method), and optionally spraying a curing solution so that the capsule is cured and insoluble in water, drying the prepared microcapsules (preferably drying in a fluidized bed), and collecting the dry particles of the double-layered microcapsules or further coating the dry particles of the double-layered microcapsules once or more times.

Another object of the present application is to provide a method for preparing probiotic microcapsules, which uses fluidized bed granulation and spray fluidization as a main method of granulation and multiple embedding of microcapsules: making edible solid phase components such as probiotics in the form of powder or granular form into a core material through fluidized granulation using a binder solution as a liquid phase component, and then uniformly wrapping the core material with one or more layers of wall material by a fluidized bed spray coating method to obtain probiotic microcapsules with one or more layers of embedding structure.

This application uses a fluidized bed process to prepare probiotic microcapsules. The fluidized bed process can greatly reduce the moisture and water activity in the microcapsule production process, and is particularly suitable for preparing components that are sensitive to moisture as microcapsules. Especially, the microcapsule uses a unique multi-layered embedding structure, and the wall material in the middle layer of the microcapsule can effectively protect and isolate the core material from contact with moisture and air. Even when the microcapsule is placed in a water-based liquid, the wall material in the middle layer can effectively prevent moisture from entering the core of the microcapsule. The outer wall material can effectively wrap and stabilize the overall structure of the microcapsule. When the microcapsule is placed in a water-based liquid, the outer wall material can help the microcapsule to be suspended in the liquid.

One of the objectives of this application is to provide a product containing probiotic microcapsules, including food or health products containing probiotic microcapsules, such as milk and dairy products, fermented flavored foods, beverages, chocolate, candies, baked goods, fruit and vegetable juice foods, etc. that contain probiotic microcapsules. In addition, the food or health products of this application also include but are not limited to the following products: meat, fish, poultry and game; meat juices; pickled, frozen, dried and cooked fruits and vegetables; jellies, jams, preserves; eggs; milk and dairy products; edible oils and fats.

The microcapsules are solid dry particle microcapsules that can be stored for a long time under normal temperature conditions. The probiotics are embedded into solid particles of 50-500 µm by using one, double or multi-layered wall materials. The application and storage conditions are less affected by the environment, and the microcapsules can be applied to the fields of medicine, health products and food. The microcapsules obtained in the present application, especially double-layered and multi-layered coated microcapsules, have a significantly increased amount of viable probiotics in the microcapsules, a uniform particle size and a shiny appearance. The microcapsules can be stored for a long time under normal temperature conditions. For example, after 30 days, 60 days or 120 days of storage, the amount of viable probiotics in the microcapsules decreases slightly, and is still high. Moreover, the above-mentioned microcapsules are resistant to acid, increasing the number of probiotic bacteria that eventually enter the intestinal tract. In addition, the microcapsules also have good hydrophobicity, and the residual bacteria on the surface of the particles are lower, thereby reducing the probability of leakage of probiotics.

Another object of this application is to provide a method for preparing food or health products containing probiotic microcapsules, which comprises the following steps:
(1) preparation of probiotic microcapsules;
(2) adding probiotic microcapsules to foods or health products, including but not limited to dairy products, fermented flavored foods, beverages, chocolates, candies, baked foods, fruit and vegetable juice foods that contain probiotic microcapsules, preferably to milk, yogurt, cheese, ice cream, milk powder, and dairy beverage;
preferably, the added amount of microcapsules containing probiotics is 0.02 to 1%.

One of the objectives of the present application is to provide a soft particle of a probiotic microcapsule, including coconut fiber fruit soft particle of a probiotic microcapsule and gel ball soft particle of a probiotic microcapsule, wherein the coconut fiber fruit soft particle of a probiotic microcapsule includes: one or more of coconut water, coconut milk, coconut jam, sucrose, white granulated sugar, High Fructose Syrup, Bacillus xylinus, acidity regulator, probiotic microcapsules, ethanol and purified water, preferably, wherein the content of sucrose is about 5-10%, the content of white granulated sugar is about 3-5%, the content of High Fructose Syrup is about 0-5%, the content of ammonium dihydrogen phosphate is about 0.3-0.8%, the content of Bacillus xylinus is about 0.1-0.5%, and the content of acidity regulator is about 1-5%. The gel ball soft particle of a probiotic microcapsule includes: one or more of white granulated sugar, sodium alginate, xanthan gum, konjac gum, carrageenan, curing solution (containing calcium lactate and calcium chloride), probiotic microcapsules, pigments, and purified water, preferably, wherein the content of white granulated sugar is about 10-15%, sodium alginate is about 0.5-1%, xanthan gum is about 0.1-0.3%, konjac gum is about 0.1-0.2%, and the concentration of calcium lactate in a curing solution is about 1-3%.

The probiotic microcapsules are solid dry particle microcapsules that can be stored for a long time under normal temperature conditions. The probiotics are embedded into solid particles of 50-500 µm by using one, double or multi-layered wall materials. The application and storage conditions are less affected by the environment, and the microcapsules can be applied to the fields of medicine, health products and food. The microcapsules, especially double-layered and multi-layered coated microcapsules, have a significantly increased amount of viable probiotics in the microcapsules, a uniform particle size and a shiny appearance. The microcapsules can be stored for a long time under normal temperature conditions. For example, after 30 days, 60 days or 120 days of storage, the amount of viable probiotics in the microcapsules decreases slightly, and is still high. Moreover, the above-mentioned microcapsules are resistant to acid, increasing the number of probiotic bacteria that eventually enter the intestinal tract. In addition, the microcapsules also have good hydrophobicity, and low residual bacteria on the surface of the particles, thereby reducing the probability of leakage of probiotics.

The soft particle of a probiotic microcapsule described in the present application has both coconut fruit functionality and probiotic properties, and has rich nutrition, excellent flavor, good chewing mouthfeel, and strong probiotic survival and function. Compared with the existing products containing coconut soft particles, this product can not only maintain higher stability under normal temperature shelf storage conditions for at least 6 months, but also has a higher number of probiotics and bacterial activity, which fully guarantees the number of probiotics that reach the human gut after the product is eaten, so as to truly exert a better probiotic effect.

The microcapsules added to this product adopt the method of embedding microcapsules in coconut fruit. Compared with the direct addition of microcapsules, this product not only increases the functional properties of coconuts, but also provides secondary protection for probiotics, making the survival rate of probiotics higher than direct addition of microcapsules, and achieving better effect. At the same time, the crisp coconut taste fully guarantees the taste and flavor of the final product. In addition, the microcapsules added in this product are also added to a sodium alginate solution and the mixture was added dropwise into a curing solution to form pellets of calcium alginate encapsulated probiotic microcapsules.

### Definition

Unless otherwise stated, all technical and scientific terms used herein have the same meaning as those commonly understood by those skilled in the art to which this application belongs, but in case of conflict, the definition in this specification shall prevail.

As used in the specification and claims, the singular forms "a", "an" and "the (said)" include plural forms unless clearly specified otherwise in the context.

Unless otherwise specified, the percentages (%) in this specification are all weight percentages (wt%).

All numerical values or expressions related to component amounts, process conditions, etc. used in the specification and claims should be understood to be modified by "about" in all cases. The term "about" when referring to a quantity or numerical range means that the quantity or numerical range referred to is an approximate value within experimental variability (or within statistical experimental error), so the quantity or numerical range can vary from the stated quantity or numerical range, for example, vary between ±5 of the stated quantity or numerical range.

All ranges referring to the same components or properties include endpoints, which can be independently combined. Since these ranges are continuous, they include every value between the minimum and maximum values. It should also be understood that any numerical range cited in this application is intended to include all sub-ranges within that range.

When this application is directed to a range of physical property such as molecular weight or chemical property, all combinations and sub-combinations of the ranges and specific embodiments within them shall be included. The term "comprising" (and related terms such as "containing" or "having" or "including") includes embodiments, e.g., any combination of substances, compositions, methods, or processes, etc., that "consists of" or "consists essentially of the described features."

The "and/or" used in this specification and claims should be understood as "alternative or both" of the associated components, that is, the components coexist in some cases and exist separately in other cases. Multiple components listed with "and/or" should be understood in the same way, that is, "one or more" related components. In addition to the components specifically identified in the "and/or" clause, other components may optionally be present, whether related or unrelated to those specifically identified components. Therefore, as a non-limiting example, referring to "A and/or B", when used to link open ending words such as "includes", in one embodiment, may only refer to A (optionally including components other than B), in another embodiment, may only refer to B (optionally including components other than A), and in yet another embodiment, refers to A and B (optionally including other components), etc.

The "or" used in the specification and claims should be understood as having the same meaning as the above-defined "and/or". For example, when separating items in a list, "or" or "and/or" should be translated as inclusive, that is, including multiple or at least one of listed components, but also including more than one, and optional any other items not listed. Only the terms clearly pointing to the opposite, such as "only one" or "exactly one", or "consisting of" as used in the claims shall mean including multiple or exactly one of the listed components. Generally, the term "or" as used herein is considered to point to an exclusive choice (i.e., one or the other, but not both) only when there is an exclusive antecedent word such as "one of", "only one", or "exactly one".

It should be understood that, unless explicitly indicated to the contrary, in any method claimed herein that includes more than one step or action, the order of the steps and actions of the method need not be limited to the mentioned order of the steps and actions of the method.

The abbreviations used in this application have the usual meanings in the fields of food, biology and chemistry.

Probiotics: the type of probiotics in the probiotic microcapsules of this application uses any probiotics approved by the state as the protection object of embedding core material of the microcapsule. It includes one or more of Bifidobacterium adolescentis, Bifidobacterium animalis (Bifidobacterium lactis), Bifidobacterium bifidum, Bifidobacterium breve, Bifidobacterium infantis, Bifidobacterium longum, Lactobacillus acidophilus, Lactobacillus casei, Lactobacillus crispatus, Lactobacillus delbrueckii subsp. Bulgaricus, Lactobacillus delbrueckii subsp. lactis, Lactobacillus fermentum, Lactobacillus gasseri, Lactobacillus helveticus, Lactobacillus johnsonii, Lactobacillus paracasei, Lactobacillus plantarum, Lactobacillus reuteri, Lactobacillus rhamnosus, Lactobacillus salivarius, Streptococcus thermophilus, Bacillus coagulans and other probiotics. The details are shown in Tables A-D below.

### A. List of strains that can be used

### in food

| | Name | Latin name | Strain No. |
|---|---|---|---|
| I | 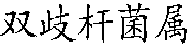 | Bifidobacterium | |
| 1 | 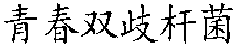 | Bifidobacterium adolescentis | |
| 2 | 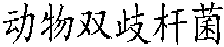 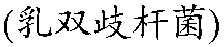 | Bifidobacterium animalis(Bifidobacterium lactis) | |
| 3 | 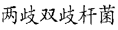 | Bifidobacterium bifidum | |
| 4 | 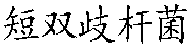 | Bifidobacterium breve | M-16V |
| 5 | 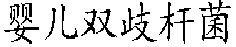 | Bifidobacterium infantis | |
| 6 | 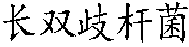 | Bifidobacterium longum | |
| II | 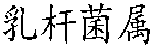 | Lactobacillus | |
| 1 | 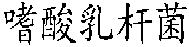 | Lactobacillus acidophilus | DSM13241 |
| | | | R0052 |
| 2 | 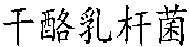 | Lactobacillus casei | |
| 3 | 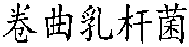 | Lactobacillus crispatus | |
| 4 | 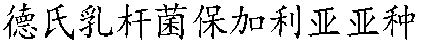 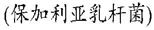 | Lactobacillus delbrueckii subsp. Bulgaricus (Lactobacillus bulgaricus) | |
| 5 | 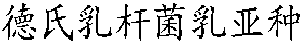 | Lactobacillus delbrueckii subsp. Lactis | |
| 6 | 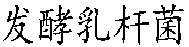 | Lactobacillus fermentum | CECT5716 |
| 7 | 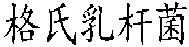 | Lactobacillus gasseri | |
| 8 | 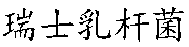 | Lactobacillus helveticus | |
| 9 | 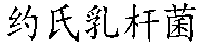 | Lactobacillus j ohnsonii | |
| 10 | 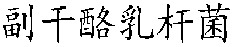 | Lactobacillus paracasei | GM080 |
| | | | GMNL-33 |
| 11 | 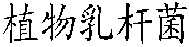 | Lactobacillus plantarum | CGMCC NO.1258 |
| | | | 299v |
| | | | ST-III |
| 12 | 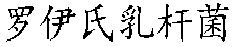 | Lactobacillus reuteri | |
| 13 | 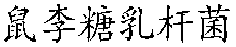 | Lactobacillus rhamnosus | R0011 |
| 14 | 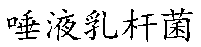 | Lactobacillus salivarius | |
| 15 | 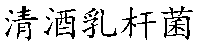 | Lactobacillus sakei | |
| III | 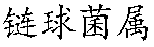 | Streptococcus | |
| 1 | 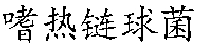 | Streptococcus thermophilus | |
| IV | 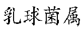 | Lactococcus | |
| 1 | 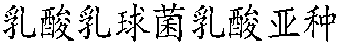 | Lactococcus Lactis subsp. Lactis | |
| 2 | 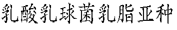 | Lactococcus Lactis subsp. Cremoris | |
| 3 | 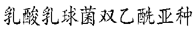 | Lactococcus Lactis subsp. Diacetylactis | |
| V | 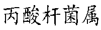 | Propionibacterium | |
| 1 | 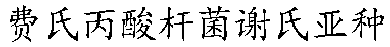 | Propionibacterium freudenreichii subsp. Shermanii | |
| 2 | 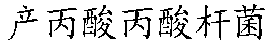 | Propionibacterium acidipropionici | |
| VI | 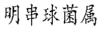 | Leuconostoc | |
| 1 | 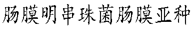 | Leuconostoc mesenteroides subsp. Mesenteroides | |
| VII | 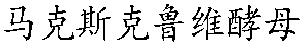 | Kluyveromyces marxianus | |
| VIII | 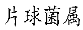 | Pediococcus | |
| 1 | 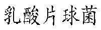 | Pediococcus acidilactici | |
| 2 | 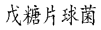 | Pediococcus pentosaceus | |
| IX | 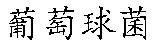 | Staphylococcus | |
| 1 | 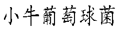 | Staphylococcus vitulinus | |
| 2 | 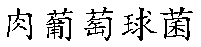 | Staphylococcus carnosus | |
| 3 | 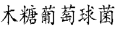 | Staphylococcus xylosus | |
| X | 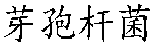 | Bacillus | |
| 1 | 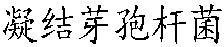 | Bacillus coagulans | |

### B. List of probiotic strains that can be used in health food

| No. | Name | Latin name | Note |
|---|---|---|---|
| I | 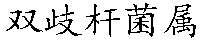 | Bifidobacterium | |
| 1 | 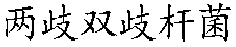 | Bifidobacterium bifidum | |
| 2 | 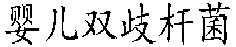 | Bifidobacterium infantis | |
| 3 | 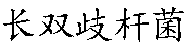 | Bifidobacterium longum | |
| 4 | 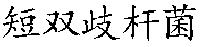 | Bifidobacterium breve | |
| 5 | 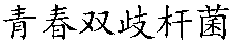 | Bifidobacterium adolescentis | |
| II | 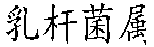 | Lactobacillus | |
| 1 | 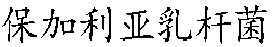 | Lactobacillus bulgaricus | |
| 2 | 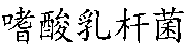 | Lactobacillus acidophilus | |
| 3 | 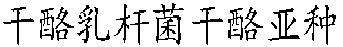 | Lactobacillus casei subsp. Casei | |
| 4 | 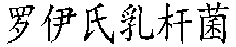 | Lactobacillus reuteri | |
| 5 | 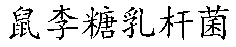 | Lactobacillus rhamnosus | |
| III | 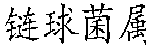 | Streptococcus | |
| 1 | 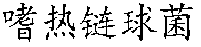 | Streptococcus thermophilus | |

### C. List of strains that can be used

### in infant food

| No. | Strain name | Latin name | Strain No. |
|---|---|---|---|
| 1 | 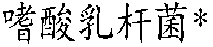 | Lactobacillus acidophilus | NCFM |
| 2 | 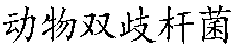 | Bifidobacterium animalis | Bb-12 |
| 3 | 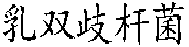 | Bifidobacterium lactis | HN019 |
| | | | Bi-07 |
| 4 | 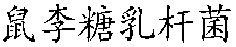 | Lactobacillus rhamnosus | LGG |
| | | | HN001 |
| 5 | 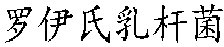 | Lactobacillus reuteri | DSM17938 |
| 6 | 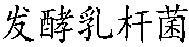 | Lactobacillus fermentum | CECT5716 |

### D. List of fungal species that can be used in health food

| No. | Chinese name | English name |
|---|---|---|
| 1 | 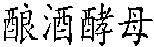 | Saccharomyces cerevisiae |
| 2 | 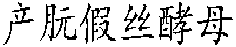 | Cadida atilis |
| 3 | 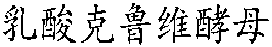 | Kluyveromyces lactis |
| 4 | 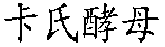 | Saccharomyces carlsbergensis |
| 5 | 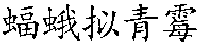 | Paecilomyces hepiali Chen et Dai, sp. Nov |
| 6 | 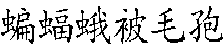 | Hirsutella hepiali Chen et Shen |
| 7 | 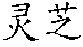 | Ganoderma lucidum |
| 8 | 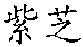 | Ganoderma sinensis |
| 9 | 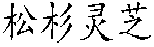 | Ganoderma tsugae |
| 10 | 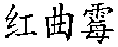 | Monacus anka |
| 11 | 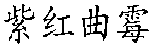 | Monacus purpureus |

**Wall material:** The microcapsule material used to coat, protect or control the release of the core material is called the wall material, capsule wall or capsule shell of the microcapsule. The wall material is the material that constitutes the outer shell of the microcapsule, and can also be called coating or packaging material.

When choosing a wall material, the properties of the wall material itself should be considered, such as permeability, stability, mechanical strength, solubility, polymerizability, electrical properties, hygroscopicity and film-forming properties, etc. For the core material of biologically active materials, it is also important to consider the toxicity of the wall material and the compatibility with the core material. In this application, wall materials that are non-toxic, have good film- or sphere-forming properties, have low immunogenicity, have good biocompatibility, are degradable, and have a product that has no toxic and side effects are selected.

In this application, preferred are film-forming materials that have good biocompatibility with live bacteria, enteric materials that can be used in foods, film-forming materials with moisture barrier properties, and film-forming materials with oxygen barrier properties.

Preferably, specific examples include one or a combination of more of:
(1) Edible protein: including animal protein such as milk protein, egg protein, casein, vegetable protein such as gluten protein, etc.;
   Preferably, the wall material of probiotic microcapsules of the present application is selected from whey protein:
   including whey protein concentrate (WPC), whey protein isolate (WPI) or whey protein peptides, wherein whey protein isolate (WPI) is especially preferred. WPI is a high-purity whey protein obtained by further processing on the basis of whey protein concentrate (WPC). The purity of WPI can reach more than 90%, and WPI is more easily digested and absorbed, safe, non-toxic and free of side effects.

Milk protein: including casein or whey protein. Whey protein refers to the protein dissolved and dispersed in whey, accounting for about 18% to 20% of milk protein, and can be divided into two parts: heat stable and heat unstable whey protein.

### Whey Protein Concentrate (WPC)

After the whey is directly dried, whey powder can be obtained, in which the content of whey protein is extremely low, generally between ten percent and twenty percent, not more than 30 percent. The product of whey after clarification, ultrafiltration, drying and other processes is concentrated whey protein. The different degree of filtration can obtain products with protein concentration ranging from 34-80%.

### Whey Protein Isolate (WPI)

Whey protein isolate is a high-purity whey protein obtained by further processing on the basis of concentrated whey protein, with a purity of more than 90%. It is expensive, 2-3 times that of whey protein concentrate, but it is also easier to be digested and absorbed. The real beauty of whey protein isolate lies in its nutritional value. It has a high content of high-quality protein, which can provide high-quality protein for certain people with specific needs, such as infants and hospitalized patients. In addition, the biologically active compounds contained in whey protein isolate, such as α-lactalbumin and β-lactoglobulin, lactoferrin and immunoglobulin, have injected fresh vitality into the market.

### Whey Protein Peptide

Whey protein peptide is a hydrolysate of whey protein and the essence of whey protein. It can participate in the process of muscle synthesis faster in the body.

Preferably, the wall material of probiotic microcapsules of the present application is selected from one or a combination of more of:
Vegetable protein: including oilseed protein, bean protein, gluten protein, etc.,
wherein oilseed protein includes: peanut protein, sesame protein, rapeseed protein, sunflower protein, cotton seed protein, safflower protein, coconut protein, etc.;
wherein bean protein includes: soy protein, broad bean protein, pea protein, mung bean protein, adzuki bean protein, kidney bean protein, etc.;
wherein gluten protein includes: rice (indica rice, japonica rice, glutinous rice) protein, wheat (wheat, barley, oats, rye) protein, corn protein, sorghum protein, millet protein, broomcorn millet protein, yellow rice protein, buckwheat protein, etc., and potato protein: including sweet potato protein, potato protein, yam protein, taro protein, tapioca protein, etc.;
wherein corn protein includes zein, corn germ protein, etc.;
preferably, the wall material of probiotic microcapsules of the present application is selected from corn protein, including zein and corn germ protein, and zein is especially preferred.

### (2) Grease:

In this application, fats and oils are collectively referred to as greases, which are esters formed by aliphatic carboxylic acids and glycerin. Those that are liquid at room temperature are called oils, and those that are solid or semi-solid are called fats. Most natural greases are mixed glycerides. Greases obtained from plant seeds are mostly oil, and greases obtained from animals are mostly fat.

In this application, according to the types of fatty acids, various natural fatty acid molecules are straight-chain fatty acids composed of different carbon chains (4-24 C). With some exceptions, all carbon atoms are in double numbers. There are two classifications of such fatty acids: one is to divide fatty acids into short-chain (4-6C), medium-chain (8-12C) and long-chain (above 12C) fatty acids based on the number of carbon atoms. The other is to divide fatty acids into saturated and unsaturated fatty acids. The general molecular formula of saturated fatty acids is C ₙ H ₂ₙ O ₂, while unsaturated fatty acids have 1, 2, 3 or more double bonds, and their general molecular formula is C ₙ H ₂ₙ₋₂ O ₂, CnH ₂ₙ₋₄ O ₂, or CnH ₂ₙ₋₆ O ₂. Linoleic acid, linolenic acid and arachidonic acid with more than two double bonds are called polyunsaturated fatty acids. In addition to the straight-chain fatty acids, there are also cyclic fatty acids, the maple seed oleic acid and ata maple seed oleic acid in maple seed oil.

The fatty acids in the greases in this application are mostly saturated or unsaturated fatty acids with even number of carbon atoms. Commonly used are saturated acids such as myristic acid (C14), palmitic acid (C16), and stearic acid (C18), and unsaturated acids such as palmitoleic acid (C16, monoene), oleic acid (C18, monoene), linoleic acid (C18, diene), and linolenic acid (C18, triene). Certain greases contain several special fatty acids, such as tung oleic acid in tung oil, rape acid in rape oil, ricinoleic acid in castor oil, and citrus acid in coconut oil.

According to the source, fat is divided into animal fat and vegetable fat. There are two major types of animal fats, one is aquatic animal fats, such as fish, shrimp, and seals, and the other is terrestrial animal fats, most of which contain saturated fatty acids and a relatively small amount of unsaturated fatty acids. Vegetable fats, such as cottonseed oil, peanut oil, rapeseed oil, soybean oil, etc., mainly contain unsaturated fatty acids, and the content of polyunsaturated fatty acids (linoleic acid) is very high, accounting for 40-50% of the total fat. But the fatty acids in coconut oil are mainly saturated fatty acids.

Medium chain triglyceride (MCT): a triglyceride composed of caproic acid (C6) to lauric acid (C12), especially a structural lipid in which the fatty acid in its composition is a fatty acid with a six-carbon chain to a twelve-carbon chain such as caprylic acid and capric acid. Natural MCT is mainly derived from vegetable oils such as coconut oil and palm oil. Triglycerides that are lower than caproic acid are short-chain fatty acid triglycerides (SCT), and those that are higher than lauric acid are called long-chain fatty acid triglycerides (LCT).

Palm oil, also called as maripa oil: including: crude palm oil (CPO), palm meal (PE), crude palm kernel oil (CPKO), palm kernel meal (PKE), refined palm oil (RBD PO), palm oil salad oil (RBD PKO), palmitoleic acid (PFAD), palm oil (abbreviated as OLEAN), palm stearin (abbreviated as STEARINE or ST), etc. Palm oil is semi-solid at room temperature, and its consistency and melting point largely depend on the content of free fatty acids. Palm oil with low acid value is often called soft oil, and oil with high acid value is called hard oil.

The wall material of probiotic microcapsules of the present application is preferably selected from palm oil, particularly preferably about 33°C palm oil, about 40°C palm oil, about 44°C palm oil, about 52°C palm oil, about 58°C palm oil, more preferably palm oil having a melting point above about 40°C, especially palm oil having a melting point of about 40-50°C, such as palm oil having a melting point of about 40°C.

Preferably, the wall material of probiotic microcapsules of the present application is selected from one or a combination of more of:
Vegetable oil: including cocoa butter substitute, cocoa butter, rapeseed oil, soybean oil, corn oil, peanut oil, cottonseed oil, sunflower oil, palm oil (solid palm oil or liquid palm oil), palm kernel oil, coconut oil, etc.; or
Animal oil: lard, tallow, fish oil, milk fat, mutton fat, etc.

Preferably, the wall material of probiotic microcapsules of the present application is selected from fats with a melting point above about 40°C, especially fats with a melting point of about 40-50°C, such as palm oil or MCT with a melting point of about 40°C.

Particularly preferred is one or a combination of more of: medium chain triglyceride (MCT), cocoa butter substitute, palm oil, lecithin, palm oil monoglyceride, and hydrogenated fat (e.g., hydrogenated palm oil, hardened oil, hydrogenated soybean oil), etc., preferably with a melting point of about 40°C or higher, and particularly preferably with a melting point of about 40-50°C.

### (3) Other materials:

Including: one or a combination of more of sodium alginate, shellac, CMC-Na, gellan, xanthan gum, k-carrageenan, cellulose acetate phthalate, maltodextrin, starch, dextrin, sucrose, lactose, dextran, corn syrup, pectin, gum arabic, chitosan, acetylated mono- or di-glyceride fatty acid ester, konjac gum, carrageenan, wax or gelatin.

Enteric wall material: Enteric solubility refers to a process in which microcapsules pass through the stomach environment without being destroyed or degraded, and finally enter the intestine, where the wall material is disintegrated, dissolved or degraded, resulting in the release of the core material inside the capsule. The enteric wall material can be a single wall material or a composite wall material, including shellac, pectin, sodium alginate, monoglycerides, triglycerides, hydrogenated fats (e.g., palm oil, hydrogenated palm oil, hardened oil, hydrogenated soybean oil, etc.).

Preferably, the wall material of probiotic microcapsules of the present application has enteric properties.

### Composite wall material

The composite wall material of the present application refers to a wall material with better performance by mixing two or more of the aforementioned wall materials. The composite wall material of the present application has a good synergistic effect, which is beneficial to improve the stability and compactness of the microcapsules containing these components in the wall material, improve the quality of the microcapsule products, and promote the application of the microcapsule technology in various industries.

**Hydrocolloid:** it usually refers to a macromolecular substance that can be dissolved in water and fully hydrated under certain conditions to form a viscous, slippery or jelly solution. It is widely used in food, medicine, chemical industry and many other fields. According to the source, hydrocolloids can be divided into: plant secretions, such as gum, guar gum, gum arabic, etc.; microbial fermentation products and metabolites, such as xanthan gum, gellan gum, etc.; seaweed extracts, such as carrageenan, agar, alginate, etc.

**Pectin:** it is a natural polysaccharide polymer compound extracted from plant cell walls. Citrus peel, apple paste, beet pulp, etc. are the most common raw materials for extracting pectin. According to the ratio of esterified galacturonic acid groups in the pectin molecule, it can be divided into high methoxy pectin (including high methoxy water-soluble tomato pectin and high methoxy citrus pectin, etc.) and low methoxy pectin (a degree of esterification of 50% is used as a distinguishing value).

**Curing** agent: The curing agent in this application includes at least one of sodium acetate, glacial acetic acid, citric acid, sodium citrate, calcium salt, and surfactant.

The curing agent is preferably a mixture of sodium acetate, glacial acetic acid and surfactant, so that protein can form an irreversible chemical gel under the combined action of non-covalent bonds and covalent bonds (e.g., disulfide bonds), and the action of surfactants is combined to increase the gel strength and stability of protein particles in the dispersed phase.

Preferably, the curing agent has a concentration of about 0.5 mol/L and a pH of about 4.5 to about 5.3, and contains a surfactant that accounts for about 0.01% to 0.1% of the volume of the curing solution solution.

**Binder:** it includes starch, modified starch, compressible starch, dextrin, lactose, animal gums such as gelatin, gum arabic, sucrose, tragacanth, liquid glucose, shellac, peel gum, rosin, hydroxymethyl cellulose sodium, methyl cellulose, povidone, hydroxypropyl cellulose, ethyl cellulose, hydroxypropyl methyl cellulose, polyethylene glycol, microcrystalline cellulose, inorganic salt, etc. The preferred binder used for probiotic microcapsules in the present application is starch, and the preferred binder concentration is 5%-20%.

### DETAILED DESCRIPTION OF THE INVENTION

### Microcapsule particles

A microcapsule with one or more layers of embedding structure, comprising: a pellet core and optionally at least one layer of shell coating the pellet core;
wherein the pellet core includes an embedded substance and a microcapsule core material, and
the at least one layer of shell coating the pellet core includes one, two or more layers of wall material.

In one embodiment, the outer diameter of the pellet core of the microcapsule is about 50-500 µm (preferably about 50 to about 300 µm), and the outer diameter of the single-layered or multi-layered coated microcapsules is about 200 to about 1000 µm (preferably about 100 to about 500 µm).

In one embodiment, the embedded substance includes a functional active substance selected from one or more of functional polysaccharides, functional lipids, functional proteins/peptides/amino acids, micro-ecological regulators, vitamins and minerals.

Preferably, the weight ratio of the embedded substance to the microcapsule core material is 1:6-1:2.5 (more preferably 1:5-1:4).

In one embodiment, the functional polysaccharide is selected from one or more of chitosan, tea polysaccharide, dietary fiber, and dextran; preferably, the functional lipid is selected from one or more of lecithin, EPA and DHA; preferably, the functional protein/peptide/amino acid is selected from one or more of taurine, lactoferrin, immunoglobulin, and whey protein peptide; preferably, the micro-ecological regulator is selected from one or more of probiotics, prebiotics, and synbiotics.

In one embodiment, the wall material is selected from one or a combination of more of:
Vegetable protein, such as soy protein, rice protein, wheat protein, corn protein, etc.; preferably corn protein, or
Animal protein, such as whey protein, casein, etc.; preferably whey protein concentrate (WPC), whey protein isolate (WPI) or whey protein peptide, especially preferably whey protein isolate (WPI); or
Greases, such as greases with a melting point of 40°C or higher, preferably with a melting point of 40-50°C, particularly preferably palm oil, medium chain glycerides, hydrogenated greases (e.g., hydrogenated palm oil, hardened oil, hydrogenated soybean oil), lecithin, cocoa butter substitutes, palm oil monoglycerides, coconut oil, soybean oil, peanut oil, sunflower oil, or
Other materials, such as glycerin, oleic acid, sodium alginate, shellac, CMC-Na, gellan, xanthan gum, k-carrageenan, cellulose acetate phthalate, maltodextrin, starch, dextrin, sucrose, lactose, dextran, corn syrup, pectin, gum arabic, chitosan, acetylated mono- or di-glyceride fatty acid esters, konjac gum, carrageenan, wax or gelatin, etc.

Preferably, the wall material is selected from whey protein or grease, particularly preferably WPI or MCT.

In one embodiment, the microcapsule core material includes one or a combination of more of:
Vegetable protein, such as soy protein, rice protein, wheat protein, corn protein, etc.; preferably corn protein, or
Animal protein, such as whey protein, casein, etc.; preferably whey protein concentrate (WPC), whey protein isolate (WPI) or whey protein peptide, especially whey protein isolate (WPI); or
Greases, such as greases with a melting point of 40°C or higher, preferably with a melting point of 40-50°C, particularly preferably palm oil, medium chain glycerides, and hydrogenated greases (e.g., hydrogenated palm oil, hardened oil, hydrogenated soybean oil), Lecithin, cocoa butter substitutes, palm oil monoglycerides, coconut oil, soybean oil, peanut oil, sunflower oil, or
Other materials, such as microcrystalline cellulose (MCC), glycerin, oleic acid, sodium alginate, shellac, CMC-Na, gellan, xanthan gum, k-carrageenan, cellulose acetate phthalate, maltodextrin, starch, dextrin, sucrose, lactose, dextran, corn syrup, pectin, gum arabic, chitosan, acetylated mono- or di-glyceride fatty acid esters, konjac gum, carrageenan, wax or gelatin, etc.

More preferably, the microcapsule core material includes microcrystalline cellulose (MCC).

In one embodiment, the embedded substance includes probiotics;
Preferably, the probiotics are selected from one or more of Bifidobacterium adolescentis, Bifidobacterium animalis (Bifidobacterium lactis), Bifidobacterium bifidum, Bifidobacterium breve, Bifidobacterium infantis, Bifidobacterium longum, Lactobacillus acidophilus, Lactobacillus casei, Lactobacillus crispatus, Lactobacillus delbrueckii subsp. Bulgaricus, Lactobacillus delbrueckii subsp. lactis, Lactobacillus fermentum, Lactobacillus gasseri, Lactobacillus helveticus, Lactobacillus johnsonii, Lactobacillus paracasei, Lactobacillus plantarum, Lactobacillus reuteri, Lactobacillus rhamnosus, Lactobacillus salivarius, Streptococcus thermophilus, and Bacillus coagulans.

Preferably, the microcapsule particles of the present application are (light) yellow spherical particles with a particle size of 300-500 microns. It is mainly composed of three parts: a primary pellet core, a water-blocking coating layer and a hydrophilic coating layer, wherein the number of embedding layers can be selected according to the characteristics of the embedded substance, and is at least one. The multi-layered embedded microcapsule particles are mainly for the purpose of being not affected by an environment of the product when the embedded substance is added to the product, and not affecting the product. At the same time, for some functional substances (e.g., probiotics) that function in the human small intestine, the microcapsules can promote their sustained release.

The primary pellet core is mainly composed of an embedded functional (active) substance and a microcapsule core material. The main function of this structure is to make the embedded substance into spherical particles, so that the subsequent water-blocking coating layer and hydrophilic coating layer can be better coated outside the pellet core to form uniform spherical particles. The embedded substance may be a hydrophilic or hydrophobic functional component (for example: functional protein, functional grease, vitamin, probiotics, etc.). The embedded substance is mixed with the microcapsule core material in a ratio of (1:3-4:1) according to its characteristics (e.g., viscosity, etc.). The core material of the microcapsules is mainly microcrystalline cellulose (MCC), which can be optionally added or replaced with sucrose, lactose, chitosan, etc. The embedded substance and the microcapsule core material are uniformly mixed to form a 250-450 µm primary pellet core.

The main function of the water-blocking coating layer is to block the embedded substance from the moisture in the product (or environment), and to protect the embedded substance from being affected by gastric acid so that the embedded substance can reach the human intestine. The water-blocking coating layer is mainly based on prolamin, and substances (including glycerin, oleic acid, etc.) that can increase the plasticity and toughness of prolamin are added at a ratio of 3%-10% to improve the performance of the water-blocking coating layer. substance. During coating, the prolamin can be dissolved by 50-75% ethanol, and the specific ratio is affected by the tolerance of the embedded substance to ethanol. The number of water-blocking coating layers can be determined according to the affected degree of the embedded substance by water.

The outermost layer of the microcapsule is a hydrophilic coating layer. The main function of this layer is that the microcapsule particles can be uniformly added to an aqueous product without agglomeration, thereby ensuring the uniformity of addition of microcapsule particles in the product. This layer is mainly composed of hydrophilic polysaccharides with film-forming properties as embedding wall materials, such as pectin, soybean polysaccharides or whey protein isolate.

### Preparation method of microcapsule particles

A method for preparing microcapsules having one or more layers of embedding structure, comprising the following steps:
(1) preparation of a primary pellet core: uniformly mixing an embedded substance and a microcapsule core material, optionally curing, and filtering to collect wet particles, and then drying to obtain a primary pellet core; and optionally
(2) preparation of multi-layered microcapsules: uniformly coating a layer of wall material outside the pellet core particles, and optionally spraying curing solution to cure a capsule wall, drying the prepared microcapsules, collecting dry particles of microcapsules or further coating them once or more times.

In one embodiment, the step (1) is to prepare pellet cores by extrusion spheronization granulation method, centrifugal granulation method, acute angle extrusion granulation method, or fluidized bed spray granulation method (preferably extrusion spheronization granulation method or centrifugal granulation method), and dry and collect the obtained pellet cores.

Preferably, the step (2) is to coat the wall material by means of fluidized bed spray.

Regarding the preparation of probiotic microcapsule particles, in order to achieve the objectives of the above-mentioned disclosure, a preferred technical solution is:
a preparation method of probiotic microcapsules, comprising the following steps: primary core making; and secondary fluidization.

In the method, the scheme of primary core making is as described in 1 or 1' below. The scheme of secondary fluidization is as described in 2.

### 1. Primary core making (extrusion spheronization granulation) scheme

The raw materials include (based on 1000 parts by weight of dry microcapsule particles): 10-30 parts by weight of probiotic mud or powder, 90-70 parts by weight of microcrystalline cellulose (MCC), 100 parts by weight of water (or a mixed solution of water and ethanol), and optional sucrose, lactose, chitosan, etc.

Preparation:
Probiotic powder/bacteria mud and microcrystalline cellulose are uniformly mixed and added to a wet granulation pot for pre-mixing for 10 minutes;
A cutting knife is turned on, a peristaltic pump is turned on at 20-30 r/min, water consumption is 13.5-14 kg, and time consumption is about 30mins;
A soft material with a water content of 49.7% is obtained;
A granulator is started, the number of extrusion revolutions is adjusted, the soft material is added, a strip-shaped soft material is extruded through an orifice plate and collected on a receiving tray. The extrusion motor is stopped, the spheronization rotation number is adjusted, the spheronization motor is started, and extruded strips of soft material are poured into a spheronization cylinder at one time and spheronized;
After the spheronization is completed, the pellets are dried in a fluidized bed or oven, and the drying temperature is 45-50°C.

In the above steps, if the probiotic powder and microcrystalline cellulose are not sufficiently mixed, the distribution of bacteria in the pellet cores produced later would be uneven, and the difference in the number of bacteria in the microcapsule particles produced later would be too large. If the feeding speed and the extrusion speed do not match, suitable strips cannot be extruded, and subsequent granulation cannot be performed. If the spheronization speed is not appropriate, a regular spherical pellet core cannot be formed, and a cylindrical or elliptical pellet core may be formed, which is not conducive to the subsequent fluidization of the water blocking layer and the addition of the product.

### 1', primary core making (centrifugal granulation) scheme

Raw materials: 10-30 parts by weight of probiotic mud or powder, 90-70 parts by weight of microcrystalline cellulose (MCC), 27-21 parts by weight of water (or hydroxypropyl methylcellulose (HPMC)), and optional sucrose, lactose, chitosan, etc.

Preparation:
The turntable of a centrifugal granulator is turned on, the probiotic powder and microcrystalline cellulose are premixed and put into the centrifugal granulator with a rotating speed of 300 revolutions/min;
Water or HPMC is added, and the centrifugal granulator is started to spray a slurry;
After the particles have risen to 100-200 microns, a mixed powder of probiotic powder and MCC is sprinkled to make the particles rise to about 300 microns. The slurry spraying is stopped, the speed of the turntable is adjusted to 700 rpm, and the particles are spheronized;
After the spheronization is completed, the pellets are dried in a fluidized bed, and the drying temperature is 45-50°C.

In the above steps, if the probiotic powder is not uniformly mixed with the microcrystalline cellulose before granulation, the bacterial count of the finished microcapsule particles will be uneven, resulting in the particles not reaching standardization. If the ratio of probiotic powder to microcrystalline cellulose is not appropriate, spherical particles cannot be rolled out in the centrifugal granulator, and they will remain in powder state or aggregate into agglomerates. If the ratio of binder to dry matter is not appropriate, spherical particles cannot be rolled out in the centrifugal granulator, and the particles will aggregate into agglomerates. If the rotating speed of the centrifugal turntable is not suitable, the primary pellet core of suitable size cannot be formed, and the pellet core may be too large or too small, which is not conducive to subsequent fluidization and product addition.

### 2. The basic plan of secondary fluidization:

Raw materials: water-blocking coating layer solution 1: 600-800 parts by weight of 75% alcohol, 150-180 parts by weight of prolamin, 70-100 parts by weight of oleic acid; water-blocking coating layer solution 2: 600-800 parts by weight of 75% alcohol, 150-180 parts by weight of prolamin, 30-50 parts by weight of oleic acid; optional water-blocking coating layer 3: medium-chain triglycerides (MCT) with 1.8% particle mass; hydrophilic coating layer solution: 95-97 parts by weight of water and 3-5 parts by weight of pectin (it can be replaced by other polysaccharides with film-forming properties).

The coating sequence of the water-blocking coating layers is not fixed; the hydrophilic coating layer is fluidized on the outermost layer of the particles.

Preparation:
MCT layer: the particles are infiltrated in MCT for 16 hours.

Other coating layers:
Using a fluidized bed spray granulation method, the coating solution is heated to 25 to 75°C, wherein the inlet air temperature of a fluidized bed is 60 to 80 °C, and the air flow is between 20 and 100.

The water-blocking coating solution is atomized by a spray gun and sprayed into a fluidized bed, so that droplets uniformly wrap the core particles obtained by the primary core making, and are dried in a fluidized bed to form particles with a single water blocking layer.

On this basis, a water-blocking coating layer solution can be sprayed uniformly outside the particles with a single water blocking layer to form particles with two or more water blocking layers.

Finally, the hydrophilic coating layer solution is sprayed outside the double-layered or multi-layered microcapsule particles.

The prepared microcapsules are dried in a fluidized bed. Each time about 50 kg of dried particles are fluidized. According to the degree of adhesion between the particles, after every 15-20 minutes of fluidization, the particles are finished by a finisher with a 40-mesh screen, and then the particles under the sieve are further fluidized, and the particles on the sieve are discarded.

Dry particles of multi-layered microcapsules are collected.

The key steps include: after fluidization of every 400 ml of coating solution, the particles are passed through a 50-80 mesh screen, and the particles larger than 80 mesh and smaller than 50 mesh are discarded.

In the above steps, if the inlet air temperature of the fluidized bed is too high, the entrapped material will lose its activity. If the inlet air temperature of the fluidized bed is too low, the particles will stick to each other. If the air flow of the fluidized bed is too low, the particles cannot be blown up in the fluidized bed and cannot be uniformly wrapped by the fluidizing wall material. If the air flow in the fluidized bed is too high, the particles will be blown onto the filter bag and stick together and cannot continue to be fluidized. If the atomization pressure is not suitable, the wall material cannot be uniformly wrapped on the pellet core. A batch of fluidization greater than 400mL will cause the adhered particles in the fluidized bed to be continuously adhered and affect the final yield. Therefore, after every 400 ml of fluidization, the adhered particles are separated in time through the screen.

Regarding the preparation of whey protein peptide microcapsule particles, in order to achieve the objective of the above-mentioned disclosure, the preferred technical solution is:
Raw materials: whey protein powder: 200-400 parts by weight; microcrystalline cellulose: 600-800 parts by weight; water-blocking coating layer solution 1: 600-800 parts by weight of 75% alcohol, 150-180 parts by weight of prolamin, 70-100 parts by weight of oleic acid; water-blocking coating layer solution 2: 600-800 parts by weight of 75% alcohol, 150-180 parts by weight of prolamin, and 30-50 parts by weight of oleic acid. Optional water-blocking coating layer 3: medium chain triglyceride (MCT) with 1.8% particle mass.

Hydrophilic coating layer solution: 95-97 parts by weight of water, 3-5 parts by weight of pectin (it can be replaced by other polysaccharides with film-forming properties).

Preparation:
The whey protein peptide powder and microcrystalline cellulose are uniformly mixed and added to a wet granulation pot for pre-mixing for 10 minutes.

The cutting knife is turned on, and the peristaltic pump is turned on, wherein the speed is 20-30 r/min, the water consumption is 13.5-14 kg, and the time is about 30 mins.

A soft material with a water content of 49.7% is obtained.

A granulator is started, the number of extrusion revolutions is adjusted, and the soft material is added. A strip-shaped soft material is extruded through the orifice plate and collected on a receiving tray. The extrusion motor is stopped, and the spheronization rotation number is adjusted. The spheronization motor is started, and the extruded strip-shaped soft materials are poured into a spheronization cylinder at one time and spheronized.

After the spheronization is completed, the pellets are dried in a fluidized bed or oven, and the drying temperature is 45-50°C.

MCT layer: the particles are infiltrated in MCT for 16 hours.

Other coating layers:
Using a fluidized bed spray granulation method, the coating solution is heated to 25 to 75°C, wherein the inlet air temperature of a fluidized bed is 60 to 80°C, and the air flow is between 20 and 100.

The water-blocking coating solution is atomized by a spray gun and sprayed into a fluidized bed, so that droplets uniformly wrap the core particles obtained by the primary core making, and are dried in a fluidized bed to form particles with a single water blocking layer.

On this basis, a water-blocking coating layer solution can be sprayed uniformly outside the particles with a single water blocking layer to form particles with two or more water blocking layers.

Finally, the hydrophilic coating layer solution is sprayed outside the double-layered or multi-layered microcapsule particles.

The prepared microcapsules are dried in a fluidized bed. Each time about 50 kg of dried particles are fluidized. According to the degree of adhesion between the particles, after every 15-20 minutes of fluidization, the particles are finished by a finisher with a 40-mesh screen, and then the particles under the sieve are further fluidized, and the particles on the sieve are discarded.

Dry particles of multi-layered microcapsules are collected.

### Probiotic microcapsules and preparation method thereof

The present application relates to a probiotic microcapsule. The probiotic microcapsule has one or more layers of embedding structure, including: probiotic core particles and optionally at least one layer of shell coating the probiotic core particles, wherein the probiotic core particles include a core material and a first layer of wall material, the core material includes one or more kinds of probiotic powder or probiotic mud, and the core material is coated by the first layer of wall material;

The at least one layer of shell coating the probiotic core particles includes one, two or more layers of wall materials, which are respectively a second layer of wall material, a third layer of wall material, or more layers of wall material.

Optionally, the above-mentioned probiotic core particles further include a binder.

Preferably, based on 1000 parts by weight of dry microcapsule particles, the weight ratio of the composition of the above microcapsules is: about 50 to 500 parts by weight of probiotic mud or powder; about 150 to 950 parts by weight of the first layer of wall material; about 0 to 350 parts by weight of the second layer of wall material; about 0 to 350 parts by weight of the third layer of wall material; and about 0 to 260 parts by weight of the fourth layer of wall material.

Preferably, in the above-mentioned preparation method of probiotic microcapsules, based on 1000 parts by weight of dry microcapsule particles,
The probiotic mud or powder is about 50 to about 500 parts by weight, more preferably, about 100 parts by weight, about 150 parts by weight, about 200 parts by weight, about 250 parts by weight, about 300 parts by weight, about 350 parts by weight, about 400 parts by weight, about 450 parts by weight or any range therebetween, particularly preferably about 250 to about 400 parts by weight;
The first layer of wall material is about 150 to about 950 parts by weight, more preferably, about 200 parts by weight, about 250 parts by weight, about 300 parts by weight, about 350 parts by weight, about 400 parts by weight, about 450 parts by weight, about 500 parts by weight, about 550 parts by weight, about 600 parts by weight, about 650 parts by weight, about 700 parts by weight, about 750 parts by weight, about 800 parts by weight, about 850 parts by weight, about 900 parts by weight, or any range therebetween, particularly preferably about 450 to about 750 parts by weight;
The optional second layer of wall material is about 0 to about 350 parts by weight, more preferably, about 100 parts by weight, about 150 parts by weight, about 200 parts by weight, about 250 parts by weight, about 300 parts by weight or any range therebetween, especially preferably about 200 to about 300 parts by weight;
The optional third layer is about 0 to about 350 parts by weight, more preferably, about 100 parts by weight, about 150 parts by weight, about 200 parts by weight, about 250 parts by weight, about 260 parts by weight, about 300 parts by weight, or any range therebetween, especially preferably about 200 to about 300 parts by weight;
The optional fourth layer is about 0 to about 250 parts by weight, more preferably, about 100 parts by weight, about 150 parts by weight, about 200 parts by weight, about 250 parts by weight, about 260 parts by weight, about 250 parts by weight, or any range therebetween, especially preferably about 100 to about 250 parts by weight.

Preferably, the probiotics are selected from one or more of Bifidobacterium adolescentis, Bifidobacterium animalis (Bifidobacterium lactis), Bifidobacterium bifidum, Bifidobacterium breve, Bifidobacterium infantis, Bifidobacterium longum, Lactobacillus acidophilus, Lactobacillus casei, Lactobacillus crispatus, Lactobacillus delbrueckii subsp. Bulgaricus, Lactobacillus delbrueckii subsp. lactis, Lactobacillus fermentum, Lactobacillus gasseri, Lactobacillus helveticus, Lactobacillus johnsonii, Lactobacillus paracasei, Lactobacillus plantarum, Lactobacillus reuteri, Lactobacillus rhamnosus, Lactobacillus salivarius, Streptococcus thermophilus, and Bacillus coagulans.

In one embodiment, the wall material of the above-mentioned probiotic microcapsules is selected from one or a combination of more of edible protein, grease, or other materials.

Preferably, the above-mentioned edible protein is selected from one or a combination of more of animal protein or plant protein,

wherein the animal protein is any animal protein suitable for the probiotic microcapsules of the present application, such as one or a combination of more of any animal protein defined in the present application, preferably milk protein, egg protein, casein, etc., more preferably whey protein, including whey protein concentrate (WPC), whey protein isolate (WPI) or whey protein peptides, especially preferably whey protein isolate (WPI);
wherein the plant protein is any plant protein suitable for the probiotic microcapsules of the present application, for example, one or a combination of more of any plant protein defined in the present application, including oilseed protein, bean protein, gluten protein, etc.,
wherein the oilseed protein is preferably, peanut protein, sesame protein, rapeseed protein, sunflower protein, cotton seed protein, safflower protein, coconut protein, etc.;
wherein the bean protein is preferably, soy protein, broad bean protein, pea protein, mung bean protein, adzuki bean protein, kidney bean protein, etc.;
wherein the gluten protein is preferably, rice (indica rice, japonica rice, glutinous rice) protein, wheat (wheat, barley, oat, rye) protein, corn protein, sorghum protein, millet protein, broomcorn millet protein, yellow rice protein, buckwheat protein, etc., and potato protein: including sweet potato protein, potato protein, yam protein, taro protein, tapioca protein, etc.;
wherein the corn protein is preferably zein, corn germ protein, etc.

Preferably, the above grease is any grease suitable for the probiotic microcapsules of the present application, for example, one or a combination of more of the greases defined in the present application, preferably selected from one or a combination of more of:

Preferably, the wall material of probiotic microcapsules of the present application is selected from one or a combination of more of:
Vegetable oil: including cocoa butter substitute, cocoa butter, rapeseed oil, soybean oil, corn oil, peanut oil, cottonseed oil, sunflower oil, palm oil (solid palm oil or liquid palm oil), palm kernel oil, coconut oil, etc.; or
Animal oil: lard, tallow, fish oil, milk fat, mutton fat, etc.

Preferably, the wall material of probiotic microcapsules of the present application is selected from a grease with a melting point above 40°C, especially greases with a melting point of 40-50°C, such as palm oil or MCT with a melting point of 40°C.

Particularly preferred is one or a combination of more of: medium chain triglycerides (MCT), cocoa butter substitute, palm oil, lecithin, palm oil monoglycerides, hydrogenated greases (e.g., hydrogenated palm oil, hardened oil, hydrogenated soybean oil), etc., the melting point of which is preferably 40°C or higher, particularly preferably a melting point of 40-50°C.

More preferably, palm oil with a melting point of 40°C or higher, especially palm oil with a melting point of 40-50°C, such as palm oil with a melting point of 40°C.

Preferably, the above-mentioned other materials are selected from one or a combination of more of: sodium alginate, shellac, CMC-Na, gellan, xanthan gum, k-carrageenan, cellulose acetate phthalate, maltodextrin, starch, dextrin, sucrose, lactose, dextran, corn syrup, pectin, gum arabic, chitosan, acetylated mono- or di-glyceride fatty acid esters, konjac gum, carrageenan, wax or gelatin, etc.

In one embodiment, the above-mentioned probiotic microcapsules have one or more layers of embedding structure, including: probiotic core particles and a layer of shell coating the probiotic core particles, wherein the probiotic core particles include a core material and a first layer of wall material, wherein the core material includes one or more kinds of probiotic powder or probiotic mud, and is coated by the first layer of wall material, and the layer of shell coating the probiotic core particle includes a second layer of wall material, an optional third layer of wall material and an optional fourth layer of wall material; optionally, the above-mentioned probiotic core particles further include a binder.

In the embodiment, the above-mentioned first layer of wall material is grease or whey protein (WPI) or a combination thereof.

In one embodiment, the above-mentioned probiotic microcapsules have one or more layers of embedding structure, wherein the second layer of the wall material is WPI or grease or a combination thereof.

In one embodiment, the probiotic microcapsules described above have one or more layers of embedding structure, wherein the first layer of wall material is WPI, preferably with about 150 to about 950 parts by weight, more preferably about 300 to 750 parts by weight;
The optional second layer of wall material is WPI, preferablywith about 0-350 parts by weight, more preferably about 300-350 parts by weight; or
The optional second layer of wall material is grease, preferably with a melting point of 40-50°C, especially palm oil or MCT, preferably, with about 0-350 parts by weight, more preferably about 150, 200 or 300 parts by weight;
The optional third layer of wall material is WPI, preferably with about 0-350 parts by weight, more preferably about 250 or 260 parts by weight; or
The optional third layer of wall material is grease, preferably with a melting point of 40-50°C, especially palm oil or MCT, preferably, with about 0-350 parts by weight, more preferably about 150, 200 or 300 parts by weight;
The optional fourth layer of wall material is WPI, preferably with about 0 to 250 parts by weight, more preferably about 300 to 350 parts by weight.

In one embodiment, the wall material of the probiotic microcapsule described above is a composite wall material selected from a combination of multiple materials.

In one aspect of the present application, the present application also relates to a method for preparing probiotic microcapsules having one or more layers of embedding structure, including: probiotic core particles and optionally at least one layer of a shell coating the probiotic core particles, wherein the probiotic core particles include a core material and a first layer of wall material, wherein the core material includes one or more kinds of probiotic powder or probiotic mud, and is coated by the first layer of wall material, and the at least one layer of shell coating the core particles includes one, two or more layers of wall material, which are respectively the second layer of wall material, the third layer of wall material, or more layers of wall material;
Optionally, the above-mentioned probiotic core particles further include a binder;
The preparation of the probiotic microcapsules described above comprises the following steps:
   (1) preparation of single-layered microcapsules: optionally uniformly mixing probiotic powder or probiotic mud with the first wall material, optionally curing, and filtering to collect wet particles and drying to obtain dry particles of single-layered microcapsule; and optionally
   (2) preparation of multi-layered microcapsules: using the above-mentioned dry particles of single-layered microcapsule as probiotic core particles, and uniformly coating a layer of wall material outside the probiotic core particles (preferably by fluidized bed spray granulation method), and optionally spraying a curing solution to cure the capsule wall, drying the prepared microcapsules (preferably drying in a fluidized bed), collecting dry particles of the double-layered microcapsules or further coating them once or more times.

In one embodiment, in the above-mentioned preparation method of probiotic microcapsules, the probiotic microcapsules obtained by the above-mentioned method are based on 1000 parts by weight of dry microcapsule particles,
The probiotic mud or powder is about 50 to about 500 parts by weight, more preferably, about 50 parts by weight, about 100 parts by weight, about 150 parts by weight, about 200 parts by weight, about 250 parts by weight, about 300 parts by weight, about 350 parts by weight, about 400 parts by weight, about 450 parts by weight, about 500 parts by weight, or any range therebetween, particularly preferably about 250 to about 400 parts by weight;
The first layer of wall material is about 150 to about 950 parts by weight, more preferably, about 200 parts by weight, about 250 parts by weight, about 300 parts by weight, about 350 parts by weight, about 400 parts by weight, about 450 parts by weight, about 500 parts by weight, about 550 parts by weight, about 600 parts by weight, about 650 parts by weight, about 700 parts by weight, about 750 parts by weight, about 800 parts by weight, about 850 parts by weight, about 900 parts by weight, about 950 parts by weight, or any range therebetween, particularly preferably about 450 to about 750 parts by weight;
The optional second layer of wall material is about 0 to about 350 parts by weight, more preferably, about 100 parts by weight, about 150 parts by weight, about 200 parts by weight, about 250 parts by weight, about 300 parts by weight, about 350 parts by weight or any range therebetween, especially preferably about 200-300 parts by weight;
The optional third layer is about 0 to about 350 parts by weight, more preferably, about 100 parts by weight, about 150 parts by weight, about 200 parts by weight, about 250 parts by weight, about 260 parts by weight, about 300 parts by weight, about 350 parts by weight or any range therebetween, particularly preferably about 200 to about 300 parts by weight.

In one embodiment, in the above-mentioned preparation method of probiotic microcapsules, the ratio of probiotic powder or probiotic mud to the first wall material is about 1:1 to about 1:19, preferably about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, or any range therebetween, particularly preferably about 1:3 to 4:6.

In one embodiment, this application also relates to a method for preparing probiotic microcapsules having one or more layers of embedding structure, comprising the following steps:
(1) Coating of the first layer of microcapsules:
   a. Optionally, adding a binder to probiotic mud or powder for granulation;
   b. Optionally, pre-treating the first wall material:
      Mixing the first wall material with water, fully dissolving, and then carrying out alternate heating and cooling treatments to form a stable gel:
      (i) Preferably, the first wall material is well mixed with water, and stirred at a low temperature of about 2 to 8°C for about 4 to 16 hours, wherein the preferred rotation speed is about 170 to 240 rpm;
      (ii) More preferably, the first wall material is further subjected to heat treatment at about 75 to about 96°C (preferably about 75°C, about 76°C, about 77°C, about 78°C, about 79°C, about 80°C, about 81°C, about 82°C, about 83°C, about 84°C, about 85°C, about 86°C, about 87°C, about 88°C, about 89°C, about 90°C, about 91°C, about 92°C, about 93°C, about 94°C, about 95°C, about 96°C, or any range therebetween) for about 30 to about 180 minutes (preferably about 35, about 40, about 45, about 50, about 55, about 60, about 65, about 70, about 75, about 80, about 85, about 90, about 95, about 100, about 105, about 110, about 115, about 120, about 125, about 130, about 135, about 140, about 145, about 150, about 155, about 160, about 165, about 170, about 175 minutes, or any range therebetween), wherein the preferred rotation speed is 85 to 115 rpm/min;
      (iii) Preferably, cooling immediately at a cooling temperature of about -20 to about 4°C; and storing at about 4°C for about 12 to about 60 hours (preferably about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 51, about 52, about 53, about 54, about 55, about 56, about 57, about 58, about 59, about 60 hours, or any range therebetween) to obtain a gel solution of the first wall material;
      Preferably, the above-mentioned first wall material is WPI, preferably with about 150 to about 950 parts by weight, more preferably about 300 to 750 parts by weight;
   c. Uniformly wrapping droplets of the first wall material on probiotic powder, probiotic mud, or the particles obtained in step a above;
   d. Curing: uniformly wrapping droplets of a curing solution on the particles obtained in step c above;
   e. Drying;
   Preferably, the ratio of probiotic powder or probiotic mud to the first wall material is 2-10:8-16;
(2) Optional coating of the second layer of microcapsules:
   f. Using the dry particles of single-layered microcapsule obtained in step (1) as core particles and mixing with the second layer of wall material to prepare double-layered microcapsules. The second layer of wall material is preferably grease (preferably grease with a melting point of 40-50°C, especially palm oil or MCT) or whey protein, preferably with about 0-350 parts by weight;
   g. Drying the microcapsules made in step f and collecting the double-layered dry microcapsule particles;
(3) Optional third or more coating:
   j. Further coating the double-layered microcapsules obtained in step (2) for the third or more times according to the aforementioned method;
   The third wall material is preferably grease (preferably grease with a melting point of 40-50°C, especially palm oil or MCT) or whey protein, preferably with about 0-350 parts by weight.

Preferably, in the above-mentioned preparation method of probiotic microcapsules, the wet particles prepared in the step of (1) coating of the first layer of microcapsules have a particle size of ≤400um, preferably 50-300um, after being dried.

Preferably, in the step of (1) coating of the first layer of microcapsules, an extrusion granulation method is used.

Preferably, in the step of (1) coating of the first layer of microcapsules, a fluidized bed granulation method is used.

Preferably, in the above-mentioned method for preparing the probiotic microcapsules, in the step of (1) coating of the first layer of microcapsules, the curing agent is selected from one or a mixture of more of sodium acetate, citric acid, sodium citrate, calcium salt, glacial acetic acid and surfactant. Preferably, the curing agent is an acetic acid-sodium acetate buffer solution, and Tween-20 accounting for about 0.01% to about 0.1% of the volume of the curing agent solution is added. Preferably, the curing agent solution has a concentration of about 0.5 mol/L and a pH of about 4.5 to about 5.3.

### Preparation method (A) of probiotic microcapsules

The present disclosure also relates to a method for preparing probiotic microcapsules having one or more layers of embedding structure, comprising: probiotic core particles and optionally at least one layer of shell coating the probiotic core particles, wherein the core particles include a core material and a first layer of wall material, wherein the core material includes one or more kinds of probiotic powder or probiotic mud, and coated by the first layer of wall material, and the at least one layer of shell coating the core particles includes two or more layers of wall materials, which are respectively a second layer of wall material, a third layer of wall material or more layers of wall material, the method comprising the following steps:
(1) preparation of single-layered microcapsules: optionally mixing the probiotic powder or probiotic mud with a binder, and granulating, and then uniformly mixing with the first wall material (preferably **acute angle extrusion granulation method)** and optionally curing and filtering to collect wet particles and drying to obtain dry particles of single-layered microcapsule; and optionally
(2) preparation of multi-layered microcapsules: using the above-mentioned dry particles of single-layered microcapsule as core particles, and uniformly coating a layer of wall material outside the core particles (preferably by fluidized bed spray granulation method), and optionally sprayed a curing solution so that the capsule wall is cured and insoluble in water, and drying the prepared microcapsules (preferably dried in a fluidized bed), and collecting dry particles of double-layered microcapsules or further coating once or more times.

The present disclosure also relates to a method for preparing probiotic microcapsules having one or more layers of embedding structure, comprising the following steps:
(1) Coating of the first layer of microcapsules: fully mixing the first wall material with probiotic powder or mud, and then granulating, optionally adding a curing agent solution dropwise, and curing, filtering and collecting the microcapsule particles, and optionally drying the obtained microcapsules to obtain probiotic core particles; and optionally
(2) Coating of the second layer of microcapsules: uniformly mixing the microcapsules obtained in step (1) with a second wall material, preparing microcapsules by a fluidized bed spray method, drying the prepared microcapsules and collecting dry particles of the double-layered microcapsules;
(3) Optionally, further coating the microcapsules obtained in step (2) in a fluidized bed for a third or more times.

In one embodiment, in the above-mentioned preparation method of probiotic microcapsules, the first wall material is whey protein, and the whey protein is subjected to a pre-denaturation treatment: the whey protein is mixed with water, fully dissolved, and then subjected to alternate heat and cold treatments to form a stable gel.

Preferably, the whey protein is mixed uniformly with water, and stirred at a low temperature of 2 to 8°C for 4 to 16 hours, and the rotation speed is 170 to 240 per minute;
More preferably, the whey protein solution is further subjected to heat treatment at about 75 to about 96°C (preferably about 75°C, about 76°C, about 77°C, about 78°C, about 79°C, about 80°C, about 81°C, about 82°C, about 83°C, about 84°C, about 85°C, about 86°C, about 87°C, about 88°C, about 89°C, about 90°C, about 91°C, about 92°C, about 93°C, about 94°C, about 95°C, about 96°C, or any range therebetween) for about 30 to about 180 minutes (preferably about 35, about 40, about 45, about 50, about 55, about 60, about 65, about 70, about 75, about 80, about 85, about 90, about 95, about 100, about 105, about 110, about 115, about 120, about 125, about 130, about 135, about 140, about 145, about 150, about 155, about 160, about 165, about 170, about 175 minutes, or any range therebetween), and the rotation speed is 85 to 115 rpm/min;
Preferably, cooling immediately, wherein the cooling temperature is about -20 to about 4°C; and storing at about 4°C for about 12 to about 60 hours (preferably about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 51, about 52, about 53, about 54, about 55, about 56, about 57, about 58, about 59, about 60 hours, or any range therebetween) to obtain a whey protein gel solution.

Preferably, in the step of (1) coating of the first layer of microcapsules, an extrusion granulation method is used.

Preferably, in the step of (1) coating of the first layer of microcapsules, an acute angle extrusion granulation method is used to granulate to obtain microcapsules coated with whey protein.

The preferred parameters are: a pore diameter of an extrusion nozzle of about 150 to about 750 µm (preferably about 150, about 200, about 250, about 300, about 350, about 400, about 450, about 500, about 550, about 600, about 650, about 700, about 750µm or any range therebetween), a preferred nozzle of a combination of about 150 to about 200µm and about 300 to about 500µm, a voltage of about 1500 to about 1800mv (preferably about 1300, about 1400, about 1500, about 1600, about 1700, about 1800 mv or any range therebetween), a frequency of about 1300 to about 1500 Hz (preferably about 1300, about 1400, about 1500 or any range therebetween), and a pressure of about 290 to about 330 mbar.

Preferably, in the above-mentioned method for preparing the probiotic microcapsules, the curing agent in the step of (1) coating of the first layer of microcapsules is selected from one or a mixture of more of sodium acetate, citric acid, sodium citrate, calcium salt, glacial acetic acid and surfactant. Preferably, the curing agent is an acetic acid-sodium acetate buffer solution, and Tween-20 accounting for about 0.01% to about 0.1% of the volume of the curing agent solution is added. Preferably, the curing agent solution has a concentration of about 0.5 mol/L and a pH of about 4.5 to about 5.3.

Preferably, in the above-mentioned method for preparing the probiotic microcapsules, in the step of (2) coating of the second layer of microcapsules, the second layer of wall material is WPI or grease (preferably palm oil or MCT) or a composition thereof.

Preferably, in the above-mentioned method for preparing the probiotic microcapsules, in the step of (2) coating of the second layer of microcapsules, a fluidized bed method is used for the second coating, a whey protein gel solution heated to 25 to 75°C is preferably used, and the inlet air temperature of a fluidized bed is preferably 30 to 80°C.

Preferably, in the above-mentioned method for preparing the probiotic microcapsules, in the step of (2) coating of the second layer of microcapsules, the second layer of wall material is grease, wherein the grease is preferably palm oil or medium-chain triglyceride (MCT), and the microcapsules obtained in step (2) are further coated with WPI for the third time.

Preferably, in the above-mentioned double-layered or multi-layered probiotic microcapsules, the outer diameter of the core particles obtained after the first layer coating is about 50-500 µm (preferably about 50 to about 300 µm), and the outer diameter of the double-layered or multi-layered microcapsules is about 200 to about 1000 µm (preferably about 100 to about 500 µm).

### Preparation method (B) of probiotic microcapsules

The present disclosure relates to a preparation method of probiotic microcapsules having one or more layers of embedding structure, including: probiotic core particles and optionally at least one layer of shell coating the probiotic core particles, wherein the core particles include a core material and a first layer of wall material, wherein the core material includes one or more kinds of probiotic powder or probiotic mud, and is coated by the first layer of wall material, and the at least one layer of shell coating the probiotic core particles includes one, two or more layers of wall materials, which are respectively a second layer of wall material, a third layer of wall material or more layers of wall material, the method comprising the following steps:
(1) preparation of single-layered microcapsules: optionally mixing probiotic powder or probiotic mud with a binder, and granulating, and then uniformly mixing with the first wall material by a **fluidized bed spray granulation method,** and optionally curing, and filtering to collect wet particles, and drying to obtain dry particles of single-layered microcapsule; and optionally
(2) preparation of multi-layered microcapsules: using the above-mentioned dry particles of single-layered microcapsule as core particles, uniformly coating a layer of wall material outside the core particles by a fluidized bed spray granulation method, and optionally spraying a curing solution so that the capsule wall is cured and insoluble in water, drying the prepared microcapsules (preferably dried in a fluidized bed), and collecting dry particles of double-layered microcapsules or further coating once or more times.

The present disclosure also relates to a method for preparing probiotic microcapsules having one or more layers of embedding structure, comprising the following steps:
1. Microcapsule core material granulation: mixing probiotic powder or probiotic mud with a binding solution, and granulating by fluidized bed spray to form particles, which account for about 5-95% of the weight of the finished microcapsules;
   Preferably, the binding solution is prepared as follows:
   A binder is dissolved in warm water, fully dissolved, and then kept at about 80 °C with continuous stirring for about 1 hour. The concentration of the binder is about 5 to 25%, preferably about 5%, about 6%, about 7%, about 8 %, about 9%, about 10%, about 11%, about 12%, about 13%, about 14%, about 15%, about 16%, about 17%, about 18%, about 19%, about 20%, about 21%, about 22%, about 23%, about 24%, about 25%, or any range therebetween;
   Preferably, the temperature of the binder is about 30 to 80°C.
2. Sieving: selecting a standard sieve with the required mesh, preferably with a particle size of about 50-400 microns; and automatically or manually sieving out particles with the particle size distribution range as core particles of microcapsules for coating in the next step;
   Preferably, the undersize material can be returned to the fluidized bed for continue granulation, and the oversize material can be ground to make fine powder and then added back to the fluidized bed for continue granulation;
3. Preparation of single-layered microcapsules: putting the particles obtained by sieving into a fluidized bed and coating with a solution of the first layer of wall material by spraying; and optionally,
4. Preparation of multi-layered microcapsules: uniformly coating the second layer of wall material outside the single layered microcapsules, preferably by fluidized bed spray granulation method, and spraying a curing solution so that the capsule walls are cured and insoluble in water; drying the prepared microcapsules in the fluidized bed, and collecting dry particles of double-layered microcapsules or further coating the third layer of wall material or more layers of wall material in the fluidized bed.

Preferably, in the above-mentioned preparation method of probiotic microcapsules, based on 1000 parts by weight of dry particles of microcapsules,
The probiotic mud or powder is about 50 to about 500 parts by weight, more preferably, about 100 parts by weight, about 150 parts by weight, about 200 parts by weight, about 250 parts by weight, about 300 parts by weight, about 350 parts by weight, about 400 parts by weight, about 450 parts by weight or any range therebetween, particularly preferably about 250 to about 400 parts by weight;
The first layer of wall material is about 150 to about 950 parts by weight, more preferably, about 200 parts by weight, about 250 parts by weight, about 300 parts by weight, about 350 parts by weight, about 400 parts by weight, about 450 parts by weight, about 500 parts by weight, about 550 parts by weight, about 600 parts by weight, about 650 parts by weight, about 700 parts by weight, about 750 parts by weight, about 800 parts by weight, about 850 parts by weight, about 900 parts by weight, or any range therebetween, particularly preferably about 450 to about 750 parts by weight;
The optional second layer of wall material is about 0 to about 350 parts by weight, more preferably, about 100 parts by weight, about 150 parts by weight, about 200 parts by weight, about 250 parts by weight, about 300 parts by weight or any range therebetween, especially preferably about 200 to about 300 parts by weight;
The optional third layer is about 0 to about 350 parts by weight, more preferably, about 100 parts by weight, about 150 parts by weight, about 200 parts by weight, about 250 parts by weight, about 260 parts by weight, about 300 parts by weight, or any range therebetween, especially preferably about 200 to about 300 parts by weight;
The optional fourth layer is about 0 to about 250 parts by weight, more preferably, about 100 parts by weight, about 150 parts by weight, about 200 parts by weight, about 250 parts by weight, about 260 parts by weight, about 300 parts by weight, or any range therebetween, especially preferably about 100 to about 250 parts by weight.

Preferably, the above-mentioned probiotics are any probiotics suitable for the probiotic microcapsules of the present disclosure, such as the probiotics defined in the present disclosure, particularly preferably, one or more of Bifidobacterium adolescentis, Bifidobacterium animalis (Bifidobacterium lactis), Bifidobacterium bifidum, Bifidobacterium breve, Bifidobacterium infantis, Bifidobacterium longum, Lactobacillus acidophilus, Lactobacillus casei, Lactobacillus crispatus, Lactobacillus delbrueckii subsp. bulgaricus, Lactobacillus delbrueckii subsp. lactis, Lactobacillus fermentum, Lactobacillus gasseri, Lactobacillus helveticus, Lactobacillus johnsonii, Lactobacillus paracasei, Lactobacillus plantarum, Lactobacillus reuteri, Lactobacillus rhamnosus, Lactobacillus salivarius, Streptococcus thermophilus, Bacillus coagulans, etc.

In one embodiment, in the above-mentioned method for preparing the probiotic microcapsules, the first layer of wall material is protein (preferably WPI), the second layer of wall material is protein (preferably WPI), and the optional third layer of wall material is protein (preferably WPI) or grease;
Preferably, the above-mentioned grease is grease with a melting point of 40-50°C;
Particularly preferred is one or a combination of more of: medium chain triglyceride (MCT), cocoa butter substitute, cocoa butter, palm oil, lecithin, palm oil monoglyceride, hydrogenated grease (e.g., hydrogenated palm oil, hardened oil, hydrogenated soybean oil), etc.;
Preferably, the coating is carried out with grease, which is selected from any grease involved in the present disclosure, and in the above-mentioned preparation method of probiotics, the percentage of grease in the weight of the finished microcapsules is 0.25% to 50%.

The present disclosure relates to a preparation method of probiotic microcapsules having one or more layers of embedding structure, comprising the following steps:
(1) Fluidized bed granulation:
   a. Preparation of binding solution:
      A binder (preferably starch) is fully dissolved in warm water, and the mixture is kept at about 30-80°C with continuous stirring for about 1 hour, preferably wherein the temperature is kept at 30°C, 35°C, 40°C, 45°C, 50°C, 55°C, 60°C, 70°C, 75°C, or 80°C or any range therebetween;
      The preferred concentration is about 5-25%, preferably 5%, 6%, 7%, 8%, 9%, 10%, 11%, 12%, 13%, 14%, 15%, 16%, 17%, 18%, 19%, 20%, 21%, 22%, 23%, 24%, 25%, or any range therebetween;
   b. Fluidized bed spray granulation:
      The probiotic powder is put into a fluidized bed, and air inlet is activated to circulate the powder. The binding solution is atomized by a spray gun and sprayed into the fluidized bed, where droplets are combined with the powder to form particles;
      wherein
      The probiotic powder is about 50 to about 500 parts by weight, more preferably about 250 to about 400 parts by weight;
      The inlet air temperature of the fluidized bed is preferably about 30 to 80°C;
(2) Particle screening:
   c. Sieving: The powder in the fluidized bed is taken out, and particles between 50 and 400 microns are selected;
(3) First coating:
   d. The selected particles are put into a fluidized bed, and air inlet is activated to circulate the powder. The first wall material, preferably grease, is atomized by a spray gun and sprayed into a fluidized bed, so that the particles are uniformly wrapped by the wall material;
   wherein, preferably, the grease is palm oil or MCT, which is about 200 to about 350 parts by weight;
(4) Optional second coating:
   e. The second wall material is atomized by a spray gun and sprayed into a fluidized bed, so that the particles obtained in the above step are uniformly wrapped by the wall material;
   wherein, preferably, the second wall material is whey protein, preferably with 150 to 950 parts by weight; more preferably 300 to 750 parts by weight;
   Pre-denaturing treatment of the whey protein: the whey protein is mixed with water, fully dissolved, and then subjected to alternate cold and heat treatments to form a stable gel;
   Preferably, the whey protein is uniformly mixed with water, and stirred at a low temperature of about 2 to 8°C for about 4 to 16 hours, preferably at a rotation speed of 170 to 240 per minute;
   More preferably, the whey protein solution is further subjected to heat treatment at about 75 to about 96°C (preferably about 75°C, about 76°C, about 77°C, about 78°C, about 79°C, about 80°C, about 81°C, about 82°C, about 83°C, about 84°C, about 85°C, about 86°C, about 87°C, about 88°C, about 89°C, about 90°C, about 91°C, about 92°C, about 93°C, about 94°C, about 95°C, about 96°C, or any range therebetween) for about 30 to about 180 minutes (preferably about 35, about 40, about 45, about 50, about 55, about 60, about 65, about 70, about 75, about 80, about 85, about 90, about 95, about 100, about 105, about 110, about 115, about 120, about 125, about 130, about 135, about 140, about 145, about 150, about 155, about 160, about 165, about 170, about 175 minutes, or any range therebetween), and the preferred rotation speed is 85 to 115 rpm/min;
   Then, preferably, cooling immediately at a cooling temperature of about -20 to about 4°C; and storing at about 4°C for about 12 to about 60 hours (preferably about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 51, about 52, about 53, about 54, about 55, about 56, about 57, about 58, about 59, about 60 hours, or any range therebetween) to obtain a whey protein gel solution;
   Then, the resulting whey protein gel solution is heated to about 25 to 75°C (preferably about 25°C, about 30°C, about 35°C, about 40°C, about 45°C, about 50°C, about 60°C, about 65°C, about 70°C, about 75°C, or any range therebetween), atomized and sprayed into a fluidized bed by a spray gun, so that the particles obtained in step d described above are uniformly wrapped by droplets;
   f. Curing: A curing solution is atomized and sprayed into a fluidized bed by a spray gun, so that the particles obtained in step e described above are uniformly wrapped by droplets;
   g. Drying: the microcapsules prepared in step f are further dried in a fluidized bed for about 1-30 minutes, and then taken out when the moisture content of particles is less than 8%;
(5) Optional third or more coatings:
   h. The above-mentioned dry particles of double-layered microcapsule are collected or further coated with the third wall material or more wall materials in a fluidized bed.

### Soft particle of a probiotic microcapsule and preparation method thereof

A soft particle of a probiotic microcapsule comprising the probiotic microcapsule according to the above solution, which includes coconut fiber fruit soft particle of a probiotic microcapsule and gel ball soft particle of a probiotic microcapsule.

In one embodiment, based on the total weight of the soft particle of a probiotic microcapsule, the soft particle contains about 0.2-0.5% by weight of probiotic microcapsules.

In one embodiment, the soft particle of a probiotic microcapsule also contains one or more of coconut water, coconut milk, coconut jam, sucrose, white granulated sugar, High Fructose Syrup, Bacillus xylinus, acidity regulator, ethanol and water, preferably, wherein the content of sucrose is about 5-10%, the content of white granulated sugar is about 3-5%, the content of High Fructose Syrup is about 0-5%, the content of ammonium dihydrogen phosphate is about 0.3-0.8%, and the content of Bacillus xylinus is about 0.1-0.5%, and the content of acidity regulator is about 1-5%.

Alternatively, the gel ball soft particle of a probiotic microcapsule according to the above-mentioned solution also contains one or more of white granulated sugar, sodium alginate, xanthan gum, konjac gum, carrageenan, curing solution (containing calcium lactate, calcium chloride), probiotic microcapsule, pigment, and purified water; preferably, wherein the content of white granulated sugar is about 10-15%, the content of sodium alginate is about 0.5-1%, the content of xanthan gum is about 0.1-0.3%, the content of konjac gum is about 0.1-0.2%, and the concentration of calcium lactate in the curing solution is about 1-3%.

In one embodiment, the present disclosure also relates to a coconut fiber fruit soft particle of a probiotic microcapsule, which comprises the above-mentioned probiotic microcapsule described herein, wherein based on the total weight of the coconut fiber fruit soft particle containing the probiotic microcapsule, the particle contains about 0.2-0.5% by weight of probiotic microcapsules, and also contains one or more of coconut water, coconut milk, coconut jam, sucrose, white granulated sugar, High Fructose Syrup, Bacillus xylinus, acidity regulator, ethanol and water, preferably, wherein the content of sucrose is about 5-10%, the content of white granulated sugar is about 3-5%, the content of High Fructose Syrup is about 0-5%, the content of ammonium dihydrogen phosphate is about 0.3-0.8%, the content of Bacillus xylinus is about 0.1-0.5%, and the content of acidity regulator is about 1-5%.

In another embodiment, the present disclosure also relates to a gel ball soft particle of a probiotic microcapsule, which comprises the above-mentioned probiotic microcapsule described herein, wherein based on the total weight of the gel ball soft particle containing the probiotic microcapsule, the particle contains about 0.2 to 0.5% by weight of probiotic microcapsules, preferably, also contains one or more of white granulated sugar, sodium alginate, xanthan gum, konjac gum, carrageenan, curing solution (containing calcium lactate, calcium chloride), probiotic microcapsule, pigment, and purified water; preferably, wherein the content of white granulated sugar is about 10-15%, the content of sodium alginate is about 0.5-1%, the content of xanthan gum is about 0.1-0.3%, the content of konjac gum is about 0.1-0.2%, and the concentration of calcium lactate in the curing solution is about 1-3%.

### Food or health products containing probiotic microcapsules

In one embodiment, the present application also relates to a food or health product containing probiotic microcapsules, which comprises the above-mentioned probiotic microcapsules described herein.

Preferably, the food or health product is selected from one or more of dairy products, fermented flavored foods, beverages, chocolates, candies, baked foods or fruit and vegetable juice foods.

Preferably, in the food or health product containing probiotic microcapsules, based on the total weight of the food or health product containing probiotic microcapsules, the weight content of the probiotic microcapsules is 0.02 to 1%, preferably 0.04 to 0.5%, or more preferably 0.06 to 0.15%;
Preferably, the probiotic microcapsule-containing food or health product further contains honey, wherein the honey has a carrier function for added microcapsules. Preferably, based on the total weight of a mixture of microcapsule and honey, the content of honey is about 80%-99.9% by weight.

This application also relates to a food or health product containing probiotic microcapsules, wherein the food or health product containing probiotic microcapsules is yogurt, preferably, the yogurt also includes raw milk and/or reconstituted milk, stabilizer, acidity regulator, leavening agent. More preferably, the yogurt comprises: based on the total weight of the probiotic microcapsule-containing yogurt, about 89-94% of raw milk/compounded milk, about 5.5-10.9% of white granulated sugar, about 0.1-0.5% of stabilizer, about 30-100 U/ton of leavening agent, and about 0.02-0.5% (preferably 0.06-0.15%) of probiotic microcapsules.

In one embodiment, this application also relates to a food or health product containing probiotic microcapsules, wherein the food or health product containing probiotic microcapsules is cheese, preferably, wherein the cheese also includes raw milk, edible salt, leavening agent or chymosin; more preferably, the cheese contains about 0.8-1.8% of edible salt, about 0.001-0.005% of leavening agent, about 0.001-0.005% of chymosin, about 0.02-0.5% (preferably about 0.06-0.15%) of probiotic microcapsules, and the rest is raw milk.

In one embodiment, this application also relates to a method for preparing food or health products containing probiotic microcapsules, which comprises the following steps:
(1) Preparing probiotic microcapsules according to the above-mentioned preparation method of probiotic microcapsules;
(2) Adding the probiotic microcapsules to food or health products, preferably, at an amount of about 0.02-1%, preferably about 0.04-0.5%, more preferably 0.06- 0.15%.

In one embodiment, this application relates to a method for preparing a food or health product containing probiotic microcapsules, wherein the food or health product containing probiotic microcapsules is yogurt, and the yogurt is prepared according to a conventional method, wherein before final pasteurization, the probiotic microcapsules are added to the prepared product, or after final pasteurization, the probiotic microcapsules are sterilized and added to the prepared product.

Preferably, the steps of sterilization treatment include:
(1) Mixing microcapsule particles with honey, preferably, wherein the ratio of microcapsule to honey is about 1:1000 to 1:10, more preferably about 1:500 to 1:10, about 1:100 to 1:10 or about 1:50 to 1:10.
(2) Treating the above-mentioned mixed microcapsule particles with ultraviolet sterilization or hydrogen peroxide, preferably, wherein the intensity of ultraviolet is about 1000 to 20000 J/L, more preferably about 5000 to 20000 J/L, about 10000 to 20000 J/L, or about 15000 to 20000J/L.

In one embodiment, this application relates to a method for preparing a food or health product containing probiotic microcapsules, wherein the food or health product containing probiotic microcapsules is yogurt, and the yogurt is prepared as follows:
a. Optionally, implementing the national standard preparation process of fermented milk;
b. Mixing raw materials other than zymogenic strains, microcapsules, and honey to prepare a mixed solution of fermented milk, preferably at about 40-80°C, and then cooling, preferably to below about 20°C;
c. Stirring, homogenizing (homogenizing pressure is preferably about 150-200 bar), then carrying out high-temperature and long-term sterilization (preferably at a sterilization temperature of 95°C, and a sterilization time of 300 seconds), and cooling to 41-43°C after sterilization;
d. Inoculating bacteria and carrying out fermentation at a temperature of 41-43°C;
e. Demulsifying, turning over a tank, and then cooling to below 25°C;
f. Pasteurizing at a sterilization temperature of 74°C and at a sterilization time of 30 seconds;
g. Cooling to 15-30°C and entering an aseptic tank;
h. Aseptic filling;
   wherein after step e and before step f, the product obtained in step e is transferred to a tank to be filled, and probiotic microcapsule particles are added; and the mixture is stirred for 15 minutes; or
   wherein before step h and after step g, sterilized probiotic microcapsules are added; or
   after step h and before step i, sterilized probiotic microcapsules are added.

In one embodiment, this application relates to a method for preparing a food or health product containing probiotic microcapsules, wherein the food or health product containing probiotic microcapsules is cheese, and the cheese is prepared according to a conventional method, including:
a. Cleaning raw milk, standardizing, and pasteurizing;
b. Cooling the pasteurized raw milk to about 32 to 43°C, adding leavening agent, and maintaining the temperature for about 50 to 60min to pre-acidify;
c. Then adding chymosin, maintaining the temperature for about 40 to 50min, stirring and then allowing to stand while maintaining the temperature;
d. After standing for 90 to 180 min, cutting the mixture into small pieces and allowing to stand for 5 to 10min;
e. Gradually raising the temperature to about 36 to 45°C with stirring, and when the pH value is about 5.8 to 6.3, carrying out whey discharge treatment;
f. After the whey discharge treatment, piling and brewing the mixture, and when the pH value of the clot is about 5.5 to 5.9, putting into a mold.
g. Molding the mixture put into the mold, then demolding, and then immediately freezing to obtain the cheese.
wherein after the pasteurized raw milk is cooled to about 32-43°C in step b, sterilized probiotic microcapsules are added; or
wherein after step d and before step e, sterilized probiotic microcapsules are added.

In one embodiment, the present application relates to a method for preparing food or health products containing probiotic microcapsules, wherein the food or health products containing probiotic microcapsules are processed cheeses, and the processed cheeses are processed according to a conventional method, including:
a. Uniformly mixing skim powder, emulsified salt and a part of white granulated sugar, stirring and hydrating at 45°C for 30 minutes;
b. Adding premixed colloid and white granulated sugar to water, and stirring well until completely dissolved;
c. Melting mozzarella cheese, cream and butter;
d. Heating the colloidal solution to 95°C, maintaining the temperature for 3 minutes, adding the solution of skim powder, and then stirring for 2 minutes while maintaining the temperature;
e. Adding the solution to the melted cheese and making up to 1000kg;
f. Homogenizing: 15Mpa and 65-70°C;
g. Sterilizing: 90 to 95°C/300s;
h. Cooling down to 75°C, filling, cooling down, and refrigerating;
wherein after pasteurization in step g and before step h, sterilized probiotic microcapsules are added; or
Before step g and after step f, probiotic microcapsules are added.

In one embodiment, this application relates to a method for preparing a food or health product containing probiotic microcapsules, wherein the food or health product containing probiotic microcapsules is a milk beverage prepared according to a conventional method, including: 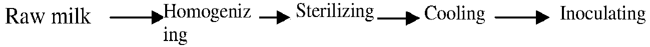 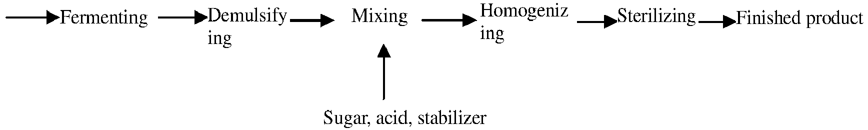 wherein probiotic capsules are added before the final sterilization, or sterilized probiotic microcapsules are added after the final sterilization.

In one embodiment, this application relates to a method for preparing a food or health product containing probiotic microcapsules, wherein the food or health product containing probiotic microcapsules is a milk beverage prepared according to a conventional method, including: 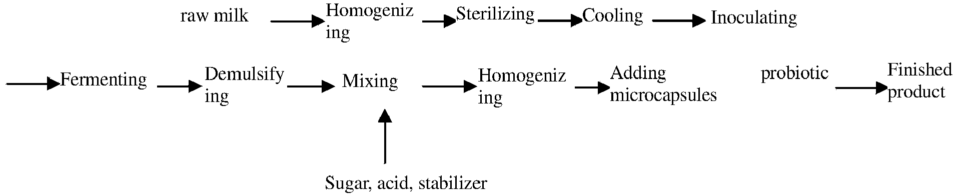 wherein sterilized probiotic microcapsules are added after the final homogenization.

In one embodiment, this application relates to a method for preparing a food or health product containing probiotic microcapsules, wherein the food or health product containing probiotic microcapsules is a solid dairy product prepared according to a conventional method, including: 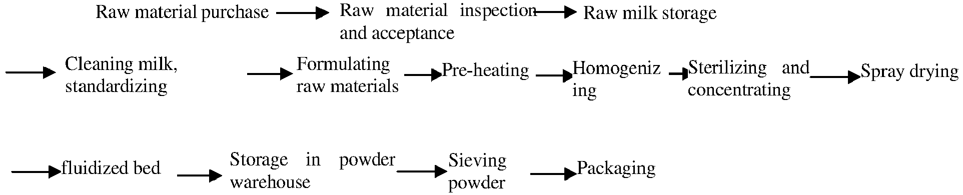 wherein probiotic microcapsules are added before the step of the fluidized bed.

In one embodiment, the present application relates to a method for preparing food or health products containing probiotic microcapsules, wherein the food or health products containing probiotic microcapsules are solid beverages prepared in a conventional method, including: 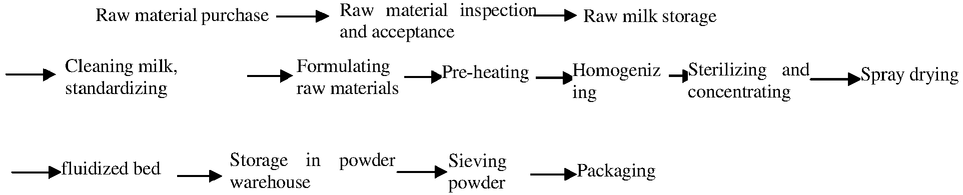 wherein probiotic microcapsules are added after the step of the fluidized bed.

In one embodiment, this application relates to a method for preparing a food or health product containing probiotic microcapsules, wherein the food or health product containing probiotic microcapsules is ice cream prepared in a conventional method, including:
White granulated sugar, additives and other accessories 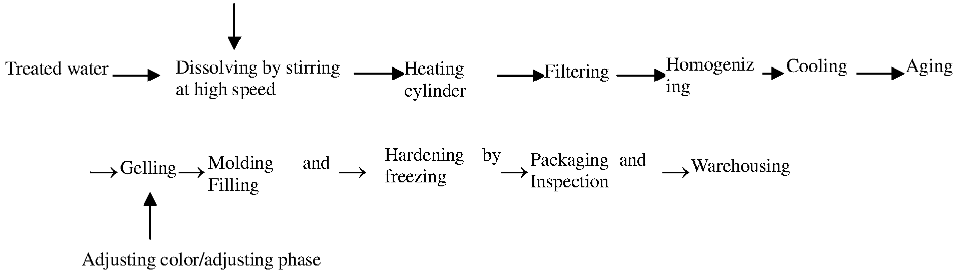 wherein probiotic capsules are added after the step of aging.

In short, in the above-mentioned technical method of adding probiotic microcapsules, the addition can be achieved by "pre-adding" or "post-adding".

It should be understood that modifications that do not substantially affect the effects of the various embodiments of this application are also included in the definition of the disclosure provided in this application. Therefore, the following examples are intended to illustrate rather than limit the present application.

### Example

### A. Preparation of probiotic microcapsules (I)

### Examples A1 to A3 and comparative examples

### Example A1

1. Raw materials (based on 1000g of dry microcapsule particles)
   750g of WPI (concentration 11%);
   6068g of water;
   250g of Bacillus coagulans powders;
   6818ml of a curing agent solution with a concentration of 0.5mol/L. The curing agent was a mixture of sodium acetate, glacial acetic acid and Tween-20. Sodium acetate had an amount of 137g, glacial acetic acid had an amount of 100ml, Tween-20 had an amount of 2.9g, and water had an amount of 6718g.
2. Preparation method
   a. The whey protein was uniformly mixed with water and stirred at 4°C at 180 rpm/min for 16 hours;
   b. The whey protein solution was heated at 78°C at 95 rpm/min for 45 minutes;
   c. The solution was cooled immediately at 0°C, and stored at 4°C for 12 hours;
   d. 137g of sodium acetate, 100ml of glacial acetic acid and 6718g of water were uniformly mixed to formulate a curing agent solution, and the curing agent solution was adjusted to a pH value of 4.6. 2.9g of Tween-20 was added, and the mixture was uniformly mixed for use;
   e. Granulation: the pretreated WPI described above and the probiotic powders were uniformly mixed and granulated;
   f. Curing: the droplets or particles obtained from the above granulation were formed into wet particles in the curing solution, and the curing time was at least 10 minutes;
   g. The cured wet particles were collected by filtration, and dried in a blast drying oven at 70°C;

The microcapsules obtained in this example were single-layered microcapsules. The amount of viable probiotics in the microcapsules reached 1.3×10¹⁰ cfu/g. The particles had a particle size of 300 µm. The particle size was uniform and the particles were shiny. After 30 days of storage at room temperature, the amount of viable probiotics in the microcapsules decreased to 9.2×10⁹cfu/g; after 60 days, the amount of viable probiotics in the microcapsules decreased to 7.7×10⁹cfu/g; after 120 days, the amount of viable probiotics in the microcapsules decreased to 4.8×10⁹cfu/g; and the number of the probiotic residue on the outer surface of the microcapsules was 2.3×10⁶cfu/g. After the single-layered microcapsules were incubated in a simulated gastric juice for 2 hours, the amount of contained viable probiotics decreased to 8.8×10⁹cfu/g, and the weight of the dry microcapsule particles increased by 19% after absorbing water for 48 hours.

### Comparative example A1

1. Raw materials (based on 1000g of dry microcapsule particles)
   750g of WPI (concentration 11%);
   6068g of water;
   250g of Bacillus coagulans powders;
   6818ml of a curing agent solution with a concentration of 0.5mol/L. The curing agent was a mixture of sodium acetate, glacial acetic acid and Tween-20. Sodium acetate had an amount of 137g, glacial acetic acid had an amount of 100ml, Tween-20 had an amount of 2.9g, and water had an amount of 6718g.
2. Preparation method
   a. The whey protein was uniformly mixed with water and stirred at 4°C at 180 rpm/min for 16 hours;
   b. The whey protein solution was heated at 78°C at 95 rpm/min for 45 minutes;
   c. The solution was cooled immediately at 0°C, and stored at 4°C for 4 hours;
   d. 137g of sodium acetate, 100ml of glacial acetic acid and 6718g of water were uniformly mixed to formulate a curing agent solution, and the curing agent solution was adjusted to a pH value of 4.6. 2.9g of Tween-20 was added, and the mixture was uniformly mixed for use;
   e. Granulation: the pretreated WPI described above and the probiotic powders were uniformly mixed and granulated;
   f. Curing: the droplets or particles obtained from the above granulation were formed into wet particles in the curing solution, and the curing time was at least 10 minutes;
   g. The cured wet particles were collected by filtration, and dried in a blast drying oven at 70°C;

The microcapsules obtained in this comparative example were single-layered microcapsules. The amount of viable probiotics in the microcapsules reached 4.8×10⁹cfu/g. The particle size was 330um. The particle size was uneven with tailing, and the gloss was not good. After 30 days of storage at room temperature, the amount of viable probiotics in the microcapsules decreased to 8.2×10⁸ cfu/g; after 60 days, the amount of viable probiotics in the microcapsules decreased to 6.1×10⁷ cfu/g; after 120 days, the amount of viable probiotics in the microcapsules decreased to 3.8×10⁶ cfu/g, and the number of the probiotic residue on the outer surface of the microcapsules was 3.6×10⁶ cfu/g. After the single-layered microcapsules were incubated in a simulated gastric juice for 2 hours, the amount of contained viable probiotics decreased to 4.1×10⁷ cfu/g, and the weight of the dry microcapsule particles increased by 65% after absorbing water for 48 hours.

### Example A2

1. Raw materials (based on 1000g of dry microcapsule particles)
   WPI (the first layer of wall material) 450g (concentration 11%);
   WPI (the second layer of wall material) 300g (concentration 11%);
   6068g of water, including 3641g for the first layer of wall material and 2427g for the second layer of wall material;
   250g of Bacillus coagulans powders;
   6818ml of a curing agent solution with a concentration of 0.5mol/L. The curing agent was a mixture of sodium acetate, glacial acetic acid and Tween-20. Sodium acetate had an amount of 137g, glacial acetic acid had an amount of 100ml, Tween-20 had an amount of 2.9g, and water had an amount of 6718g.
2. Preparation method
   a. The whey protein was uniformly mixed with water and stirred at 4°C at 180 rpm/min for 16 hours;
   b. The whey protein solution was heated at 78°C at 95 rpm/min for 45 minutes;
   c. The solution was cooled immediately at 0°C, and stored at 4°C for 12 hours;
   d. 137g of sodium acetate, 100ml of glacial acetic acid and 6718g of water were uniformly mixed to formulate a curing agent solution, and the curing agent solution was adjusted to a pH value of 4.6. 2.9g of Tween-20 was added, and the mixture was uniformly mixed for use;
   e. Granulation: the pretreated WPI described above and the probiotic powders were uniformly mixed and granulated;
   f. Curing: the droplets or particles obtained from the above granulation were formed into wet particles in the curing solution, and the curing time was at least 10 minutes;
   g. The cured wet particles were collected by filtration, and dried in a blast drying oven at 70°C;
   h. Fluidized bed spray granulation method: 300g of WPI was pretreated as described above to obtain a whey protein solution or gel solution. The whey protein solution or gel solution was heated to 50 °C, and mixed with the particles in step g to uniformly wrap the particles in step g. The inlet air temperature of the fluidized bed was 65°C, and the air flow was set according to the batch weight and fluidization state of the WPI. The particles were dried in a fluidized bed after curing.

The microcapsules obtained in this example were double-layered microcapsules. The amount of viable probiotics in the microcapsules reached 1.3×10¹⁰ cfu/g. The particle size was 330 µm. The particle size was uniform and the particles were shiny. After 30 days of storage at room temperature, the amount of viable probiotics in the microcapsules decreased to 9.8×10⁹ cfu/g; after 60 days, the amount of viable probiotics in the microcapsules decreased to 8.8×10⁹ cfu/g; after 120 days, the amount of viable probiotics in the microcapsules decreased to 7.2×10⁹ cfu/g, and the number of the probiotic residue on the outer surface of the microcapsules was 2.2×10⁴ cfu/g. After the double-layered microcapsules were incubated in a simulated gastric juice for 2 hours, the amount of contained viable probiotics decreased to 9.0×10⁹ cfu/g, and the weight of the dry microcapsule particles increased by 9% after absorbing water for 48 hours.

### Example A3

1. Raw materials (based on 1000g of dry microcapsule particles)
   WPI (the first layer of wall material) 300g (concentration 11%);
   MCT (the second layer of wall material) 200g (concentration 100%);
   WPI (the third layer of wall material) 250g (concentration 11%);
   4450g of water, including 2427g for the first layer of wall material and 2023g for the third layer of wall material;
   250g of Bacillus coagulans powders;
   5000ml of a curing agent solution with a concentration of 0.5mol/L. The curing agent was a mixture of sodium acetate, glacial acetic acid and Tween-20. Sodium acetate had an amount of 101g, glacial acetic acid had an amount of 74ml, Tween-20 had an amount of 2g, and water had an amount of 4926g.
2. Preparation method
   a. The whey protein was uniformly mixed with water and stirred at 4°C at 180 rpm/min for 16 hours;
   b. The whey protein solution was heated at 78°C at 95 rpm/min for 45 minutes;
   c. The solution was cooled immediately at 0°C, and stored at 4°C for 12 hours;
   d. 101g of sodium acetate, 74ml of glacial acetic acid and 4926g of water were uniformly mixed to formulate a curing agent solution. The curing agent solution was adjusted to a pH value of 4.6, and 2g of Tween-20 was added. The mixture was uniformly mixed for use.
   e. Granulation: the pretreated WPI described above and the probiotic powders were uniformly mixed and granulated;
   f. Curing: the droplets or particles obtained from the above granulation were formed into wet particles in the curing solution, and the curing time was at least 10 minutes;
   g. The cured wet particles were collected by filtration, and dried in a blast drying oven at 70°C;
   h. Fluidized bed spray granulation method:
      Spray coating of the second layer of grease film: 200g of the liquid MCT grease was heated to 55°C; the inlet air temperature of the fluidized bed was 40°C; the air flow was set according to the batch weight and fluidization state of the MCT solution; and particles were dried in a fluidized bed to obtain double-layered grease particles;
      Spray coating of the third layer of WPI: 250g of WPI was uniformly mixed with 2023g of water to make a whey protein solution (or a gel solution if necessary); the solution was heated to 50°C; the inlet air temperature of the fluidized bed was 40°C; the air flow was set according to the batch weight and fluidization state of the WPI solution; and the particles were dried in a fluidized bed after curing.

The microcapsules obtained in this example were three-layered microcapsules. The amount of viable probiotics in the microcapsules reached 1.3×10¹⁰ cfu/g. The particle size was 330 µm. The particle size was uniform and the particles were shiny. After 30 days of storage at room temperature, the amount of viable probiotics in the microcapsules decreased to 1.3×10¹⁰ cfu/g; after 60 days, the amount of viable probiotics in the microcapsules decreased to 9.8×10⁹ cfu/g; after 120 days, the amount of viable probiotics in the microcapsules decreased to 8.8×10⁹ cfu/g, and the number of the probiotic residue on the outer surface of the microcapsules was 1.2×10³ cfu/g. After the three-layered microcapsules were incubated in a simulated gastric juice for 2 hours, the amount of contained viable probiotics decreased to 1.0×10¹⁰ cfu/g, and the weight of the dry microcapsule particles increased by 1% after absorbing water for 48 hours.

### Examples A4 to A8 and comparative examples

### Example A4

1. Raw materials (based on 1000g of dry microcapsule particles)
   WPI: 550g, concentration 11%;
   Bacillus coagulans powder: 250g;
   Curing agent solution: 0.5mol/L, pH-4.6
   MCT: 200g
   Starch: 5% concentration
2. Preparation method
   a. The whey protein was uniformly mixed with water and stirred at 4°C at 180 rpm/min for 16 hours;
   b. The whey protein solution was heated at 78°C at 95 rpm/min for 45 minutes;
   c. The solution was cooled immediately at 0°C, and stored at 4°C for 12 hours;
   d. 137g of sodium acetate, 100ml of glacial acetic acid and 6718g of water were uniformly mixed to formulate a curing agent solution, and the curing agent solution was adjusted to a pH value of 4.6. 2.9g of Tween-20 was added, and the mixture was uniformly mixed for use;
   e. Granulation: the probiotic powder, binder solution and MCT were uniformly mixed to make grease droplets uniformly wrap particles;
   f. The whey protein solution was added to make droplets uniformly wrap the particles obtained in step e;
   g. Curing: droplets of the curing solution were used to uniformly wrap the particles obtained in step f;
   h. The microcapsules prepared in step g were dried.

The microcapsules obtained in this example were double-layered microcapsules. The amount of viable probiotics in the microcapsules reached 1.3×10¹⁰ cfu/g. The particle size was 150-250 µm. The particle size was uniform and the particles were shiny. After 30 days of storage at room temperature, the amount of viable probiotics in the microcapsules decreased to 9.3×10⁹ cfu/g; after 60 days, the amount of viable probiotics in the microcapsules decreased to 7.5×10⁹ cfu/g; after 120 days, the amount of viable probiotics in the microcapsules decreased to 4.8×10⁹ cfu/g; and the number of the probiotic residue on the outer surface of the microcapsules was 2.3×10⁶ cfu/g. After the double-layered microcapsules were incubated in a simulated gastric juice for 2 hours, the amount of contained viable probiotics decreased to 8.8×10⁹ cfu/g, and the weight of the dry microcapsule particles increased by 15% after absorbing water for 48 hours.

### Comparative example A2

1. Raw materials (based on 1000g of dry microcapsule particles)
   WPI: 550g, concentration 11%;
   250g of Bacillus coagulans powders;
   Curing agent solution: 0.5mol/L, pH-4.6
   MCT: 200g
   Starch: 10% concentration
2. Preparation method
   a. The whey protein was uniformly mixed with water and stirred at 4°C at 180 rpm/min for 16 hours;
   b. The whey protein solution was heated at 78°C at 95 rpm/min for 45 minutes;
   c. The solution was cooled immediately at 0°C, and stored at 4°C for 8 hours;
   d. 137g of sodium acetate, 100ml of glacial acetic acid and 6718g of water were uniformly mixed to formulate a curing agent solution, and the curing agent solution was adjusted to a pH value of 4.6. 2.9g of Tween-20 was added, and the mixture was uniformly mixed for use;
   e. Granulation: the probiotic powder, binder solution and MCT were uniformly mixed to make grease droplets uniformly wrap particles;
   f. The whey protein solution was added to make droplets uniformly wrap the particles obtained in step e;
   g. Curing: droplets of the curing solution were used to uniformly wrap the particles obtained in step f;
   h. The microcapsules prepared in step g were dried.

The microcapsules obtained in this comparative example were double-layered microcapsules. The amount of viable probiotics in the microcapsules reached 4.8×10⁹ cfu/g. The particle size was 150-250 µm. The particle size was uneven with tailing, and the gloss was not good. After 30 days of storage at room temperature, the amount of viable probiotics in the microcapsules decreased to 8.2×10⁸ cfu/g; after 60 days, the amount of viable probiotics in the microcapsules decreased to 6.1×10⁷ cfu/g; after 120 days, the amount of viable probiotics in the microcapsules decreased to 3.8×10⁶ cfu/g; and the number of the probiotic residue on the outer surface of the microcapsules was 3.6×10⁶ cfu/g. After the double-layered microcapsules were incubated in a simulated gastric juice for 2 hours, the amount of contained viable probiotics decreased to 4.1×10⁷ cfu/g, and the weight of the dry microcapsule particles increased by 50% after absorbing water for 48 hours.

### Comparative example A3

1. Raw materials (based on 1000g of dry microcapsule particles)
   WPI: 550g, concentration 11%;
   250g of Bacillus coagulans powders;
   Curing agent solution: 0.5mol/L, pH-4.6
   MCT: 200g
   Starch: temperature 60°C, concentration 25%
2. Preparation method
   a. The whey protein was uniformly mixed with water and stirred at 4°C at 180 rpm/min for 16 hours;
   b. The whey protein solution was heated at 78°C at 95 rpm/min for 45 minutes;
   c. The solution was cooled immediately at 0°C, and stored at 4°C for 40 hours;
   d. 137g of sodium acetate, 100ml of glacial acetic acid and 6718g of water were uniformly mixed to formulate a curing agent solution, and the curing agent solution was adjusted to a pH value of 4.6. 2.9g of Tween-20 was added, and the mixture was uniformly mixed for use;
   e. Granulation: the probiotic powder, binder solution and MCT were uniformly mixed to make grease droplets uniformly wrap particles;
   f. The whey protein solution was added to make droplets uniformly wrap the particles obtained in step e;
   g. Curing: droplets of the curing solution were used to uniformly wrap the particles obtained in step f;
   h. The microcapsules prepared in step g were dried.

The microcapsules obtained in this comparative example were single-layered microcapsules. The amount of viable probiotics in the microcapsules reached 5.4×10⁹ cfu/g. The particle size was 150-250 µm. The particle size was uneven with tailing. After 30 days of storage at room temperature, the amount of viable probiotics in the microcapsules decreased to 5.2×10⁸ cfu/g; after 60 days, the amount of viable probiotics in the microcapsules decreased to 3.0×10⁷ cfu/g; after 120 days, the amount of viable probiotics in the microcapsules decreased to 2.2×10⁶ cfu/g; and the number of the probiotic residue on the outer surface of the microcapsules was 1.6×10⁶ cfu/g. After the double-layered microcapsules were incubated in a simulated gastric juice for 2 hours, the amount of contained viable probiotics decreased to 3.1×10⁷ cfu/g, and the weight of the dry microcapsule particles increased by 50% after absorbing water for 48 hours.

### Example A5

1. Raw materials (based on 1000g of dry microcapsule particles)
   MCT (the first layer of wall material) 200g (concentration 100%);
   WPI (the second layer of wall material) 350g (concentration 11%);
   MCT (the third layer of wall material) 200g (concentration 100%);
   250g of Bacillus coagulans powders;
   Curing agent solution: 0.5mol/L, pH-4.6
   Starch: 10% concentration
2. Preparation method
   a. The whey protein was uniformly mixed with water and stirred at 4°C at 180 rpm/min for 16 hours;
   b. The whey protein solution was heated at 78°C at 95 rpm/min for 45 minutes;
   c. The solution was cooled immediately at 0°C, and stored at 4°C for 16 hours;
   d. 137g of sodium acetate, 100ml of glacial acetic acid and 6718g of water were uniformly mixed to formulate a curing agent solution, and the curing agent solution was adjusted to a pH value of 4.6. 2.9g of Tween-20 was added, and the mixture was uniformly mixed for use;
   e. Granulation: the probiotic powder, binder solution and MCT were uniformly mixed to make grease droplets uniformly wrap particles;
   f. The whey protein solution was added to make droplets uniformly wrap the particles obtained in step e;
   g. Curing: droplets of the curing solution were used to uniformly wrap the particles obtained in step f;
   h. The microcapsules prepared in step g were dried;
   k. The particles obtained in step h were subjected to a third coating with MCT according to the aforementioned steps, wherein the MCT was 200 g.

The microcapsules obtained in this example were three-layered microcapsules. The amount of viable probiotics in the microcapsules reached 1.3×10¹⁰ cfu/g. The particle size was 150-250 µm. The particle size was uniform and the particles were shiny. After 30 days of storage at room temperature, the amount of viable probiotics in the microcapsules decreased to 9.9×10⁹ cfu/g; after 60 days, the amount of viable probiotics in the microcapsules decreased to 8.9×10⁹ cfu/g; after 120 days, the amount of viable probiotics in the microcapsules decreased to 7.2×10⁹ cfu/g, and the number of the probiotic residue on the outer surface of the microcapsules was 0 cfu/g. After the three-layered microcapsules were incubated in a simulated gastric juice for 2 hours, the amount of contained viable probiotics decreased to 9.0×10⁹ cfu/g, and the weight of the dry microcapsule particles increased by 5% after absorbing water for 48 hours.

### Example A6

1. Raw materials (based on 1000g of dry microcapsule particles)
   MCT (the first layer of wall material) 200g (concentration 100%);
   WPI (the second layer of wall material) 300g (concentration 11%);
   MCT (the third layer of wall material) 150g (concentration 100%);
   WPI (the fourth layer of wall material) 100g (concentration 11%);
   250g of Bacillus coagulans powders;
   Curing agent solution: 0.5mol/L, pH-4.6
   Starch: 10% concentration
2. Preparation method
   a. The whey protein was uniformly mixed with water and stirred at 4°C at 180 rpm/min for 16 hours;
   b. The whey protein solution was heated at 78°C at 95 rpm/min for 45 minutes;
   c. The solution was cooled immediately at 0°C, and stored at 4°C for 16 hours;
   d. 137g of sodium acetate, 100ml of glacial acetic acid and 6718g of water were uniformly mixed to formulate a curing agent solution, and the curing agent solution was adjusted to a pH value of 4.6. 2.9g of Tween-20 was added, and the mixture was uniformly mixed for use;
   e. Granulation: the probiotic powder, binder solution and MCT were uniformly mixed to make grease droplets uniformly wrap particles;
   f. The whey protein solution was added to make droplets uniformly wrap the particles obtained in step e;
   g. Curing: droplets of the curing solution were used to uniformly wrap the particles obtained in step f;
   h. The microcapsules prepared in step g were dried;
   k. The particles obtained in step j were subjected to a third coating with MCT according to the aforementioned steps;
   l. The particles obtained in step k were subjected to a fourth coating with WPI according to the aforementioned steps.

The microcapsules obtained in this example were four-layered microcapsules. The amount of viable probiotics in the microcapsules reached 1.3×10¹⁰ cfu/g. The particle size was 150-400 µm. The particle size was uniform and the particles were shiny. After 30 days of storage at room temperature, the amount of viable probiotics in the microcapsules remained at 1.3×10¹⁰ cfu/g. After 60 days, the amount of viable probiotics in the microcapsules decreased to 9.8×10⁹ cfu/g. After 120 days, the amount of viable probiotics in the microcapsules decreased to 9.0×10⁹ cfu/g, and the number of the probiotic residue on the outer surface of the microcapsules was 0 cfu/g. After the four-layered microcapsules were incubated in a simulated gastric juice for 2 hours, the amount of contained viable probiotics decreased to 1.0×10¹⁰ cfu/g, and the weight of the dry microcapsule particles increased by 1% after absorbing water for 48 hours.

### Example A7

1. Raw materials (based on 1000g of dry microcapsule particles)
   WPI: 750g, concentration 11%;
   250g of Bacillus coagulans powders;
   Curing agent solution: 0.5mol/L, pH-4.6
   MCT: 0g
   Starch: temperature 50°C, concentration 15%
2. Preparation method
   a. The whey protein was uniformly mixed with water and stirred at 4°C at 180 rpm/min for 16 hours;
   b. The whey protein solution was heated at 78°C at 95 rpm/min for 45 minutes;
   c. The solution was cooled immediately at 0°C, and stored at 4°C for 30 hours;
   d. 137g of sodium acetate, 100ml of glacial acetic acid and 6718g of water were uniformly mixed to formulate a curing agent solution, and the curing agent solution was adjusted to a pH value of 4.6. 2.9g of Tween-20 was added, and the mixture was uniformly mixed for use;
   e. Granulation: the probiotic powder, binder solution and WPI were uniformly mixed to make WPI uniformly wrap the particles;
   f. Curing: droplets of the curing solution were used to uniformly wrap the particles obtained in step e;
   g. The microcapsules prepared in step f were dried.

The microcapsules obtained in this example were double-layered microcapsules. The amount of viable probiotics in the microcapsules reached 1.0×10¹⁰ cfu/g. The particle size was 150-250 µm. The particle size was uniform and the particles were shiny. After 30 days of storage at room temperature, the amount of viable probiotics in the microcapsules decreased to 8.9×10⁹ cfu/g; after 60 days, the amount of viable probiotics in the microcapsules decreased to 7.0×10⁹ cfu/g; after 120 days, the amount of viable probiotics in the microcapsules decreased to 4.5×10⁹ cfu/g, and the number of the probiotic residue on the outer surface of the microcapsules was 3.8×10⁶ cfu/g. After the double-layered microcapsules were incubated in a simulated gastric juice for 2 hours, the amount of contained viable probiotics decreased to 8.2×10⁹ cfu/g, and the weight of the dry microcapsule particles increased by 10% after absorbing water for 48 hours.

### Comparative example A4

1. Raw materials (based on 1000g of dry microcapsule particles)
   WPI: 750g, concentration 11%;
   250g of Bacillus coagulans powders;
   Curing agent solution: 0.5mol/L, pH-4.6
   MCT: 0g
   Starch: temperature 60°C, concentration 5%
2. Preparation method
   a. The whey protein was uniformly mixed with water and stirred at 4°C at 180 rpm/min for 16 hours;
   b. The whey protein solution was heated at 78°C at 95 rpm/min for 45 minutes;
   c. The solution was cooled immediately at 0°C, and stored at 4°C for 65 hours;
   d. 137g of sodium acetate, 100ml of glacial acetic acid and 6718g of water were uniformly mixed to formulate a curing agent solution, and the curing agent solution was adjusted to a pH value of 4.6. 2.9g of Tween-20 was added, and the mixture was uniformly mixed for use;
   e. Granulation: the probiotic powder, binder solution and WPI were uniformly mixed to make WPI uniformly wrap the particles;
   f. Curing: droplets of the curing solution were used to uniformly wrap the particles obtained in step e;
   g. The microcapsules prepared in step f were dried.

The double-layered microcapsule particles of this comparative example were not shaped, and most of them were flocculent in the curing solution.

### Example A8:

1. Raw materials (based on 1000g of dry microcapsule particles)
   WPI: 550g, concentration 11%;
   250g of Bacillus coagulans powders;
   Curing agent solution: 0.5mol/L, pH-4.6
   MCT: 200g
   Starch: temperature 70°C, concentration 20%
2. Preparation method
   a. The whey protein was uniformly mixed with water and stirred at 4°C at 180 rpm/min for 16 hours;
   b. The whey protein solution was heated at 78°C at 95 rpm/min for 45 minutes;
   c. The solution was cooled immediately at 0°C, and stored at 4°C for 50 hours;
   d. 137g of sodium acetate, 100ml of glacial acetic acid and 6718g of water were uniformly mixed to formulate a curing agent solution, and the curing agent solution was adjusted to a pH value of 4.6. 2.9g of Tween-20 was added, and the mixture was uniformly mixed for use;
   e. Granulation: the probiotic powder, binder solution and MCT were uniformly mixed to make grease droplets uniformly wrap particles;
   f. The whey protein solution was added to make droplets uniformly wrap the particles obtained in step e;
   g. Curing: droplets of the curing solution were used to uniformly wrap the particles obtained in step f;
   h. The microcapsules prepared in step g were dried.

The microcapsules obtained in this example were double-layered microcapsules. The amount of viable probiotics in the microcapsules reached 1.2×10¹⁰ cfu/g. The particle size was 150-250 µm. The particle size was uneven with tailing. After 30 days of storage at room temperature, the amount of viable probiotics in the microcapsules decreased to 9.0×10⁹ cfu/g; after 60 days, the amount of viable probiotics in the microcapsules decreased to 7.4×10⁹ cfu/g; after 120 days, the amount of viable probiotics in the microcapsules decreased to 4.6×10⁹ cfu/g; and the number of the probiotic residue on the outer surface of the microcapsules was 4.2×10⁶ cfu/g. After the double-layered microcapsules were incubated in a simulated gastric juice for 2 hours, the amount of contained viable probiotics decreased to 8.0×10⁹ cfu/g, and the weight of the dry microcapsule particles increased by 15% after absorbing water for 48 hours.

The preparation process and microcapsule particle test results of the above examples were summarized in the following table:

| **Microcapsule Examples A1-A3 and Comparative Example A1** | | | | | |
|---|---|---|---|---|---|
| **Process parameters** | **Parameter range** | **Example A1** | **Example A2** | **Example A3** | **Comparative Example A1** |
| WPI (the first layer) concentration/% | 9-13 | 11 | 11 | 11 | 11 |
| WPI (the first layer) heat treatment temperature/°C | 75-96 | 78 | 78 | 78 | 78 |
| WPI (the first layer) heat treatment time/min | 30-180 | 45 | 45 | 45 | 45 |
| Cooling time/h | 12-60 | 12 | 12 | 12 | 4 |
| Pore diameter of extrusion nozzle/µm | 150-750 | 200 | 200 | 200 | 200 |
| Granulation voltage for extrusion method/mV | 1500-1800 | 1600 | 1600 | 1600 | 1600 |
| Granulation frequency of extrusion method/Hz | 1300-1500 | 1300 | 1300 | 1300 | 1300 |
| Fluidization temperature/°C | 30-80 | 65 | 65 | 40 | 65 |
| Part by weight of probiotics | 50-500 | 250 | 250 | 250 | 250 |
| Part by weight of WPI (the first layer) | 150-950 | 750 | 450 | 300 | 750 |
| Part by weight of WPI (the second layer) | 0-350 | 0 | 300 | 0 | 0 |
| Part by weight of MCT (the second layer) | 0-350 | 0 | 0 | 200 | 0 |
| Part by weight of WPI (the third layer) | 0-250 | 0 | 0 | 250 | 0 |

| **Microcapsule Examples A4-A8 and Comparative Example A2-A4** | | | | | | | | | |
|---|---|---|---|---|---|---|---|---|---|
| **Process parameters** | **Parameter range** | **Ex. A4** | **Ex. AS** | **Ex. A6** | **Ex. A7** | **Ex. A8** | **Comparative Ex. A2** | **Comparative Ex. A3** | **Comparative Ex. A4** |
| Concentratio n of WPI/% | 9-13 | 11 | 11 | 11 | 11 | 11 | 11 | 11 | 11 |
| Heat treatment temperature of WPI/°C | 75-96 | 78 | 78 | 78 | 78 | 78 | 78 | 78 | 78 |
| Heat treatment time of WPI/min | 30-180 | 45 | 45 | 45 | 45 | 45 | 45 | 45 | 45 |
| Curing solution concentratio n 0.5mol/l, PH | 4.5-5.3 | 4.6 | 4.6 | 4.6 | 4.6 | 4.6 | 4.6 | 4.6 | 4.6 |
| Cooling time/h | 12-60 | 12 | 16 | 16 | 30 | 50 | 16 | 16 | 65 |
| Binder temperature/ °C | 30-80 | 30 | 40 | 40 | 50 | 70 | 70 | 90 | 60 |
| Binder concentratio n/mass percentage | 5-20% | 5% | 10% | 10% | 15% | 20% | 10% | 10% | 5% |
| Fluidization temperature/ °C | 30-80 | 30 | 40 | 40 | 50 | 70 | 20 | 90 | 40 |
| Temperature of protein solution | 25-75 | 35 | 30 | 35 | 25 | 50 | 20 | 35 | 25 |
| Part by weight of probiotics | 50-500 | 250 | 250 | 250 | 250 | 250 | 250 | 250 | 250 |
| Part by weight of MCT (the first layer) | 0-350 | 200 | 200 | 200 | 0 | 200 | 200 | 200 | 0 |
| Part by weight of WPI (the second layer) | 150-950 | 550 | 350 | 300 | 750 | 550 | 350 | 300 | 750 |
| Part by weight of MCT (the third layer) | 0-350 | 0 | 200 | 150 | 0 | 0 | 200 | 150 | 0 |
| Part by weight of WPI (the fourth layer) | 0-250 | 0 | 0 | 100 | 0 | 0 | 0 | 100 | 0 |

2. The test results of the microcapsule particles of the present examples A1-A8 and the comparative example A1-A4 were as follows:

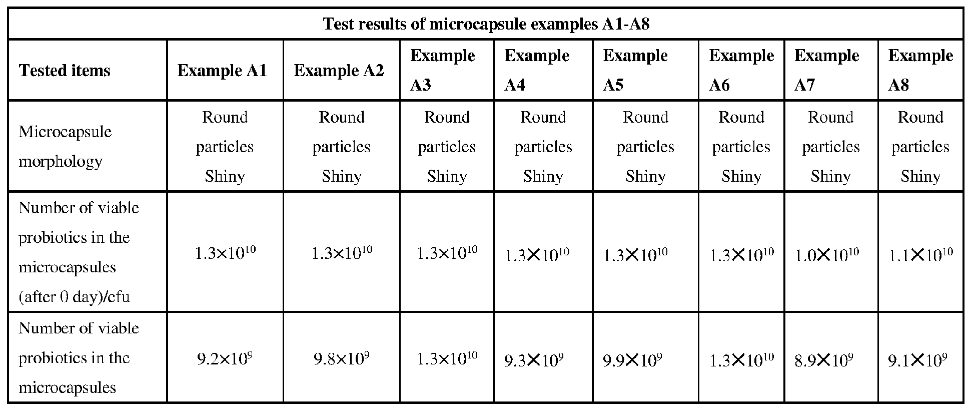

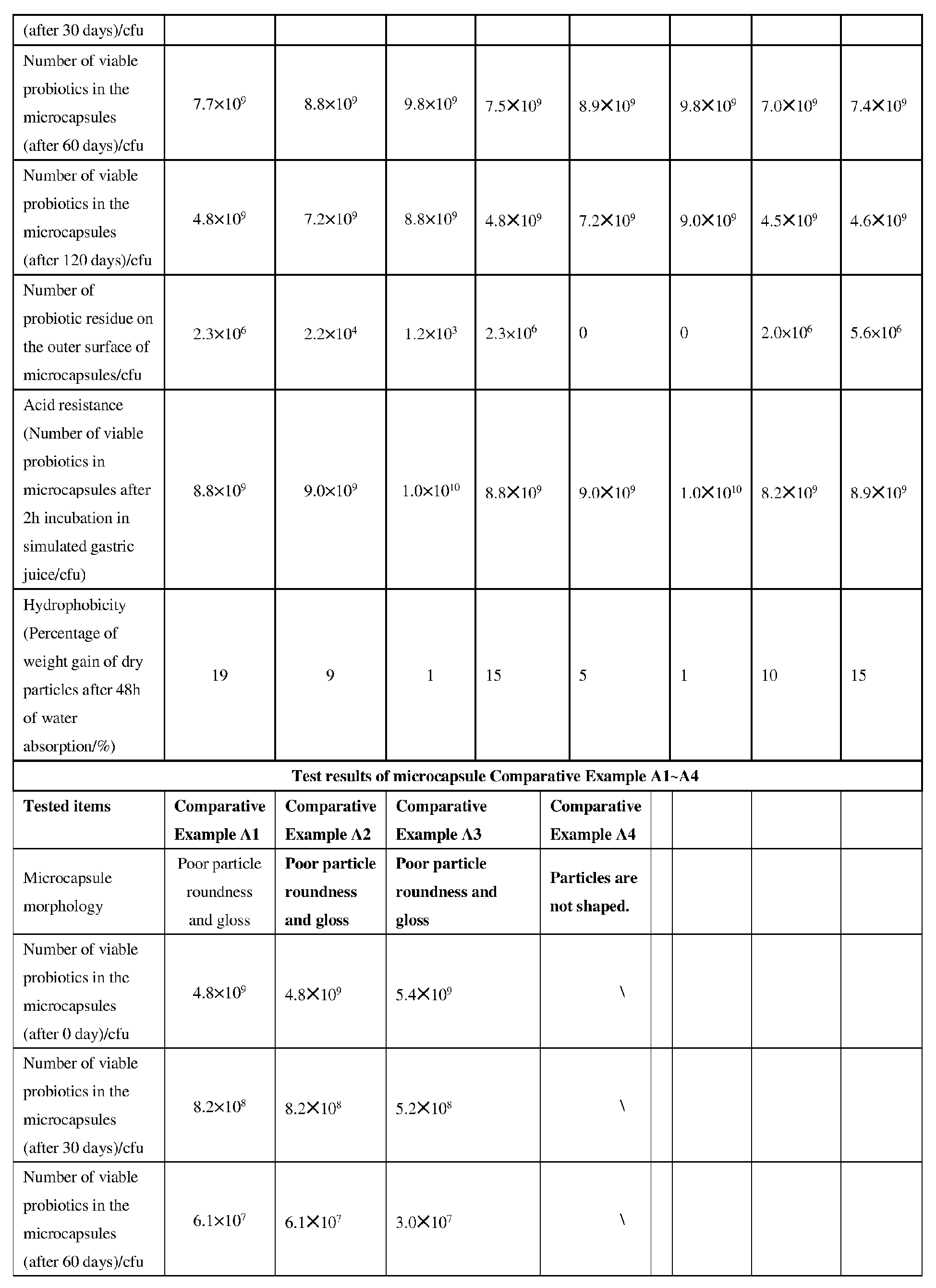

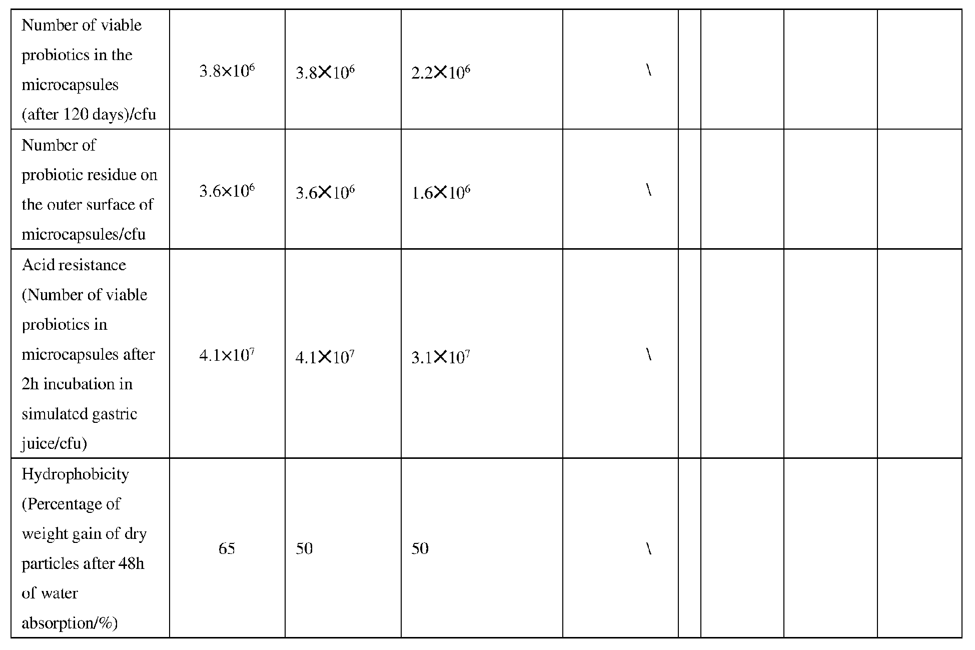

### B. Preparation of yogurt

### Examples B1-B5, comparative examples B1-B2

The specific operations of Examples B1-B5 and Comparative Examples B1-B2 of the present application were as follows:
1. Preparing fermented milk according to the national standard preparation process of fermented milk
   a. The raw materials other than zymogenic strains, microcapsules, and honey were mixed, preferably at about 40-80°C, to prepare a mixture of fermented milk, and then cooling, preferably to below about 20°C;
   b. The mixture was stirred, homogenizing (preferably at a homogenizing pressure of about 150-200bar), then subjected to high-temperature and long-term sterilization (preferably at a sterilization temperature of 95°C, and a sterilization time of 300 seconds), and then cooled to 41-43°C after the sterilization;
   c. Inoculation, and fermentation at 41-43°C;
   d. Demulsifying, turning over a tank, and then cooling to below 25°C;
   e. Pasteurizing at a sterilization temperature of 74°C and at a sterilization time of 30 seconds;
   f. Cooling to 15-30°C and entering an aseptic tank;
   g. Aseptic filling;
2. Adding probiotic microcapsule particles
   wherein after step e and before step f, the product obtained in step e was transferred to a tank to be filled; probiotic microcapsule particles were directly added, and stirred for 15 minutes.

Using the basic preparation method, probiotic microcapsules were directly added before pasteurization:

| Raw material ratio | Indicator range | Example B1 | Example B2 | Example B3 | Example B4 | Example B5 | Comparative Example B 1 | Comparative Example B2 |
|---|---|---|---|---|---|---|---|---|
| The amount of probiotic microcapsules added | 0.02-1% | 0.02 | 0.04 | 0.06 | 0.5 | 1 | 0.01 | 1.1 |

### Examples B6-B10, Comparative Examples B3-B4

The specific operations of Examples B6-B10 and Comparative Examples B3-B4 of the present application were as follows:
1. Preparing fermented milk according to the national standard preparation process of fermented milk
   a. The raw materials other than zymogenic strains, microcapsules, and honey were mixed, preferably at about 40 to 80°C, to prepare a mixture of fermented milk, and then cooled, preferably to below about 20°C;
   b. The mixture was stirred, homogenized (preferably at a homogenizing pressure of about 150-200bar), then subjected to high-temperature and long-term sterilization (preferably at a sterilization temperature of 95°C, and a sterilization time of 300 seconds), and then cooled to 41-43°C after the sterilization;
   c. Inoculation and fermentation at 41-43°C;
   d. Demulsifying, turning over a tank, and then cooling to below 25°C;
   e. Pasteurizing at a sterilization temperature of 75°C and a sterilization time of 25 seconds;
   f. Cooling to 15-30°C and entering an aseptic tank;
   g. Aseptic filling;
2. Adding probiotic microcapsule particles

Before step g and after step f, the sterilized probiotic microcapsules were added, wherein honey was used as a carrier to sterilize probiotic microcapsules, and sterile online addition was used. Examples and Comparative Examples were as follows:

| Raw material ratio | Indicator range | Example B6 | Example B7 | Example B8 | Example B9 | Example B10 | Comparative Example B3 | Comparative Example B4 |
|---|---|---|---|---|---|---|---|---|
| The amount of probiotic microcapsules added | 0.02-1% | 0.02 | 0.04 | 0.06 | 0.5 | 1 | 0.01 | 1.1 |
| Microcapsules: Honey | 1:1000-1:10 | 1:1000 | 1:500 | 1:100 | 1:50 | 1:10 | 1:1100 | 1:9 |
| UV treatment intensity of the microcapsule mixture | 1000-20000J/L | 1000 | 5000 | 10000 | 15000 | 20000 | 900 | 21000 |

**The product results and evaluations of Examples B1-B5 and Comparative Examples B1-B2 in this application were as follows:**

| Evaluated items | Example B1 | Example B2 | Example B3 | Example B4 | Example B5 | Comparative Example B1 | Comparative Example B2 |
|---|---|---|---|---|---|---|---|
| Taste | Smooth and delicate taste, pH 4.25 | Smooth and delicate taste, pH 4.25 | Smooth and delicate taste, pH 4.25 | Smooth and delicate taste, moderate acidity, pH 4.25 | Smooth and delicate taste, moderate acidity, pH 4.25 | Smooth and delicate taste, light taste, pH 4.25 | Obvious graininess, pH 4.25 |
| Whey precipitation | No whey precipitati on | No whey precipitation | No whey precipitati on | No whey precipitati on | Slight whey precipitati on | No whey precipitati on | No whey precipitatio n |
| Texture | Smooth | Smooth | Smooth | Smooth | Smooth | Smooth | Rough texture |
| Stability | Slightly layered during shelf life | Not layered during shelf life | Not layered during shelf life | Not layered during shelf life | Not layered during shelf life | Not layered during shelf life | Layered |
| The number of probiotics during shelf life, cfu/ml | ≥1.0^{∗}10⁶ | ≥1.0^{∗}10⁶ | ≥1.0^{∗}10⁶ | ≥1.0^{∗}10⁶ | ≥1.0^{∗}10⁶ | <1.0^{∗}10⁶ | ≥1.0^{∗}10⁶ |
| Acid resistance (The number of viable probiotics in microcapsules after being digested in simulated gastric juice for 2 hours/cfu) | ≥1.0^{∗}10⁶ | ≥1.0^{∗}10⁶ | ≥1.0^{∗}10⁶ | ≥1.0^{∗}10⁶ | ≥1.0^{∗}10⁶ | <1.0^{∗}10⁶ | ≥1.0^{∗}10⁶ |
| Miscellaneous bacteria residue | None | None | None | None | None | None | None |
| Overview | Acceptable | Relatively good | Good | Relatively good | Acceptable | Unacceptable | Unacceptable |

**The product results and evaluations of Examples B6-B10 and Comparative Examples B3-B4 in this application were as follows:**

| Evaluate d items | Example B6 | Example B7 | Example B8 | Example B9 | Example B10 | Comparative Example B3 | Comparativ e Example B4 |
|---|---|---|---|---|---|---|---|
| Taste | Smooth and delicate taste, pH 4.2 | Smooth and delicate taste, pH 4.2 | Smooth and delicate taste, pH 4.2 | Smooth and delicate taste, moderate acidity, pH 4.2 | Smooth and delicate taste, moderate acidity, pH 4.2 | Smooth and delicate taste, light taste, pH 4.2 | Obvious graininess, pH 4.2 |
| Whey precipitation | No whey precipitation | No whey precipitation | No whey precipitation | No whey precipitation | Slight whey precipitation | No whey precipitation | No whey precipitatio n |
| Texture | Smooth | Smooth | Smooth | Smooth | Smooth | Smooth | Rough texture |
| Stability | Slightly layered during shelf life | Not layered during shelf life | Not layered during shelf life | Not layered during shelf life | Not layered during shelf life | Not layered during shelf life | Layered |
| The number of probiotics during shelf lifecfu/ml | ≥1.0^{∗}10⁶ | ≥1.0^{∗}10⁶ | ≥1.0^{∗}10⁶ | ≥1.0^{∗}10⁶ | ≥1.0^{∗}10⁶ | <1.0^{∗}10⁶ | ≥1.0^{∗}10⁶ |
| Acid resistanc e (The number of viable probioti cs in microca psules after being digested in simulate d gastric juice for 2 hours/cf u) | ≥1.0^{∗}10⁶ | ≥1.0^{∗}10⁶ | ≥1.0^{∗}10⁶ | ≥1.0^{∗}10⁶ | ≥1.0^{∗}10⁶ | <1.0^{∗}10⁶ | ≥1.0^{∗}10⁶ |
| Miscella neous bacteria residue | None | None | None | None | None | Yes | None |

### L. Preparation of microcapsules (probiotic microcapsules) (II)

Used in the following examples:
The specification of Bacillus coagulans was 90 billion CFU/g.

Prolamin was zein, which was easily soluble in 75-92% ethanol.

The extrusion spheronization granulator was consisted of a wet mixing granulator and a low-shear vertical extrusion spheronization machine. The wet mixing granulator was used for a mixing of dry powders and a mixing of dry powders and water to make raw materials into wet materials. Low-shear vertical extrusion spheronization machine was used to extrude, cut, and shape wet materials to obtain a regular round pellet core.

### Examples L1 to 3 and comparative examples

### Example L1

Raw materials (based on 150kg of dry microcapsule particles):
Pellet core: 200g of Bacillus coagulans, 800g of microcrystalline cellulose, 1000g of water.
Water-blocking coating layer solution 1: Prolamin 288g, oleic acid 112g, 75% ethanol 1200g.
Water-blocking coating layer solution 2: prolamin 54.4g, glycerin 12.8g, 75% ethanol 252.8g.
Hydrophilic coating layer solution: pectin 110g, water 2090g.

Preparation:
Bacillus coagulans powders were uniformly mixed with microcrystalline cellulose. The mixture was added into a wet granulation pot, and premixed for 10 minutes.

A cutting knife was turned on, and a peristaltic pump was turned on at 20-30r/min. Water consumption was 1000kg, and time was 30mins. A soft material with a water content of 49.7% was obtained.

A granulator was started with a feeding speed of 30rpm and an extrusion speed of 50rpm for 60min. A soft material was added, and a strip-shaped soft material was extruded through an orifice plate. The strip-shaped soft material was collected on a receiving tray, and kept below 45°C.

The extrusion motor was stopped. The spheronization speed was adjusted to 400rpm, and the spheronization motor was started for spheronization for 100s. The extruded strip-shaped soft material was poured into a spheronization cylinder at one time and rounded.

After the spheronization was completed, the pellets were dried in a fluidized bed at a drying temperature of 45-50°C to obtain a primary pellet core.

A fluidized bed spray granulation method was used, wherein the fluidized bed inlet air temperature was set to 55°C, the air flow was 32, and the atomization pressure was 2.5.

1600 g of the water-blocking coating solution 1 was sprayed into the fluidized bed through spray gun atomization to make the droplets uniformly wrap the pellet core. After fluidization of every 400 ml of coating solution, particles were passed through a 50-80 mesh screen, and the particles larger than 80 mesh and smaller than 50 mesh were discarded.

320 g of the water-blocking coating solution 2 was sprayed into the fluidized bed through spray gun atomization, and the particles after 50-80 mesh sieving were retained. A double-layered water-blocked particles were thus formed.

220 g of the hydrophilic coating solution was sprayed into the fluidized bed through spray gun atomization, and the particles after 50-80 mesh sieving were retained to obtain the final particles.

The microcapsules obtained in this example were three-layered microcapsules. The amount of viable probiotics in the microcapsules reached 1.3×10¹⁰ cfu/g. The amount of viable probiotics outside the microcapsules reached 7.8×10³ cfu/g. The particle size was 180-270 µm. The particle size was uniform, and the particles were ecru and shiny. After 30 days of storage at room temperature, the amount of viable probiotics in the microcapsules decreased to 1.2×10¹⁰ cfu/g; after 60 days, the amount of viable probiotics in the microcapsules decreased to 1.15×10¹⁰ cfu/g; after 120 days, the amount of viable probiotics in the microcapsules decreased to 1.05×10¹⁰ cfu/g, and the number of the probiotic residue on the outer surface of the microcapsules was 4.5×10³ cfu/g. The amount of viable probiotics contained in the microcapsules decreased to 1.1×10¹⁰ cfu/g after 2 hours of incubation in the simulated gastric juice.

### Example L2

Raw materials (based on 150kg of dry microcapsule particles):
Pellet core: 200g of Bacillus coagulans, 800g of microcrystalline cellulose, 1000g of water.
Water-blocking coating layer solution 1: Prolamin 270g, oleic acid 105g, 75% ethanol 1125g.
Water-blocking coating layer solution 2: prolamin 54.4g, glycerin 12.8g, 75% ethanol 252.8g.
Hydrophilic coating layer solution: pectin 110g, water 2090g.

Preparation:
Bacillus coagulans powders were uniformly mixed with microcrystalline cellulose. The mixture was added into a wet granulation pot, and premixed for 10 minutes.

A cutting knife was turned on, and a peristaltic pump was turned on at 20-30r/min. Water consumption was 1000kg, and time was 30mins. A soft material with a water content of 49.7% was obtained.

A granulator was started with a feeding speed of 30rpm and an extrusion speed of 50rpm for 60min. A soft material was added, and a strip-shaped soft material was extruded through an orifice plate. The strip-shaped soft material was collected on a receiving tray, and kept below 45°C.

The extrusion motor was stopped. The spheronization speed was adjusted to 400rpm, and the spheronization motor was started for spheronization for 100s. The extruded strip-shaped soft material was poured into a spheronization cylinder at one time and rounded.

After the spheronization was completed, the pellets were dried in a fluidized bed at a drying temperature of 45-50°C to obtain a primary pellet core.

A fluidized bed spray granulation method was used, wherein the fluidized bed inlet air temperature was set to 55°C, the air flow was 32, and the atomization pressure was 2.5.

1600 g of the water-blocking coating solution 1 was sprayed into the fluidized bed through spray gun atomization to make the droplets uniformly wrap the pellet core. After fluidization of every 400 ml of coating solution, particles were passed through a 50-80 mesh screen, and the particles larger than 80 mesh and smaller than 50 mesh were discarded.

320 g of the water-blocking coating solution 2 was sprayed into the fluidized bed through spray gun atomization, and the particles after 50-80 mesh sieving were retained. A double-layered water-blocked particles were thus formed.

220 g of the hydrophilic coating solution was sprayed into the fluidized bed through spray gun atomization, and the particles after 50-80 mesh sieving were retained to obtain the final particles.

The microcapsules obtained in this example were three-layered microcapsules. The amount of viable probiotics in the microcapsules reached 1.35×10¹⁰ cfu/g. The amount of viable probiotics outside the microcapsules reached 8.2×10³ cfu/g. The particle size was 180-270 µm. The particle size was uniform, and the particles were ecru and shiny. After 30 days of storage at room temperature, the amount of viable probiotics in the microcapsules decreased to 1.28×10¹⁰ cfu/g; after 60 days, the amount of viable probiotics in the microcapsules decreased to 1.14×10¹⁰ cfu/g; after 120 days, the amount of viable probiotics in the microcapsules decreased to 1.0×10¹⁰ cfu/g, and the number of the probiotic residue on the outer surface of the microcapsules was 4.0×10³ cfu/g. The amount of viable probiotics contained in the microcapsules decreased to 1.05×10¹⁰ cfu/g after 2 hours of incubation in the simulated gastric juice.

### Example L3

Raw materials (based on 1500g of dry microcapsule particles):
Pellet core: 200g of Bacillus coagulans, 800g of microcrystalline cellulose, 1000g of water.
Water-blocking coating layer solution 1: prolamin 292.5g, oleic acid 157.5g, 75% ethanol 1200g.
Water-blocking coating layer solution 2: prolamin 54.4g, glycerin 12.8g, 75% ethanol 252.8g.
Hydrophilic coating layer solution: pectin 110g, water 2090g.

Preparation:
Bacillus coagulans powders were uniformly mixed with microcrystalline cellulose. The mixture was added into a wet granulation pot, and premixed for 10 minutes.

A cutting knife was turned on, and a peristaltic pump was turned on at 20-30r/min. Water consumption was 1000g, and time was 30mins. A soft material with a water content of 49.7% was obtained.

A granulator was started with a feeding speed of 30rpm and an extrusion speed of 50rpm for 60min. A soft material was added, and a strip-shaped soft material was extruded through an orifice plate. The strip-shaped soft material was collected on a receiving tray, and kept below 45°C.

The extrusion motor was stopped. The spheronization speed was adjusted to 420rpm, and the spheronization motor was started for spheronization for 100s. The extruded strip-shaped soft material was poured into a spheronization cylinder at one time and rounded.

After the spheronization was completed, the pellets were dried in a fluidized bed at a drying temperature of 45-50°C to obtain a primary pellet core.

A fluidized bed spray granulation method was used, wherein the fluidized bed inlet air temperature was set to 55°C, the air flow was 32, and the atomization pressure was 2.5.

1800 g of the water-blocking coating solution 1 was sprayed into the fluidized bed through spray gun atomization to make the droplets uniformly wrap the pellet core. After fluidization of every 400 ml of coating solution, particles were passed through a 50-80 mesh screen, and the particles larger than 80 mesh and smaller than 50 mesh were discarded.

320 g of the water-blocking coating solution 2 was sprayed into the fluidized bed through spray gun atomization, and the particles after 50-80 mesh sieving were retained. A double-layered water-blocked particles were thus formed.

220 g of the hydrophilic coating solution was sprayed into the fluidized bed through spray gun atomization, and the particles after 50-80 mesh sieving were retained to obtain the final particles.

The microcapsules obtained in this example were three-layered microcapsules. The amount of viable probiotics in the microcapsules reached 1.2×10¹⁰ cfu/g. The amount of viable probiotics outside the microcapsules reached 7.5×10³ cfu/g. The particle size was 180-270 µm. The particle size was uniform, and the particles were ecru and shiny. After 30 days of storage at room temperature, the amount of viable probiotics in the microcapsules decreased to 1.1×10¹⁰ cfu/g; after 60 days, the amount of viable probiotics in the microcapsules decreased to 9.8×10⁹ cfu/g; after 120 days, the amount of viable probiotics in the microcapsules decreased to 1.0×10¹⁰ cfu/g, and the number of the probiotic residue on the outer surface of the microcapsules was 7×10³ cfu/g. The amount of viable probiotics contained in the microcapsules decreased to 1.15×10¹⁰ cfu/g after 2 hours of incubation in the simulated gastric juice.

### Example L4

Raw materials (based on 150kg of dry microcapsule particles):
Pellet core: 180g of Bacillus coagulans, 820g of microcrystalline cellulose, 1000g of water.
Water-blocking coating layer solution 1: prolamin 288g, oleic acid 112g, 75% ethanol 1200g.
Water-blocking coating layer solution 2: prolamin 54.4g, glycerin 12.8g, 75% ethanol 252.8g.
Hydrophilic coating layer solution: pectin 110g, water 2090g.

Preparation:
Bacillus coagulans powders were uniformly mixed with microcrystalline cellulose. The mixture was added into a wet granulation pot, and premixed for 10 minutes.

A cutting knife was turned on, and a peristaltic pump was turned on at 20-30r/min. Water consumption was 1000g, and time was 30mins. A soft material with a water content of 49.7% was obtained.

A granulator was started with a feeding speed of 30rpm and an extrusion speed of 50rpm for 60min. A soft material was added, and a strip-shaped soft material was extruded through an orifice plate. The strip-shaped soft material was collected on a receiving tray, and kept below 45°C.

The extrusion motor was stopped. The spheronization speed was adjusted to 420rpm, and the spheronization motor was started for spheronization for 100s. The extruded strip-shaped soft material was poured into a spheronization cylinder at one time and rounded.

After the spheronization was completed, the pellets were dried in a fluidized bed at a drying temperature of 45-50°C to obtain a primary pellet core.

A fluidized bed spray granulation method was used, wherein the fluidized bed inlet air temperature was set to 55°C, the air flow was 32, and the atomization pressure was 2.5.

1600 g of the water-blocking coating solution 1 was sprayed into the fluidized bed through spray gun atomization to make the droplets uniformly wrap the pellet core. After fluidization of every 400 ml of coating solution, particles were passed through a 50-80 mesh screen, and the particles larger than 80 mesh and smaller than 50 mesh were discarded.

320 g of the water-blocking coating solution 2 was sprayed into the fluidized bed through spray gun atomization, and the particles after 50-80 mesh sieving were retained. A double-layered water-blocked particles were thus formed.

220 g of the hydrophilic coating solution was sprayed into the fluidized bed through spray gun atomization, and the particles after 50-80 mesh sieving were retained to obtain the final particles.

The microcapsules obtained in this example were three-layered microcapsules. The amount of viable probiotics in the microcapsules reached 1.25×10¹⁰ cfu/g. The amount of viable probiotics outside the microcapsules reached 7.5×10³ cfu/g. The particle size was 180-270 µm. The particle size was uniform, and the particles were ecru and shiny. After 30 days of storage at room temperature, the amount of viable probiotics in the microcapsules decreased to 1.13×10¹⁰ cfu/g; after 60 days, the amount of viable probiotics in the microcapsules decreased to 1.0×10¹⁰ cfu/g; after 120 days, the amount of viable probiotics in the microcapsules decreased to 9.9×10⁹ cfu/g, and the number of the probiotic residue on the outer surface of the microcapsules was 4.5×10³ cfu/g. The amount of viable probiotics contained in the microcapsules decreased to 1.0×10¹⁰ cfu/g after 2 hours of incubation in the simulated gastric juice.

### Example L5

Raw materials (based on 150kg of dry microcapsule particles):
Pellet core: 200g of Bacillus coagulans, 800g of microcrystalline cellulose, 1000g of water.
Water-blocking coating layer solution 1: prolamin 288g, oleic acid 112g, 75% ethanol 1200g.
Water-blocking coating layer solution 2: prolamin 54.4g, glycerin 12.8g, 75% ethanol 252.8g.
Hydrophilic coating layer solution: pectin 110g, water 2090g.

Preparation:
Bacillus coagulans powders were uniformly mixed with microcrystalline cellulose. The mixture was added into a wet granulation pot, and premixed for 10 minutes.

A cutting knife was turned on, and a peristaltic pump was turned on at 20-30r/min. Water consumption was 1000g, and time was 30mins. A soft material with a water content of 49.7% was obtained.

A granulator was started with a feeding speed of 30rpm and an extrusion speed of 47rpm for 60min. A soft material was added, and a strip-shaped soft material was extruded through an orifice plate. The strip-shaped soft material was collected on a receiving tray, and kept below 45°C.

The extrusion motor was stopped. The spheronization speed was adjusted to 400rpm, and the spheronization motor was started for spheronization for 100s. The extruded strip-shaped soft material was poured into a spheronization cylinder at one time and rounded.

After the spheronization was completed, the pellets were dried in a fluidized bed at a drying temperature of 45-50°C to obtain a primary pellet core.

A fluidized bed spray granulation method was used, wherein the fluidized bed inlet air temperature was set to 55°C, the air flow was 32, and the atomization pressure was 2.5.

320 g of the water-blocking coating solution 2 was sprayed into the fluidized bed through spray gun atomization, and the particles after 50-80 mesh sieving were retained. A double-layered water-blocked particles were thus formed.

1600 g of the water-blocking coating solution 1 was sprayed into the fluidized bed through spray gun atomization to make the droplets uniformly wrap the pellet core. After fluidization of every 400 ml of coating solution, particles were passed through a 50-80 mesh screen, and the particles larger than 80 mesh and smaller than 50 mesh were discarded.

220 g of the hydrophilic coating solution was sprayed into the fluidized bed through spray gun atomization, and the particles after 50-80 mesh sieving were retained to obtain the final particles.

The microcapsules obtained in this example were three-layered microcapsules. The amount of viable probiotics in the microcapsules reached 1.3×10¹⁰ cfu/g. The amount of viable probiotics outside the microcapsules reached 7.8×10³ cfu/g. The particle size was 180-270 µm. The particle size was uniform, and the particles were khaki and shiny. After 30 days of storage at room temperature, the amount of viable probiotics in the microcapsules decreased to 1.2×10¹⁰ cfu/g; after 60 days, the amount of viable probiotics in the microcapsules decreased to 1.15×10¹⁰ cfu/g; after 120 days, the amount of viable probiotics in the microcapsules decreased to 1.05×10¹⁰ cfu/g, and the number of the probiotic residue on the outer surface of the microcapsules was 4.5×10³ cfu/g. The amount of viable probiotics contained in the microcapsules decreased to 1.1×10¹⁰ cfu/g after 2 hours of incubation in the simulated gastric juice.

### Example L6

Raw materials (based on 150kg of dry microcapsule particles):
Pellet core: 200g of Bacillus coagulans, 800g of microcrystalline cellulose, 1000g of water.

Grease layer: MCT 200g.

Water-blocking coating layer solution 1: prolamin 288g, oleic acid 112g, 75% ethanol 1200g.

Water-blocking coating layer solution 2: prolamin 54.4g, glycerin 12.8g, 75% ethanol 252.8g.

Hydrophilic coating layer solution: pectin 110g, water 2090g.

Preparation:
Bacillus coagulans powders were uniformly mixed with microcrystalline cellulose. The mixture was added into a wet granulation pot, and premixed for 10 minutes.

A cutting knife was turned on, and a peristaltic pump was turned on at 20-30r/min. Water consumption was 1000g, and time was 30mins. A soft material with a water content of 49.7% was obtained.

A granulator was started with a feeding speed of 30rpm and an extrusion speed of 50rpm for 60min. A soft material was added, and a strip-shaped soft material was extruded through an orifice plate. The strip-shaped soft material was collected on a receiving tray, and kept below 45°C.

The extrusion motor was stopped. The spheronization speed was adjusted to 400rpm, and the spheronization motor was started for spheronization for 100s. The extruded strip-shaped soft material was poured into a spheronization cylinder at one time and rounded.

After the spheronization was completed, the pellets were dried in a fluidized bed at a drying temperature of 45-50°C to obtain a primary pellet core.

1000g of primary pellet cores were mixed with 200g of MCT and soaked for 12 hours.

A fluidized bed spray granulation method was used, wherein the fluidized bed inlet air temperature was set to 55°C, the air flow was 32, and the atomization pressure was 2.5.

1600 g of the water-blocking coating solution 1 was sprayed into the fluidized bed through spray gun atomization to make the droplets uniformly wrap the pellet core. After fluidization of every 400 ml of coating solution, particles were passed through a 50-80 mesh screen, and the particles larger than 80 mesh and smaller than 50 mesh were discarded.

320 g of the water-blocking coating solution 2 was sprayed into the fluidized bed through spray gun atomization, and the particles after 50-80 mesh sieving were retained. A double-layered water-blocked particles were thus formed.

220 g of the hydrophilic coating solution was sprayed into the fluidized bed through spray gun atomization, and the particles after 50-80 mesh sieving were retained to obtain the final particles.

The microcapsules obtained in this example were four-layered microcapsules. The amount of viable probiotics in the microcapsules reached 1.23×10¹⁰ cfu/g. The amount of viable probiotics outside the microcapsules reached 7.×10³ cfu/g. The particle size was 180-270 µm. The particle size was uniform, and the particles were ecru and shiny. After 30 days of storage at room temperature, the amount of viable probiotics in the microcapsules decreased to 1.09×10¹⁰ cfu/g; after 60 days, the amount of viable probiotics in the microcapsules decreased to 1.02×10¹⁰ cfu/g; after 120 days, the amount of viable probiotics in the microcapsules decreased to 9.8×10⁹ cfu/g, and the number of the probiotic residue on the outer surface of the microcapsules was 3.9×10³ cfu/g. The amount of viable probiotics contained in the microcapsules decreased to 1.1×10¹⁰ cfu/g after 2 hours of incubation in the simulated gastric juice.

### Example L7

Raw materials (based on 150kg of dry microcapsule particles):
Pellet core: 200g of Bacillus coagulans, 800g of microcrystalline cellulose, 1000g of water.

Water-blocking coating layer solution 1: prolamin 260g, oleic acid 140g, 75% ethanol 1200g.

Water-blocking coating layer solution 2: prolamin 54.4g, glycerin 12.8g, 75% ethanol 252.8g.

Hydrophilic coating layer solution: pectin 110g, water 2090g.

Preparation:
Bacillus coagulans powders were uniformly mixed with microcrystalline cellulose. The mixture was added into a wet granulation pot, and premixed for 10 minutes.

A cutting knife was turned on, and a peristaltic pump was turned on at 20-30r/min. Water consumption was 1000g, and time was 30mins. A soft material with a water content of 49.7% was obtained.

A granulator was started with a feeding speed of 30rpm and an extrusion speed of 50rpm for 60min. A soft material was added, and a strip-shaped soft material was extruded through an orifice plate. The strip-shaped soft material was collected on a receiving tray, and kept below 45°C.

The extrusion motor was stopped. The spheronization speed was adjusted to 400rpm, and the spheronization motor was started for spheronization for 100s. The extruded strip-shaped soft material was poured into a spheronization cylinder at one time and rounded.

After the spheronization was completed, the pellets were dried in a fluidized bed at a drying temperature of 45-50°C to obtain a primary pellet core.

A fluidized bed spray granulation method was used, wherein the fluidized bed inlet air temperature was set to 55°C, the air flow was 32, and the atomization pressure was 2.5.

1600 g of the water-blocking coating solution 1 was sprayed into the fluidized bed through spray gun atomization to make the droplets uniformly wrap the pellet core. After fluidization of every 400 ml of coating solution, particles were passed through a 50-80 mesh screen, and the particles larger than 80 mesh and smaller than 50 mesh were discarded.

320 g of the water-blocking coating solution 2 was sprayed into the fluidized bed through spray gun atomization, and the particles after 50-80 mesh sieving were retained. A double-layered water-blocked particles were thus formed.

220 g of the hydrophilic coating solution was sprayed into the fluidized bed through spray gun atomization, and the particles after 50-80 mesh sieving were retained to obtain the final particles.

The microcapsules obtained in this example were three-layered microcapsules. The amount of viable probiotics in the microcapsules reached 1.22×10¹⁰ cfu/g. The amount of viable probiotics outside the microcapsules reached 7.3×10³ cfu/g. The particle size was 180-270 µm. The particle size was uniform, and the particles were ecru and shiny. After 30 days of storage at room temperature, the amount of viable probiotics in the microcapsules decreased to 1.18×10¹⁰ cfu/g; after 60 days, the amount of viable probiotics in the microcapsules decreased to 1.15×10¹⁰ cfu/g; after 120 days, the amount of viable probiotics in the microcapsules decreased to 1.05×10¹⁰ cfu/g, and the number of the probiotic residue on the outer surface of the microcapsules was 3.9×10³ cfu/g. The amount of viable probiotics contained in the microcapsules decreased to 1.1×10¹⁰ cfu/g after 2 hours of incubation in the simulated gastric juice.

### Example L8

Raw materials (based on 150kg of dry microcapsule particles):
Pellet core: 200g of Bacillus coagulans, 800g of microcrystalline cellulose, 1000g of water.
Water-blocking coating layer solution 1: prolamin 288g, oleic acid 112g, 75% ethanol 1200g.
Water-blocking coating layer solution 2: prolamin 54.4g, glycerin 12.8g, 75% ethanol 252.8g.
Hydrophilic coating layer solution: pectin 110g, water 2090g.

Preparation:
Bacillus coagulans powders were uniformly mixed with microcrystalline cellulose. The mixture was added into a wet granulation pot, and premixed for 10 minutes.

A cutting knife was turned on, and a peristaltic pump was turned on at 20-30r/min. Water consumption was 1000g, and time was 30mins. A soft material with a water content of 49.7% was obtained.

A granulator was started with a feeding speed of 30rpm and an extrusion speed of 50rpm for 60min. A soft material was added, and a strip-shaped soft material was extruded through an orifice plate. The strip-shaped soft material was collected on a receiving tray, and kept below 45°C.

The extrusion motor was stopped. The spheronization speed was adjusted to 400rpm, and the spheronization motor was started for spheronization for 100s. The extruded strip-shaped soft material was poured into a spheronization cylinder at one time and rounded.

After the spheronization was completed, the pellets were dried in a fluidized bed at a drying temperature of 45-50°C to obtain a primary pellet core.

A fluidized bed spray granulation method was used, wherein the fluidized bed inlet air temperature was set to 59°C, the air flow was 32, and the atomization pressure was 2.5.

1600 g of the water-blocking coating solution 1 was sprayed into the fluidized bed through spray gun atomization to make the droplets uniformly wrap the pellet core. After fluidization of every 400 ml of coating solution, particles were passed through a 50-80 mesh screen, and the particles larger than 80 mesh and smaller than 50 mesh were discarded.

320 g of the water-blocking coating solution 2 was sprayed into the fluidized bed through spray gun atomization, and the particles after 50-80 mesh sieving were retained. A double-layered water-blocked particles were thus formed.

220 g of the hydrophilic coating solution was sprayed into the fluidized bed through spray gun atomization, and the particles after 50-80 mesh sieving were retained to obtain the final particles.

The microcapsules obtained in this example were three-layered microcapsules. The amount of viable probiotics in the microcapsules reached 1.27×10¹⁰ cfu/g. The amount of viable probiotics outside the microcapsules reached 7.8×10³ cfu/g. The particle size was 180-270 µm. The particle size was uniform, and the particles were ecru and shiny. After 30 days of storage at room temperature, the amount of viable probiotics in the microcapsules decreased to 1.2×10¹⁰ cfu/g; after 60 days, the amount of viable probiotics in the microcapsules decreased to 1.15×10¹⁰ cfu/g; after 120 days, the amount of viable probiotics in the microcapsules decreased to 1.05×10¹⁰ cfu/g, and the number of the probiotic residue on the outer surface of the microcapsules was 4.5×10³ cfu/g. The amount of viable probiotics contained in the microcapsules decreased to 9.7×10⁹ cfu/g after 2 hours of incubation in the simulated gastric juice.

### Comparative example L1

Raw materials (based on 150kg of dry microcapsule particles):
Pellet core: 400g of Bacillus coagulans, 600g of microcrystalline cellulose, 1000g of water.
Water-blocking coating layer solution 1: prolamin 288g, oleic acid 112g, 75% ethanol 1200g.
Water-blocking coating layer solution 2: prolamin 54.4g, glycerin 12.8g, 75% ethanol 252.8g.
Hydrophilic coating layer solution: pectin 110g, water 2090g.

Preparation:
Bacillus coagulans powders were uniformly mixed with microcrystalline cellulose. The mixture was added into a wet granulation pot, and premixed for 10 minutes.

A cutting knife was turned on, and a peristaltic pump was turned on at 20-30r/min. Water consumption was 1000g, and time was 30mins. A soft material with a water content of 49.7% was obtained.

A granulator was started with a feeding speed of 30rpm and an extrusion speed of 50rpm for 60min. A soft material was added, and a strip-shaped soft material was extruded through an orifice plate. The strip-shaped soft material was collected on a receiving tray, and kept below 45°C.

The extrusion motor was stopped. The spheronization speed was adjusted to 400rpm, and the spheronization motor was started for spheronization for 100s. The extruded strip-shaped soft material was poured into a spheronization cylinder at one time and rounded.

After the spheronization was completed, the pellets were dried in a fluidized bed at a drying temperature of 45-50°C to obtain a primary pellet core.

A fluidized bed spray granulation method was used, wherein the fluidized bed inlet air temperature was set to 55°C, the air flow was 32, and the atomization pressure was 2.5.

1600 g of the water-blocking coating solution 1 was sprayed into the fluidized bed through spray gun atomization to make the droplets uniformly wrap the pellet core. After fluidization of every 400 ml of coating solution, particles were passed through a 50-80 mesh screen, and the particles larger than 80 mesh and smaller than 50 mesh were discarded.

320 g of the water-blocking coating solution 2 was sprayed into the fluidized bed through spray gun atomization, and the particles after 50-80 mesh sieving were retained. A double-layered water-blocked particles were thus formed.

220 g of the hydrophilic coating solution was sprayed into the fluidized bed through spray gun atomization, and the particles after 50-80 mesh sieving were retained to obtain the final particles.

The primary pellet cores obtained in this example were not shaped.

### Comparative example L2

Raw materials (based on 150kg of dry microcapsule particles):
Pellet core: 200g of Bacillus coagulans, 800g of microcrystalline cellulose, 1000g of water.
Water-blocking coating layer solution 1: prolamin 48.8g, oleic acid 31.2g, 75% ethanol 1200g.
Water-blocking coating layer solution 2: prolamin 127.68g, glycerin 40.32g, 75% ethanol 632g.
Hydrophilic coating layer solution: pectin 110g, water 2090g.

Preparation:
Bacillus coagulans powders were uniformly mixed with microcrystalline cellulose. The mixture was added into a wet granulation pot, and premixed for 10 minutes.

A cutting knife was turned on, and a peristaltic pump was turned on at 20-30r/min. Water consumption was 1000g, and time was 30mins. A soft material with a water content of 49.7% was obtained.

A granulator was started with a feeding speed of 30rpm and an extrusion speed of 50rpm for 60min. A soft material was added, and a strip-shaped soft material was extruded through an orifice plate. The strip-shaped soft material was collected on a receiving tray, and kept below 45°C.

The extrusion motor was stopped. The spheronization speed was adjusted to 400rpm, and the spheronization motor was started for spheronization for 100s. The extruded strip-shaped soft material was poured into a spheronization cylinder at one time and rounded.

After the spheronization was completed, the pellets were dried in a fluidized bed at a drying temperature of 45-50°C to obtain a primary pellet core.

A fluidized bed spray granulation method was used, wherein the fluidized bed inlet air temperature was set to 55°C, the air flow was 32, and the atomization pressure was 2.5.

1600 g of the water-blocking coating solution 1 was sprayed into the fluidized bed through spray gun atomization to make the droplets uniformly wrap the pellet core. After fluidization of every 400 ml of coating solution, particles were passed through a 50-80 mesh screen, and the particles larger than 80 mesh and smaller than 50 mesh were discarded.

320 g of the water-blocking coating solution 2 was sprayed into the fluidized bed through spray gun atomization, and the particles after 50-80 mesh sieving were retained. A double-layered water-blocked particles were thus formed.

220 g of the hydrophilic coating solution was sprayed into the fluidized bed through spray gun atomization, and the particles after 50-80 mesh sieving were retained to obtain the final particles.

In this example, small lumps of different sizes and irregular shapes were obtained. The amount of viable probiotics in the microcapsules reached 6.8×10⁸ cfu/g, and the amount of viable probiotics outside the microcapsules reached 4.2×10⁶ cfu/g. The particle size was 180-270 µm. After 30 days of storage at room temperature, the amount of viable probiotics in the microcapsules decreased to 4.8×10⁸ cfu/g; after 60 days, the amount of viable probiotics in the microcapsules decreased to 8.0×10⁷ cfu/g; after 120 days, the amount of viable probiotics in the microcapsules decreased to 2.2×10⁷ cfu/g, and the number of the probiotic residue on the outer surface of the microcapsules was 9.4×10⁵ cfu/g. The amount of viable probiotics contained in the microcapsules decreased to 3.0×10⁸ cfu/g after 2 hours of incubation in the simulated gastric juice.

### Comparative example L3

Raw materials (based on 150kg of dry microcapsule particles):
Pellet core: 200g of Bacillus coagulans, 800g of microcrystalline cellulose, 1000g of water.
Water-blocking coating layer solution 1: prolamin 48.8g, oleic acid 31.2g, 75% ethanol 1200g.
Water-blocking coating layer solution 2: prolamin 127.68g, glycerin 40.32g, 75% ethanol 632g.
Hydrophilic coating layer solution: pectin 110g, water 2090g.

Preparation:
Bacillus coagulans powders were uniformly mixed with microcrystalline cellulose. The mixture was added into a wet granulation pot, and premixed for 10 minutes.

A cutting knife was turned on, and a peristaltic pump was turned on at 20-30r/min. Water consumption was 1000g, and time was 30mins. A soft material with a water content of 49.7% was obtained.

A granulator was started with a feeding speed of 30rpm and an extrusion speed of 50rpm for 60min. A soft material was added, and a strip-shaped soft material was extruded through an orifice plate. The strip-shaped soft material was collected on a receiving tray, and kept below 45°C.

The extrusion motor was stopped. The spheronization speed was adjusted to 400rpm, and the spheronization motor was started for spheronization for 100s. The extruded strip-shaped soft material was poured into a spheronization cylinder at one time and rounded.

After the spheronization was completed, the pellets were dried in a fluidized bed at a drying temperature of 45-50°C to obtain a primary pellet core.

A fluidized bed spray granulation method was used, wherein the fluidized bed inlet air temperature was set to 70°C, the air flow was 32, and the atomization pressure was 2.5.

1600 g of the water-blocking coating solution 1 was sprayed into the fluidized bed through spray gun atomization to make the droplets uniformly wrap the pellet core. After fluidization of every 400 ml of coating solution, particles were passed through a 50-80 mesh screen, and the particles larger than 80 mesh and smaller than 50 mesh were discarded.

320 g of the water-blocking coating solution 2 was sprayed into the fluidized bed through spray gun atomization, and the particles after 50-80 mesh sieving were retained. A double-layered water-blocked particles were thus formed.

220 g of the hydrophilic coating solution was sprayed into the fluidized bed through spray gun atomization, and the particles after 50-80 mesh sieving were retained to obtain the final particles.

The microcapsules obtained in this example were three-layered microcapsules. The amount of viable probiotics in the microcapsules reached 2.3×10⁸ cfu/g, and the amount of viable probiotics outside the microcapsules reached 2.8×10² cfu/g. The particle size was 180-270 µm. The particle size was uniform, and the particles were ecru and shiny. After 30 days of storage at room temperature, the amount of viable probiotics in the microcapsules decreased to 1.3×10⁸ cfu/g; after 60 days, the amount of viable probiotics in the microcapsules decreased to 9.8×10⁷ cfu/g; after 120 days, the amount of viable probiotics in the microcapsules decreased to 5.8×10⁷ cfu/g, and the number of the probiotic residue on the outer surface of the microcapsules was 1.2×10³ cfu/g. The amount of viable probiotics contained in the microcapsules decreased to 1.0×10⁸ cfu/g after 2 hours of incubation in the simulated gastric juice.

The preparation process and microcapsule particle test results of the above examples were summarized in the following table:

| | | Microcapsule Examples L1-8 and Comparative Example L1-3 | | | | | | | | | | |
|---|---|---|---|---|---|---|---|---|---|---|---|---|
| Process parameters | Param eter range | Ex. L1 | Ex. L2 | Ex. L3 | Ex. L4 | Ex. L5 | Ex. L6 | Ex. L7 | Ex. L8 | Comparat ive Ex. L1 | Comparat ive Ex. L2 | Comparat ive Ex. L3 |
| Weight ratio of probiotics to microcrystalline cellulose/% | 20-30 | 25 | 25 | 25 | 22 | 25 | 25 | 25 | 25 | 40 | 25 | 25 |
| Part by weight of water consumption for pellet cores | 90-110 | 100 | 100 | 100 | 100 | 100 | 100 | 100 | 100 | 100 | 100 | 100 |
| Composition of the first layer of water blocking layer | / | prola min + oleic acid | prola min + oleic acid | prola min + oleic acid | prola min + oleic acid | prola min + glyc erin | MC T | MC T | prola min + oleic acid | prola min + oleic acid | prola min + oleic acid | prola min + oleic acid |
| Composition of the second layer of water blocking layer | / | prola min + glyc erin | prola min + glyc erin | prola min + glyc erin | prola min + glyc erin | prola min + oleic acid | prola min + oleic acid | prola min + oleic acid | prola min + glyc erin | prola min + glyc erin | prola min + glyc erin | prola min + glyc erin |
| Composition of the third layer of water blocking layer | / | / | / | / | / | / | prola min + glyc erin | prola min + glyc erin | / | / | / | / |
| Part by weight of solution of water-blocking coating layer of oleic acid | 1500-1 800 | 1600 | 1500 | 1800 | 1600 | 1600 | 1600 | 1600 | 1600 | 1600 | 320 | 1600 |
| Part by weight of solution of water-blocking coating layer of glycerin | 300-34 0 | 320 | 320 | 320 | 320 | 310 | 320 | 320 | 320 | 320 | 800 | 320 |
| Weight ratio of oleic acid to prolamin/% | 0.35-0.4 | 0.39 | 0.39 | 0.35 | 0.39 | 0.39 | 0.39 | 0.35 | 0.39 | 0.39 | 0.39 | 0.39 |
| Weight ratio of glycerin to prolamin/% | 0.2-0.2 5 | 0.24 | 0.24 | 0.24 | 0.24 | 0.24 | 0.24 | 0.24 | 0.24 | 0.24 | 0.24 | 0.24 |
| Feeding speed of granulator/RPM, | 28-32 | 30 | 30 | 30 | 30 | 30 | 30 | 30 | 30 | 30 | 30 | 30 |
| Extrusion speed of granulator/RPM | 45-50 | 50 | 50 | 50 | 50 | 47 | 50 | 50 | 50 | 50 | 50 | 50 |
| Spheronization speed of granulator/RPM | 350-45 0 | 400 | 400 | 420 | 420 | 400 | 400 | 380 | 400 | 400 | 400 | 400 |
| Inlet air temperature of fluidized bed/°C | 55-60 | 55 | 55 | 55 | 55 | 55 | 55 | 55 | 59 | 55 | 55 | 70 |
| Air flow of fluidized bed (m³/h) | 30-34 | 32 | 32 | 32 | 32 | 32 | 32 | 32 | 32 | 32 | 32 | 32 |
| Atomization pressure/bar | 2-3 | 2.5 | 2.5 | 2.5 | 2.5 | 2.5 | 2.5 | 2.5 | 2.5 | 2.5 | 2.5 | 2.5 |

| Test results of microcapsule examples Ll-8 | | | | | | | | |
|---|---|---|---|---|---|---|---|---|
| Tested items | Example L1 | Example L2 | Example L3 | Example L4 | Example L5 | Example L6 | Example L7 | Example L8 |
| Microcapsule morphology | Round particles Shiny spherical | Round particles Shiny spherical | Round particles Shiny spherical | Round particles Shiny spherical | Round particles Shiny spherical | Round particles Shiny spherical | Round particles Shiny spherical | Round particles Shiny spherical |
| Color of microcapsule | Ecru | Ecru | Ecru | Ecru | Khaki | Ecru | Ecru | Ecru |
| Number of viable probiotics in the microcapsules (after 0 day)/cfu | 1.3×10¹⁰ | 1.35×10¹⁰ | 1.2×10¹⁰ | 1.3×10¹⁰ | 1.3×10¹⁰ | 1.23×10¹⁰ | 1.22×10¹⁰ | 1.27×10¹⁰ |
| Number of viable probiotics outside microcapsules (after 0 day)/cfu | 7.8×10³ | 8.2×10³ | 7.5×10³ | 7.8×10³ | 7.8×10³ | 7.0×10³ | 7.3×10³ | 7.8×10³ |
| Number of viable probiotics in the microcapsules (after 30 days)/cfu | 1.2×10¹⁰ | 1.28×10¹⁰ | 1.1×10¹⁰ | 1.2×10¹⁰ | 1.2×10¹⁰ | 1.09×10^{1O} | 1.18×10¹⁰ | 1.2×10¹⁰ |
| Number of viable probiotics in the microcapsules (after 60 days)/cfu | 1.15×10¹⁰ | 1.14×10¹⁰ | 9.8×10⁹ | 1.15×10¹⁰ | 1.15×10¹⁰ | 1.02×10¹⁰ | 1.15×10¹⁰ | 1.15×10¹⁰ |
| Number of viable probiotics in the microcapsules (after 120 days)/cfu | 1.05×10¹⁰ | 1.00×10¹⁰ | 9.1×10⁹ | 1.05×10¹⁰ | 1.05×10¹⁰ | 9.8×10¹⁰ | 1.05×10¹⁰ | 1.05×10¹⁰ |
| Number of probiotic residue on the outer surface of microcapsules/cfu | 4.5×10³ | 7×10³ | 4.0×10³ | 4.5×10³ | 4.5×10³ | 3.9×10³ | 4.0×10³ | 4.5×10³ |
| Acid resistance (Number of viable probiotics in microcapsules after 2h incubation in simulated gastric juice/cfu) | 1.1×10¹⁰ | 1.05×10¹⁰ | 1.15×10¹⁰ | 1.1×10¹⁰ | 1.1×10¹⁰ | 1.1×10¹⁰ | 1.1×10¹⁰ | 9.7×10⁹ |

| Test results of microcapsule Comparative Examples L1-3 | | | |
|---|---|---|---|
| Tested items | Example L1 | Example L2 | Example L3 |
| Microcapsule morphology | Primary pellet core is not shaped | Particles are bonded together to form lumps | Round particles Shiny |
| Number of viable probiotics in the microcapsules (after 0 day)/cfu | / | 6.8×10⁸ | 2.3×10⁸ |
| Number of viable probiotics outside microcapsules (after 0 day)/cfu | / | 4.2×10⁶ | 2.8×10² |
| Number of viable probiotics in the microcapsules (after 30 days)/cfu | / | 4.8×10⁸ | 1.3×10⁸ |
| Number of viable probiotics in the microcapsules (after 60 days)/cfu | / | 8.0×10⁷ | 9.8×10⁷ |
| Number of viable probiotics in the microcapsules (after 120 days)/cfu | / | 2.2×10⁷ | 5.8×10^{7'} |
| Number of probiotic residue on the outer surface of microcapsules/cfu | / | 9.4×10⁵ | 1.2×10³ |
| Acid resistance (Number of viable probiotics in microcapsules after 2h incubation in simulated gastric juice/cfu) | / | 3.0×10⁸ | 1.0×10⁸ |

### M. Preparation of microcapsules (whey protein peptide microcapsules)

### Examples Ml-6, Comparative Examples Ml-2

The basic process of preparation of whey protein peptide microcapsules was similar to that of probiotic microcapsules. The specific parameter differences were shown in the following table:

| | Examples M1-4 and Comparative Example M1-2 of whey protein peptide microcapsules | | | | | | |
|---|---|---|---|---|---|---|---|
| Process parameters | Parameter range | Example M1 | Example M2 | Example M3 | Example M4 | Comparati ve Example M1 | Comparati ve Example M2 |
| Weight ratio of whey protein peptide to microcrystalline cellulose/% | 20-40 | 30 | 30 | 30 | 30 | 30 | 40 |
| Part by weight of water consumption for pellet cores | 90-110 | 100 | 100 | 100 | 100 | 100 | 100 |
| Composition of the first layer of water blocking layer | / | prolamin + oleic acid | prolamin + oleic acid | prolamin + oleic acid | MCT | prolamin + oleic acid | MCT |
| Composition of the second layer of water blocking layer | / | prolamin + glycerin | prolamin + glycerin | prolamin + glycerin | prolamin + oleic acid | prolamin + glycerin | prolamin + oleic acid |
| Composition of the third layer of water blocking layer | / | / | / | / | prolamin + glycerin | / | prolamin + glycerin |
| Part by weight of solution of water-blocking coating layer of oleic acid | 1500-1800 | 1600 | 1500 | 1600 | 1600 | 320 | 1600 |
| Part by weight of solution of water-blocking coating layer of glycerin | 300-340 | 320 | 320 | 320 | 320 | 320 | 320 |
| Weight ratio of oleic acid to prolamin/% | 0.35-0.4 | 0.39 | 0.39 | 0.35 | 0.39 | 0.39 | 0.39 |
| Weight ratio of glycerin to prolamin/% | 0.2-0.25 | 0.24 | 0.24 | 0.24 | 0.24 | 0.24 | 0.24 |
| Feeding speed of granulator/RPM, | 28-32 | 30 | 30 | 30 | 30 | 30 | 30 |
| Extrusion speed of granulator/RPM | 45-50 | 50 | 50 | 50 | 50 | 50 | 50 |
| Spheronization speed of granulator/RPM | 350-450 | 400 | 400 | 420 | 420 | 400 | 400 |
| Inlet air temperature of fluidized bed/°C | 55-70 | 60 | 65 | 60 | 60 | 60 | 85 |
| Air flow of fluidized bed (m3/h) | 30-34 | 32 | 32 | 32 | 32 | 32 | 50 |
| Atomization pressure/bar | 2-3 | 2.5 | 2.5 | 2.5 | 2.5 | 2.5 | 2.5 |

| Test results of microcapsule Examples M1-4, Comparative Examples M1-2 | | | | | | |
|---|---|---|---|---|---|---|
| Tested items | Example M1 | Example M2 | Example M3 | Example M4 | Comparative Example M1 | Comparative Example M2 |
| Microcapsule morphology | Round particles Shiny spherical | Round particles Shiny spherical | Round particles Shiny spherical | Round particles Shiny spherical | Round particles Shiny spherical | No shaped particles |
| Color of microcapsule | Ecru | Ecru | Ecru | Ecru | Ecru | / |
| Taste of microcapsules | No obvious bitter and sour taste | No obvious bitter and sour taste | No obvious bitter and sour taste | No obvious bitter and sour taste | Obvious bitter and sour taste | No obvious bitter and sour taste |
| Content of peptides in microcapsules (mg/g) | 65 | 65 | 60 | 60 | 68 | / |

## Claims

1. A microcapsule having one or more layers of embedding structure, comprising: a pellet core and optionally at least one layer of shell coating the pellet core;
wherein the pellet core includes an embedded substance and a microcapsule core material, and
the at least one layer of shell coating the pellet core includes one, two or more layers of wall material.

2. The microcapsule according to claim 1, wherein the outer diameter of the pellet core of the microcapsule is about 50-500 µm (preferably about 50 to about 300 µm), and the outer diameter of the single-layered or multi-layered coated microcapsule is about 200 to about 1000 µm (preferably about 100 to about 500 µm).

3. The microcapsule according to claim 1 or 2, wherein the embedded substance includes a functional active substance selected from one or more of functional polysaccharides, functional lipids, functional proteins/peptides/amino acids, micro-ecological regulators, vitamins and minerals;
preferably, the weight ratio of the embedded substance to the microcapsule core material is 1:6-1:2.5 (more preferably 1:5-1:4).

4. The microcapsule according to claim 3, wherein the functional polysaccharide is selected from one or more of chitosan, tea polysaccharide, dietary fiber, and dextran; preferably, the functional lipid is selected from one or more of lecithin, EPA and DHA; preferably, the functional protein/peptide/amino acid is selected from one or more of taurine, lactoferrin, immunoglobulin, and whey protein peptide; preferably, the micro-ecological regulator is selected from one or more of probiotics, prebiotics, and synbiotics.

5. The microcapsule according to any one of claims 1 to 4, wherein the wall material is selected from one or a combination of more of:
vegetable protein, such as soy protein, rice protein, wheat protein, corn protein, etc.; preferably corn protein, or
animal protein, such as whey protein, casein, etc.; preferably whey protein concentrate (WPC), whey protein isolate (WPI) or whey protein peptide, especially preferably whey protein isolate (WPI); or
greases, such as greases with a melting point of 40°C or higher, preferably with a melting point of 40-50°C, particularly preferably palm oil, medium chain glycerides, hydrogenated greases (e.g., hydrogenated palm oil, hardened oil, hydrogenated soybean oil), lecithin, cocoa butter substitutes, palm oil monoglycerides, coconut oil, soybean oil, peanut oil, sunflower oil, or
other materials, such as glycerin, oleic acid, sodium alginate, shellac, CMC-Na, gellan, xanthan gum, k-carrageenan, cellulose acetate phthalate, maltodextrin, starch, dextrin, sucrose, lactose, dextran, corn syrup, pectin, gum arabic, chitosan, acetylated mono- or di-glyceride fatty acid esters, konjac gum, carrageenan, wax or gelatin, etc.;
preferably, the wall material is selected from whey protein or grease, particularly preferably WPI or MCT.

6. The microcapsule according to any one of claims 1 to 5, wherein the microcapsule core material comprises one or a combination of more of:
vegetable protein, such as soy protein, rice protein, wheat protein, corn protein, etc.; preferably corn protein, or
animal protein, such as whey protein, casein, etc.; preferably whey protein concentrate (WPC), whey protein isolate (WPI) or whey protein peptide, especially preferably whey protein isolate (WPI); or
greases, such as greases with a melting point of 40°C or higher, preferably with a melting point of 40-50°C, particularly preferably palm oil, medium chain glycerides, hydrogenated greases (e.g., hydrogenated palm oil, hardened oil, hydrogenated soybean oil), lecithin, cocoa butter substitutes, palm oil monoglycerides, coconut oil, soybean oil, peanut oil, sunflower oil, or
other materials, such as microcrystalline cellulose (MCC), glycerin, oleic acid, sodium alginate, shellac, CMC-Na, gellan, xanthan gum, k-carrageenan, cellulose acetate phthalate, maltodextrin, starch, dextrin, sucrose, lactose, dextran, corn syrup, pectin, gum arabic, chitosan, acetylated mono- or di-glyceride fatty acid esters, konjac gum, carrageenan, wax or gelatin, etc.;
more preferably, the microcapsule core material includes microcrystalline cellulose (MCC).

7. The microcapsule according to any one of claims 1 to 6, wherein the embedded substance includes probiotics;
preferably, the probiotics are selected from one or more of Bifidobacterium adolescentis, Bifidobacterium animalis (Bifidobacterium lactis), Bifidobacterium bifidum, Bifidobacterium breve, Bifidobacterium infantis, Bifidobacterium longum, Lactobacillus acidophilus, Lactobacillus casei, Lactobacillus crispatus, Lactobacillus delbrueckii subsp. Bulgaricus, Lactobacillus delbrueckii subsp. lactis, Lactobacillus fermentum, Lactobacillus gasseri, Lactobacillus helveticus, Lactobacillus johnsonii, Lactobacillus paracasei, Lactobacillus plantarum, Lactobacillus reuteri, Lactobacillus rhamnosus, Lactobacillus salivarius, Streptococcus thermophilus, and Bacillus coagulans.

8. A method for preparing microcapsules having one or more layers of embedding structure, comprising the following steps:
(1) preparation of a primary pellet core: uniformly mixing an embedded substance and a microcapsule core material, optionally curing, and filtering to collect wet particles, and then drying to obtain a primary pellet core; and optionally
(2) preparation of multi-layered microcapsules: uniformly coating a layer of wall material outside the pellet core particles described above, optionally spraying a curing solution to cure a capsule wall, drying the prepared microcapsules, collecting dry particles of microcapsules or further coating them once or more times.

9. The preparation method according to claim 8, wherein in the step (1), the pellet core is prepared by extrusion spheronization granulation method, centrifugal granulation method, acute angle extrusion granulation method, or fluidized bed spray granulation method (preferably the extrusion spheronization granulation method or the centrifugal granulation method), and the obtained pellet core is dried and collected;
preferably, in the step (2), the wall material is coated by means of fluidized bed spray.

10. Use of the microcapsule according to any one of the preceding claims in food and health product industries (e.g., as a dietary supplement), for example, in heat-processed food or frozen food; preferably, in fermented flavored food, beverages, chocolates, candies such as chewing gum, baked food such as puddings, fruit and vegetable juice foods; more preferably, as dairy additives added to milk, yogurt, cheese, ice cream, milk powder, or dairy beverages (most preferably yogurt).

11. A probiotic microcapsule having one or more layers of embedding structure, comprising: probiotic core particles and optionally at least one layer of shell coating the probiotic core particles,
wherein the probiotic core particles include a core material and a first layer of wall material, wherein the core material includes one or more kinds of probiotic powder or probiotic mud, and the core material is coated with the first layer of wall material, and
the at least one layer of shell coating the probiotic core particles includes one, two or more layers of wall materials, which are respectively a second layer of wall material, a third layer of wall material, or more layers of wall material.

12. The probiotic microcapsule according to claim 11, wherein based on 1000 parts by weight of dry microcapsule particles, the weight ratio of the composition of the microcapsule described above is: 50 to 500 parts by weight of probiotic mud or probiotic powder; about 0-950 parts by weight, preferably about 150-950 parts by weight or 0-350 parts by weight of the first layer of wall material; about 0-950 parts by weight, preferably about 150-950 parts by weight or 0-350 parts by weight of the second layer of wall material; about 0 to 350 parts by weight of the third layer of wall material; and about 0 to 250 parts by weight of the fourth layer of wall material.

13. The probiotic microcapsule according to claim 11 or 12, wherein the outer diameter of the probiotic core particles obtained after the first layer of coating is about 50-500 µm (preferably about 50 to about 300 µm), and the outer diameter of the double-layered or multi-layered microcapsule is about 200 to about 1000 µm (preferably about 100 to about 500 µm).

14. The probiotic microcapsule according to any one of claims 11-13, wherein the wall material is selected from one or a combination of more of:
vegetable protein, such as soy protein, rice protein, wheat protein, corn protein, etc.; preferably corn protein, or
animal protein, such as whey protein, casein, etc.; preferably whey protein concentrate (WPC), whey protein isolate (WPI) or whey protein peptide, especially preferably whey protein isolate (WPI); or
greases, such as greases with a melting point of 40°C or higher, preferably with a melting point of 40-50°C, particularly preferably palm oil, medium chain glycerides, hydrogenated greases (e.g., hydrogenated palm oil, hardened oil, hydrogenated soybean oil), lecithin, cocoa butter substitutes, palm oil monoglycerides, coconut oil, soybean oil, peanut oil, sunflower oil, or
other materials, such as glycerin, oleic acid, sodium alginate, shellac, CMC-Na, gellan, xanthan gum, k-carrageenan, cellulose acetate phthalate, maltodextrin, starch, dextrin, sucrose, lactose, dextran, corn syrup, pectin, gum arabic, chitosan, acetylated mono- or di-glyceride fatty acid esters, konjac gum, carrageenan, wax or gelatin, etc.

15. The probiotic microcapsule according to any one of claims 11 to 14, wherein the wall material is selected from whey protein or grease, wherein WPI or MCT is particularly preferred.

16. The probiotic microcapsule according to any one of claims 11-15, wherein the probiotic is selected from one or more of Bifidobacterium adolescentis, Bifidobacterium animalis (Bifidobacterium lactis), Bifidobacterium bifidum, Bifidobacterium breve, Bifidobacterium infantis, Bifidobacterium longum, Lactobacillus acidophilus, Lactobacillus casei, Lactobacillus crispatus, Lactobacillus delbrueckii subsp. Bulgaricus, Lactobacillus delbrueckii subsp. lactis, Lactobacillus fermentum, Lactobacillus gasseri, Lactobacillus helveticus, Lactobacillus johnsonii, Lactobacillus paracasei, Lactobacillus plantarum, Lactobacillus reuteri, Lactobacillus rhamnosus, Lactobacillus salivarius, Streptococcus thermophilus, and Bacillus coagulans.

17. A preparation method of probiotic microcapsules having one or more layers of embedding structure, comprising the following steps:
(1) preparation of single-layered microcapsules: uniformly mixing probiotic powder or probiotic mud with the first wall material and other materials, optionally curing, then filtering to collect wet particles, and then drying to obtain dry particles of single-layered microcapsule; and optionally
(2) preparation of multi-layered microcapsules: using the above-mentioned dry particles of single-layered microcapsule as probiotic core particles, uniformly coating a layer of wall material outside the probiotic core particles, optionally spraying a curing solution to cure the capsule wall, drying the prepared microcapsules, and collecting dry particles of double-layered microcapsules or further coating the dry particles of double-layered microcapsules once or more times.

18. A preparation method of probiotic microcapsules having one or more layers of embedding structure, comprising the following steps:
(1) coating of the first layer of microcapsules:
a. Optionally, adding a binder to probiotic mud or probiotic powder for granulation;
b. Optionally, pre-treating the first wall material:
the first wall material is mixed with water, fully dissolved, and then alternately heated and cooled to form a stable gel:
(i) preferably, the first wall material is mixed with water, and stirred at a low temperature of about 2 to 8°C for about 4 to 16 hours;
(ii) more preferably, the first wall material is further heated at about 75 to about 96°C for about 30 to about 180 minutes;
(iii) more preferably, the first wall material is cooled immediately at a cooling temperature of about -20 to about 4°C, and stored at about 4°C for about 12 to about 60 hours to obtain a gel solution of the first wall material;
preferably, wherein the above-mentioned first wall material is WPI, preferably with about 150 to about 950 parts by weight, or more preferably with about 300 to 750 parts by weight;
c. uniformly wrapping droplets of the first wall material on probiotic powder, probiotic mud, or the particles obtained in step a described above;
d. curing: uniformly wrapping droplets of a curing solution on the particles obtained in step c described above;
e. drying;
(2) coating of optional second layer of microcapsule:
f. the dry particles of single-layered microcapsule obtained in step (1) are used as probiotic core particles and mixed with the second layer of wall material to prepare double-layered microcapsules, wherein the second wall material is preferably grease (preferably grease with a melting point of 40-50°C, especially palm oil or MCT) or whey protein, preferably with about 0-350 parts by weight;
g. drying the microcapsules prepared in step f and collecting dry particles of double-layered microcapsule;
(3) Optional third or more coating:
j. The double-layered microcapsules obtained in step (2) are further coated for the third or more times according to the aforementioned method;
wherein the third wall material is preferably grease (preferably grease with a melting point of 40-50°C, especially palm oil or MCT) or whey protein, preferably with about 0-350 parts by weight.

19. A probiotic microcapsule having one or more layers of embedding structure, which is prepared according to the preparation method of probiotic microcapsules of any one of claims 17-18.

20. Use of the probiotic microcapsule according to any one of the preceding claims in food and health product industries (e.g., dietary supplements), for example, in heat-processed foods or frozen foods, preferably in fermented flavored foods, beverages, chocolate, candy such as chewing gum, baked food such as pudding, fruit and vegetable juice food; more preferably, as dairy additives, for example, added to milk, yogurt, cheese, ice cream, milk powder, dairy beverages.

21. A preparation method of probiotic microcapsules having one or more layers of embedding structure, comprising the following steps:
(1) Coating of the first layer of microcapsules: uniformly mixing the first wall material with probiotic powder or probiotic mud, then granulating (preferably extrusion granulation method or fluidized bed spray granulation method), optionally curing by adding into a curing agent solution dropwise, filtering and collecting the microcapsule particles, and optionally drying the resulting microcapsules to obtain dry particles of single-layered microcapsule; and optionally
(2) Coating of the second layer of microcapsules: uniformly mixing the microcapsules obtained in step (1) with the second wall material, preparing microcapsules by a fluidized bed spray method, drying the prepared microcapsules and collecting dry particles of double-layered microcapsules;
(3) Optionally, further coating the microcapsules obtained in step (2) in a fluidized bed for a third or more times.

22. The preparation method of probiotic microcapsules according to claim 21, wherein based on 1000 parts by weight of dry microcapsule particles, the probiotic mud or probiotic powder is about 50 to 500 parts by weight, preferably about 250-400 parts by weight; the first layer of wall material is about 150 to about 950 parts by weight, preferably about 450-750 parts by weight; the second layer of wall material is about 0 to about 350 parts by weight, preferably about 200-300 parts by weight; the third layer of wall material is about 0 to about 350 parts by weight; and the fourth layer of wall material is about 0 to about 260 parts by weight.

23. The preparation method of probiotic microcapsules according to any one of claims 21-22, wherein the first wall material is whey protein,
preferably, wherein the whey protein is subjected to a pre-denaturation treatment: the whey protein is mixed with water, fully dissolved, and then alternately cooled and heated to form a stable gel;
preferably, wherein the whey protein is uniformly mixed with water and stirred at a low temperature of about 2 to 8°C for about 4 to 16 hours; more preferably, the whey protein solution is further heated at about 75 to about 96°C for about 30 to about 180 minutes; preferably, the whey protein solution is cooled immediately at a cooling temperature of about -20 to about 4 °C, and then stored at about 4 °C for about 10-60 hours or more to obtain a whey protein gel solution.

24. The preparation method of probiotic microcapsules according to any one of claims 21 to 23, wherein in the step of (2) coating of the second layer of microcapsules, a fluidized bed method is used for the second coating, preferably wherein aqueous solution, emulsion or gel solution of the second wall material heated to about 25 to 75°C is used, preferably wherein the inlet air temperature of the fluidized bed is 30 to 80°C.

25. The preparation method of probiotic microcapsules according to any one of claims 21 to 24, wherein in the step of (2) coating of the second layer of microcapsules, the second wall material is protein, preferably WPI.

26. A food or health product containing probiotic microcapsule, wherein based on the total weight of the probiotic microcapsule-containing food or health product, the weight percentage of probiotic microcapsules is about 0.02 to 1%, preferably about 0.04 to 0.5%, more preferably, about 0.06-0.15%;
preferably, wherein the food or health product containing probiotic microcapsule further contains honey, syrup, fruit juice, preferably honey, preferably, wherein based on the total weight of a mixture of microcapsule and honey, the weight percentage of the honey is about 80%-99.9%;
especially preferably, the ratio of the microcapsule to the honey is 1:1000 to 1:10, preferably 1:500 to 1:50, more preferably 1:100.

27. The food or health product containing probiotic microcapsule according to claim 26, wherein the probiotic microcapsules have one or more layers of embedding structure, including: probiotic core particles and optionally at least one layer of shell coating the probiotic core particles;
wherein the probiotic core particles include a core material and a first layer of wall material, wherein the core material includes one or more kinds of probiotic powder or probiotic mud, and the core material is coated by the first layer of wall material, and
the at least one layer of shell coating the probiotic core particles includes one, two or more layers of wall materials, which are respectively a second layer of wall material, a third layer of wall material, or more layers of wall material;
preferably, based on 1000 parts by weight of dry microcapsule particles, the weight ratio of the composition of the above-mentioned microcapsules is: 50 to 500 parts by weight of probiotic mud or probiotic powder; about 0 to 950 parts by weight, preferably about 150 to 950 parts by weight or 0 to 350 parts by weight of the first layer of wall material; about 0 to 950 parts by weight, preferably about 150 to 950 parts by weight or 0 to 350 parts by weight of the second layer of wall material; about 0 to 350 parts by weight of the third layer of wall material; and about 0 to 250 parts by weight of the fourth layer of wall material.

28. The food or health product containing probiotic microcapsule according to claim 26 or 27, wherein the food or health product is selected from one or more of milk and dairy products, fermented flavored foods, beverages, chocolates, candies, baked foods or fruit and vegetable juice foods,
preferably, wherein the food or health product containing probiotic microcapsule is yogurt, preferably, wherein the yogurt comprises: based on the total weight of the probiotic microcapsule-containing yogurt, about 89-94% of raw milk/reconstituted milk, about 5.5-10.9% of white granulated sugar, about 0.1-0.5% of stabilizer, about 30-100 U/ton of leavening agent, and about 0.02-0.5%, preferably about 0.06-0.15% of probiotic microcapsules;
preferably, wherein the food or health product containing probiotic microcapsule is native cheese or processed cheese, preferably, wherein the native cheese also includes raw milk, edible salt, leavening agent or chymosin; more preferably, wherein the cheese contains about 0.8-1.8% of edible salt, about 0.001-0.005% of leavening agent, about 0.001-0.005% of chymosin, and about 0.02-0.5% of probiotic microcapsules (preferably about 0.06-0.15%), and the rest is raw milk;
preferably, wherein the food or health product containing probiotic microcapsule is a milk beverage, preferably, the milk beverage contains about 0.02-1% of probiotic microcapsules (preferably about 0.04-0.5%),
also preferably, wherein the food or health product containing probiotic microcapsule further contains honey, and more preferably, the ratio of the microcapsule to the honey is 1:1000 to 1:10, preferably 1:500 to 1:50, more preferably, 1:100;
preferably, wherein the food or health product containing probiotic microcapsule is a solid dairy product, preferably, wherein the solid dairy product contains about 0.02 to 1% of probiotic microcapsules;
preferably, wherein the food or health product containing probiotic microcapsule is a solid beverage, preferably, wherein the solid beverage contains about 0.02 to 1% of probiotic microcapsules;
preferably, wherein the food or health product containing probiotic microcapsule is ice cream, preferably, wherein the ice cream contains about 0.02 to 1% of probiotic microcapsules (preferably about 0.04 to 0.5%).

29. A method for preparing the food or health product containing probiotic microcapsule according to claims 26-28, comprising the following steps:
adding the probiotic microcapsules to food or health product, preferably, at an amount of about 0.02 to 1%, preferably about 0.04 to 0.5%, more preferably about 0.06 to 0.15%;
preferably, before final pasteurization, the probiotic microcapsules are added to the prepared product,
or
preferably, after final pasteurization, the probiotic microcapsules are sterilized and then added to the prepared product.

30. The method for preparing the food or health product containing probiotic microcapsule according to claim 29, wherein the step of sterilization treatment comprises:
(1) mixing the microcapsule particles with honey, preferably, wherein the ratio of the microcapsule to the honey is about 1:1000 to 1:10, more preferably about 1:500 to 1:10, about 1:100 to 1:10 or about 1:50 to 1:10;
(2) sterilizing the above-mentioned mixed microcapsule particles with ultraviolet, preferably, at an ultraviolet intensity of about 1,000 to 20,000 J/L, more preferably about 5,000 to 20,000 J/L, about 10,000 to 20,000 J/L, or about 15,000 to 20,000 J/L.

31. The method for preparing the food or health product containing probiotic microcapsule according to any one of claims 29 to 30, wherein the food or health product is yogurt, and the method is as follows:
a. mixing raw materials other than zymogenic strains and microcapsules to prepare a solution of mixed raw materials for fermented milk, preferably at about 40-80°C, and then cooled, preferably to below about 20°C;
b. stirring, homogenizing (preferably at a homogenizing pressure of about 150-200 bar), then carrying out high-temperature and long-term sterilization (preferably at a sterilization temperature of 95°C and a sterilization time of 300 seconds), and then cooling to about 41-43°C after sterilization;
c. inoculation and fermentation at 41-43°C;
d. demulsifying, turning over a tank, and then cooling to below about 25°C;
e. pasteurizing at a temperature of about 74°C and for a period of about 30 seconds;
f. cooling to 15-30°C and entering an aseptic tank;
g. aseptic filling;
wherein before step f and after step e, sterilized probiotic microcapsules are added; or
after step d and before step e, probiotic microcapsules are added.

32. A soft particle of a probiotic microcapsule, wherein the probiotic microcapsule according to any one of the proceeding claims is embedded in the soft particle.

33. A method for preparing microcapsule particles, comprising the following steps:
1) Primary core making:
1.1) uniformly mixing the embedded substance with the microcapsule core material, and then adding to a container for premixing;
1.2) preparing pellet cores by extrusion spheronization granulation method or centrifugal granulation method, and drying and collecting the obtained pellet cores;
2) secondary fluidization:
2.1) preparing water-blocking coating layer solution and hydrophilic coating layer solution;
2.2) using fluidized bed spray granulation method to make the water-blocking coating solution wrap the pellet core prepared by primary core making, and drying in a fluidized bed to form water-blocked coated particles;
2.3) spraying the hydrophilic coating layer solution outside the water-blocked coated particles, and drying and collecting the prepared microcapsule particles.

34. The preparation method according to claim 33, wherein in step 1), the embedded substance is a functional active substance selected from one or more of functional polysaccharides, functional lipids, functional proteins/peptides/amino acids, micro-ecological regulators, vitamins and minerals;
preferably, wherein the weight ratio of the embedded substance to the microcapsule core material is 1:6-1:2.5 (more preferably 1:5-1:4);
preferably, in step 1), a certain amount of water and dry powder are added; more preferably, the weight ratio of the added water to the dry powder is 1:1.5-1.5:1 (more preferably 1:1.2-1.2:1).

35. The preparation method according to claim 34, wherein the functional polysaccharide is selected from one or more of chitosan, tea polysaccharide, dietary fiber, and dextran; preferably, the functional lipid is selected from one or more of lecithin, EPA and DHA; preferably, the functional protein/peptide/amino acid is selected from one or more of taurine, lactoferrin, immunoglobulin, and whey protein peptide; preferably, the micro-ecological regulator is selected from one or more of probiotics, prebiotics, and synbiotics.

36. The preparation method according to any one of claims 33-35, wherein in step 2), the water-blocking coating layer solution comprises two or more water-blocking coating layer solutions (preferably, the two or more water-blocking coating layer solutions sequentially wrap the pellet core prepared by the primary core making); preferably, the main component of the water-blocking coating layer solution includes prolamin; preferably, the main components of the water-blocking coating layer solution are prolamin and oleic acid, or prolamin and glycerin (preferably, the weight ratio of prolamin and oleic acid is 3:1 to 1:1, more preferably 2.5:1 to 1.2:1; preferably, the weight ratio of prolamin to glycerin is 4.5:1 to 5:1, more preferably 4.5:1 to 4.8:1).

37. The preparation method according to any one of claims 33-35, between steps 2.2) and 2.3), further comprising the step of infiltrating the water-blocked coated particles in medium chain triglycerides (MCT) to form a selectable water-blocking coating layer; preferably, wherein the infiltration time is 12-24 hours (preferably 16 hours).

38. The preparation method according to any one of claims 33-35, wherein step 2.2) comprises: using a fluidized bed spray granulation method to raise the temperature of the water-blocking coating layer solution, wherein the inlet air temperature of the fluidized bed is 50-80°C, and the air flow is 20-100m³/h; preferably, wherein the water-blocking coating solution is atomized by a spray gun and then sprayed into the fluidized bed, so that droplets uniformly wrap the pellet core particles obtained by the primary core making, and the resulting particles are dried in a fluidized bed to form a single-layered water-blocked particles;
preferably, wherein the inlet air temperature of the fluidized bed is 50-70°C (more preferably 55-60°C), and the air flow is 50-90m³/h (more preferably 60-80m³/h);
more preferably, wherein after each 400 mL of the coating solution is fluidized, particles are passed through a 50-80 mesh screen, and particles larger than 80 mesh and smaller than 50 mesh are discarded;
preferably, step 2.2) further includes: after the single-layered water-blocked particles are formed, a fluidized bed spray granulation method is used to uniformly spray other water-blocking coating layer solution to form double-layered or multi-layered water-blocked coated particles.

39. The preparation method according to any one of claims 33-35, wherein step 2.3) further comprises: drying the prepared microcapsule particles in a fluidized bed, and after multiple fluidizations, collecting microcapsule particles.

40. The preparation method according to any one of claims 33-35, wherein when the granulation method in step 1.2) is extrusion spheronization granulation method, the embedded substance is mixed uniformly with the microcapsule core material, and the mixture is added to a wet granulation pot for premixing (preferably at a premixing time of 8-15min (more preferably 10 min));
preferably, wherein after spheronization granulation, the obtained particles are dried in a fluidized bed or oven (preferably at a drying temperature of 45-65°C (more preferably 45-50°C));
preferably, wherein the feeding speed is 25-35 RPM (more preferably 28-32 RPM); preferably, wherein the extrusion speed is 40-50 RPM (more preferably 45-50 RPM); preferably, wherein the spheronization speed is 350-420 RPM (more preferably 380-420 RPM).

41. The preparation method according to any one of claims 33-35, wherein when the granulation method in step 1.2) is centrifugal granulation method, the embedded substance is mixed uniformly with the microcapsule core material; preferably, wherein a turntable of a centrifugal granulator is used, and the embedded substance and the microcapsule core material are put into the turntable for premixing;
preferably, wherein after spheronization granulation, the obtained particles are dried in a fluidized bed (at a drying temperature of 40-60°C (more preferably 45-50°C));
preferably, wherein the rotating speed of the centrifuge turntable is 600-800 RPM (more preferably 700-750 RPM); preferably, wherein the ratio of the binder to the dry matter is 25-38 RPM (more preferably 30-35 RPM).

42. The microcapsule particles prepared by the preparation method according to any one of claims 33-41.

43. A microcapsule particle comprising three parts: a pellet core, a water-blocking coating layer and a hydrophilic coating layer; wherein the pellet core includes an embedded substance and a microcapsule core material; the water-blocking coating layer is one or more coating layers, which include prolamin; the hydrophilic coating layer is one or more coating layers, which include hydrophilic polysaccharide.

44. The microcapsule particle according to claim 43, wherein the embedded substance is a functional active substance selected from one or more of functional polysaccharides, functional lipids, functional proteins/peptides/amino acids, micro-ecological regulators, vitamins and minerals (preferably one or more of functional protein peptides, micro-ecological regulators and functional greases, more preferably functional protein peptides, polyunsaturated fatty acids, and/or micro-ecological regulators).

45. The microcapsule particle according to claim 44, wherein the functional polysaccharide is selected from one or more of chitosan, tea polysaccharide, dietary fiber, and dextran; preferably, the functional lipid is selected from one or more of lecithin, EPA and DHA; preferably, the functional protein/peptide/amino acid is selected from one or more of taurine, lactoferrin, immunoglobulin, and whey protein peptide; preferably, the micro-ecological regulator is selected from one or more of probiotics, prebiotics, and synbiotics.

46. The microcapsule particle according to claim 43 or 44, wherein the microcapsule core material comprises one or a combination of more of:
vegetable protein, such as soy protein, rice protein, wheat protein, corn protein, etc.; preferably corn protein, or
animal protein, such as whey protein, casein, etc.; preferably whey protein concentrate (WPC), whey protein isolate (WPI) or whey protein peptide, especially preferably whey protein isolate (WPI); or
greases, such as greases with a melting point of 40°C or higher, preferably with a melting point of 40-50°C, particularly preferably palm oil, medium chain glycerides, hydrogenated greases (e.g., hydrogenated palm oil, hardened oil, hydrogenated soybean oil), lecithin, cocoa butter substitutes, palm oil monoglycerides, coconut oil, soybean oil, peanut oil, sunflower oil, or
other materials, such as microcrystalline cellulose (MCC), glycerin, oleic acid, sodium alginate, shellac, CMC-Na, gellan, xanthan gum, k-carrageenan, cellulose acetate phthalate, Maltodextrin, starch, dextrin, sucrose, lactose, dextran, corn syrup, pectin, gum arabic, chitosan, acetylated mono- or di-glyceride fatty acid esters, konjac gum, carrageenan, wax or gelatin, etc.;
(preferably, the main component of the microcapsule core material is microcrystalline cellulose (MCC)).

47. The microcapsule particle according to claim 43 or 44, wherein the water-blocking coating layer further comprises the microcapsule core material comprises one or a combination of more of:
vegetable protein, such as soy protein, rice protein, wheat protein, corn protein, etc.; preferably corn protein, or
animal protein, such as whey protein, casein, etc.; preferably whey protein concentrate (WPC), whey protein isolate (WPI) or whey protein peptide, especially preferably whey protein isolate (WPI); or
greases, such as greases with a melting point of 40°C or higher, preferably with a melting point of 40-50°C, particularly preferably palm oil, medium chain glycerides, hydrogenated greases (e.g., hydrogenated palm oil, hardened oil, hydrogenated soybean oil), lecithin, cocoa butter substitutes, palm oil monoglycerides, coconut oil, soybean oil, peanut oil, sunflower oil, or
other materials, such as one or more of glycerin, oleic acid, sodium alginate, shellac, CMC-Na, gellan, xanthan gum, k-carrageenan, cellulose acetate phthalate, maltodextrin, starch, dextrin, sucrose, lactose, dextran, corn syrup, pectin, gum arabic, chitosan, acetylated mono- or di-glyceride fatty acid esters, konjac gum, carrageenan, wax or gelatin; preferably, wherein the prolamin is selected from one or more of zein and/or glutinous rice prolamin; preferably, wherein the water-blocking coating layer has one or more layers (more preferably 2-4 layers, most preferably 2-3 layers).

48. The microcapsule particle according to claim 43 or 44, wherein the main components of the hydrophilic coating layer include pectin, soybean polysaccharide, and/or whey protein isolate; preferably, wherein the hydrophilic coating layer has one layer.

49. The microcapsule particle according to claim 43, wherein the weight ratio of the embedded substance to the microcapsule core material is 1:6-1:2.5 (more preferably 1:5-1:4).

50. Use of the microcapsule particle according to any one of claims 42-49 as food and health products, such as heat-processed foods or frozen foods (preferably used in dairy products (e.g., milk, yogurt, cheese, ice cream, milk powder, milk beverage), fermented flavored foods, beverages, chocolates, candies such as chewing gum, baked foods such as pudding, or fruit and vegetable juices).
